# EUROPEAN PATENT SPECIFICATION

(11) **EP 1 728 864 B1**
(45) Date of publication and mention of the grant of the patent: **01.08.2012**
(21) Application number: 06012391.6
(22) Date of filing: 26.01.1996
(51) Int. Cl.: C12N 15/13, C07K 16/28, G01N 33/577, C07K 19/00, C12N 5/20, A61K 47/48, A61K 39/395, A61K 51/10

(54) **Monoclonal antibodies specific for different epitopes of human gp39 and methods for their use in diagnosis and therapy**
Spezifische monoklonale Antikörper für unterschiedliche Epitope des humanen gp39 und Verfahren zu ihrer Verwendung in Diagnose und Therapie
Anticorps monoclonaux spécifiques de differents epitopes de gp39 humaine et procédés concernant leur utilisation à des fins de diagnostic et de thérapie

(30) Priority: 26.01.1995 US 379057
(43) Date of publication of application: 06.12.2006
(62) Divisional of application: 96906207.4
(73) Proprietor: Bristol-Myers Squibb Company, New York, NY 10154 (US)
(72) Inventor: Siadak, Anthony W., Covington, WA 98042 (US); Hollenbaugh, Diane, Seattle WA 98117-2235 (US); Gilliland, Lisa K., Oxford OX2 8LH (GB); Gordon, Marcia L., Seattle WA 98117-2523 (US); Bajorath, Jurgen, Lynnwood WA 98037-7542 (US); Aruffo, Alejandro A., Edmonds WA 98020-3334 (US); Harris, Linda J., Seattle WA 98112-3313 (US)
(74) Representative: Reitstötter - Kinzebach

(56) References cited:
- EP-A- 0 555 880
- WO-A-95/06480
- WO-A-95/06666
- J. BAJORATH ET AL.: "Identification of residues on CD40 and its ligand which are critical for the receptor-ligand interaction." BIOCHEMISTRY, vol. 34, no. 6, 14 February 1995 (1995-02-14), pages 1833-1844, XP002007296 WASHINGTON, DC, USA
- A. ARUFFO ET AL.: "The CD40 ligand, gp39, is defective in activated T cells from patients with X-linked hyper-IgM syndrome." CELL, vol. 72, 29 January 1993 (1993-01-29), pages 291-300, XP002007297 CAMBRIDGE, MA, USA
- D. HOLLENBAUGH ET AL.: "Expression of functional CD40 by vascular endothelial cells." THE JOURNAL OF EXPERIMENTAL MEDICINE, vol. 182, no. 1, 1 July 1995 (1995-07-01), pages 33-40, XP000574867 NEW YORK, NY, USA
- L. RIECHMANN ET AL.: "Reshaping human antibodies for therapy." NATURE, vol. 332, 24 March 1988 (1988-03-24), pages 323-327, XP000020414 LONDON, GB

## Description

### Background of the Invention

A successful immune response requires coordinated interaction of multiple cell types. The interaction between T-helper cells (Th) and antigen-presenting cells (APC) such as B cells, monocytes, and dendritic cells results from complex communications involving signals received through soluble cytokines or membrane-bound proteins as well as adhesive interactions. Many of these signals are not specific to a directed immune response and the proteins are broadly distributed.

A number of important T cell surface proteins involved in cell-cell interactions have been identified including CD2. CD4, CD8, CD28, LFA-I, CTLA-4 and gp39. These proteins participate in cell-cell contact by binding to their counter-receptors on APC and provide important costimulatory signals to T cells which modulate signals received through the T-cell antigen receptor. These costimulatory signals arc necessary for the T cell to become fully engaged and express both membrane-bound and soluble factors required for the proper activation of the T cell-dependent effector cells (B cells, natural killer cells, monocytes, neutrophils, etc.). The gp39/CD40 T cell ligand/B cell receptor pair plays a critical role in the humoral immune response. *In vitro* studies have shown that this receptor/ligand pair is involved in B cell proliferation, antibody and cytokine production and cell viability. Studies *in vivo*, both through blocking with a monoclonal antibody or by observation of a genetic defect in gp39, have validated the *in vitro* results, and extended them to the requirement for a functional gp39 for germinal center formation during immune response to antigen.

CD40 is a 50 kDa type I membrane glycoprotein expressed by B cells, macrophages, follicular dendritic cells, thymic epithelium, normal basal epithelium, some carcinoma and melanoma-derived cell lines (Clark and Ledbetter 1986, Proc. Nat'l. Acad Sci. USA 83:4494; Paerlie et al. 1985, Cancer Immunol. Immunother. 20:23, Ledbetter et al. 1987, J. Immunol. 138:788; Young et al. 1989, Int. J. Cancer 43:786; Galy and Spits 1992, J. Immunol. 149:775, Alderson et al. 1993, J. Exp. Med. 178:669) and recently has been reported to be expressed on T cells (Armitage et al. 1993, Eur. J. Immunol. 23: 2326). It has been shown to be an important signaling molecule with a range of downstream effects in multiple systems. Early studies showed that CD40 was involved in B cell activation. Crosslinking CD40 with anti-CD40 monoclonal antibody induces B cell aggregation via LFA-I (Gordon et al. 1988, J. lmmunol. 140:1425, Barrett et al., 1991, J. Immunol. 146:1722), increases Ser/Thr (Gordon et al. 1988, *supra*) and Tyr (Uckun et al. 1991, J. Biol. Chem. 266:17478) phosphorylation of a number of intracellular substrates and provides a "competence" signal that allows B cells to proliferate and undergo class switching when stimulated with the appropriate second signal. For example, anti-CD40 monoclonal antibody can synergize with PMA (Gordon et al. 1987, Eur. J. Immunol. 17:1535) or anti-CD20 monoclonal antibody (Clark and Ledbetter 1986, *supra*) to induce B cell proliferation, with IL-4 to induce B cell proliferation (Gordon et al. 1987, *supra*; Rousset et al. 1991, J. Exp. Med. 172:705) and IgE secretion (Jabara et al. 1990, J. Exp. Med. 172:1861 ; Gascan et al. 1991, J. lmmunol. 147:8; Rousset et al. 1991, *supra*: Zhang el al. 1991, J. Immunol. 146:1836, Shapira et al. 1992, J. Exp. Med. 175:289) and with IL-10 and TGF-β to induce IgA secretion by slgD R cells (DeFrance et al. 1992. J. Exp. Med. 175:671).

Isolation of cDNA clone encoding human CD40 (Stamenkovic et al. 1989. EMBO J. 8:1403) shows that CD40 has a significant homology to the nerve growth factor receptor family. Using a soluble form of CD40. CD40-immunoglobulin fusion protein (CD40-1g) (Armitage et al. 1992. Nature 357:80; Lane et al. 1992. Eur. J. Immunol. 22:2573; Noelle et al 1992, Proc. Nat'l. Acad. Sci. USA 89:6550), it was found that the CD40 ligand (gp39, CD40-L), a protein of approximately 39 kDa, was expressed by activated human and murine T cells. In addition, blocking studies with CD40-Ig (Fanslow et al. 1992, J. Immunol. 149:655; Noelle et al. 1992, *supra*) or an anti-murine gp39 monoclonal antibody (MR1) Noelle et al. 1992, *supra*) showed that preventing gp39-CD40 binding resulted in inhibition ofB cell biological responses.

Complementary DNA encoding both murine (Armitage et al. 1992, Nature 357:80) and human (Hollenbaugh et al. 1992, EMBO J. 11:4313; Spriggs et al. 1992, J. Exp. Med. 176:1543) gp39 or a soluble recombinant form of gp39 and IL-4 or gp39 and 1L-10 can drive human B cells to secrete IgE and IgA, or IgG and IgM, respectively (Aruffo et al. 1993. Cell 72:291). Taken together, these results suggest that gp39 may be a T cell "switch" responsible for some aspects of B cell differentiation and isotype switching (Noelle et al. 1992, Immunol. Today 13:431).

Recently, the gene encoding gp39 was mapped to Xq26, the X chromosome region where the gene responsible for hyper -lgM syndrome (HIM) had previously been mapped (Aruffo et al. 1993, Cell 72:291). The gp39 molecules in the HIM patients were found to be functionally abnormal. Activated T cells have been found to produce normal levels of mRNA, but the gp39 encoded is defective (Aruffo et al 1993, *supra*; DiSanto et al. 1993, Nature 361:541).

Hyper-IgM syndrome is one of at least seven inherited immunodeficiencies mapped to the X-chromosome (Kinnon and Levinsky 1992, J. Inherit. Metab Dis. 15:674). The disease is characterized by low or absent IgG, IgA and IgE levels. normal or elevated levels of IgM. normal numbers of recirculating B cells, susceptibility to bacterial and opportunistic infections (including *Pneumocystic carinii*), no germinal centers, autoimmunity, neutropenia, X-linked and autosomal forms, and gp39 ligand gene defects in the X-linked form of the disease. Common Variable Immunodeficiency (CVI) is another group of immunodeficiency disorders characterized by abnormal antibody responses and recurrent bacterial infections. Clinical presentations of CVI are diverse, as the disorders described by the term include a wide variety of as yet uncharacterized detects. Disease states described as CVI commonly show decreased or absent serum IgG and IgA, while the levels of IgM may be normal or decreased. Although most CVI patients have normal T cell numbers and responses, some may have decreased numbers, abnormal CD4/CD8 cell ratios or abnormal T cell function. There is also an increased probability of autoimmune antibodies in this patient population.

Mutations in the gene encoding gp39 result in deletions giving rise to frame shifts and premature stop codons, or point mutations resulting in amino acid substitutions (Allen et al. 1993, Science 259:990. DiSanto et al. 1993, *supra*; Fuleichan et al. 1993, *supra*, Korthauer et al. 1993, *supra*; Aruffo et al. 1993, *supra*; Collard et al. 1993. Immunol. Today 14:559). The effect of these mutations on expression of gp39 by activated T cells has been examined using soluble CD40-Ig, polyclonal antibody raised against a gp39 bacterial fusion protein (anti-TRAP) (Graf et al. 1992. Eur. J. Immunol 22:3191; Korthauer et al. 1993, Nature 361:539) and a gp39 specific monoclonal antibody 5c8 (Lederman et al. 1992, J. Exp. Med. 175:1091). Staining with soluble CD40-Ig, gp39 expression was found to be absent, while that for anti-TRAP was normal on T cells from one out of three patients tested, which was confirmed using the monoclonal antibody. These results show that expression of gp39 is variable in HIM patients and it has been suggested that further work is needed to determine whether the variation in surface expression of mutant forms of gp39 correlates with HIM disease severity. In the absence of a family history of X-HIM, the disease is difficult to distinguish from CVI. The methods currently used to identity a defect in gp39 as the causative agent in X-HIM include the sequencing of nucleotides comprising the gp39 gene from cDNA formed from mRNA isolated from *in vitro* activated lymphocytes that do not bind CD40, but do contain mRNA encoding gp39. This method has been used to show one patient diagnosed with CVI actually suffers from hyper IgM syndrome. However, the methods are laborious and would be very expensive to use on a more generalized basis.

What is needed in the an are additional monoclonal antibodies reactive with different epitopes of gp39 which can be easily used to assay for mutant forms of gp39 and for other purposes in diagnostics to distinguish between common variable immunodeficiency and X-linked hyper-IgM, and in therapeutic methods to modulate disease states responsive to interactions between CD40 and its ligand gp39.

### Summary of the Invention

Herein described are monoclonal antibodies capable of binding to at least twelve separate epitopes on human gp39. Further described are antigen binding fragments and recombinant binding proteins, derived from the monoclonal antibodies of the present invention, which also bind to gp39. Also described are specific hybridomas which secrete monoclonal antibodies which bind to the twelve epitopes on gp39 disclosed.

Herein described are the monoclonal antibody antigen binding fragment or recombinant binding protein thereof is characterized by binding to a mutant form of human gp39 with a somewhat reduced or similar binding avidity when compared to the binding avidity to wild-type gp39 when the mutant form of gp39 comprises glutamic acid 129, serine 131 and threonine 135, tyrosine 145, asparagine 180 or phenylalanine 201 and glutamic acid 202 replaced by alanine, and further has a poor binding avidity to a mutant of gp39 comprising lysine 143 replaced by alanine than to wild-type gp39. The antibody is further characterized by the inability to bind gp39 in a Western blot. Specific examples of monoclonal antibodies having these characteristics are those secreted by the hybridomas 39-1.7 designated 11812, 39-1.128 designated ATCC HB 11818 and 39-1.26 designated ATCC HB 11820. In one embodiment, the invention provides a monoclonal antibody, an antigen binding fragment or recombinant binding protein thereof, which is specific for human gp39, wherein the antibody is that secreted by the hybridoma 39-1.7 designated ATCC HB 11812, 39-1.128 designated ATCC HB 11818, or 39-1.26 designated ATCC HB 11820.

Each of the groups of monoclonal antibodies recognize epitopes of gp39 and can be manipulated either chemically or by recombinant methods that generate either antigen binding fragments or recombinant binding proteins. Examples of antigen binding fragments are the Fab, (Fab')₂ or Fv created by enzyme digestion of whole antibody. Recombinant binding proteins of the present invention include any molecule which maintains the antigen specificity of the parental antibody and has been recombined with other amino acid residue sequences. Examples include chimeric antibodies, sFvs, humanized antibodies and fusion molecules.

In still another embodiment of the present invention, the monoclonal antibodies or recombinant binding proteins can be conjugated to a detectable marker or a therapeutic agent. Examples of detectable markers include fluorophores, radioactive isotopes, enzymes or chromophores. Therapeutic agents contemplated by the present invention can include radioisotopes, toxin, or a chemotherapeutic agent, such as a cytotoxic drug. In addition to conjugation techniques, the recombinant binding proteins of the present invention can be constructed to form fusion proteins that comprise a variable region derived from a monoclonal antibody of the present invention and an enzyme, protein toxin or proteinaceous therapeutic agent.

In yet another embodiment of the present invention, an in vitro method for the detection of X-linked hyper IgM syndrome is disclosed. The method comprises the steps of providing isolated peripheral blood lymphocytes from a patient suspected of having symptoms associated by the syndrome, activating the peripheral blood lymphocytes, fixing and permeabilizing the isolated and activated peripheral blood lymphocytes, admixing a monoclonal antibody described with the activated, fixed and permeabilized peripheral blood lymphocytes, and detecting antibody bound to the cells. The antibody can be labeled with a detectable marker or can be unlabeled. When used unlabeled, a further step of adding a secondary antibody (which is labeled) specific for the first antibody is carried out prior to the detection step. The detectable marker can be, for example, a fluorophore, radioactive isotope, enzyme or chromophore.

Further, the present invention provides hybridomas which secrete specific antibodies reactive with each of the epitopes described by the present invention. Each of these hybridomas was deposited with the American Type Culture Collection, 12301 Parklawn Drive, Rockville, MD 20852 on January 20, 1995 under the conditions of the Budapest Treaty.

In yet another embodiment of the present invention, an isolated and purified nucleic acid sequence which encodes amino acid sequences for immunoglobulin light and heavy chains of immunoglobulin molecules which recognize epitopes of human gp39 are described by the present invention. In particular, the nucleic acid sequence encodes an amino acid sequence of the immunoglobulin light chain variable region depicted in Sequence ID# 16. Also disclosed are specific nucleotide sequences which encode these amino acid sequences. Those are depicted in and 15. Also, the nucleic acid sequences which encode immunoglobulin heavy chain variable regions having the amino acid residue sequence depicted in and Sequence ID# 18 are provided. Particular nucleotide sequences which encode the amino acid residue sequences are provided in and Sequence ID# 17.

The present invention also provides pharmaceutical compositions comprising the monoclonal antibodies, antigen binding fragments or recombinant binding proteins thereof described herein combined with a pharmaceutically acceptable carrier These compositions can include the monoclonal antibody, antigen binding fragment, or recombinant binding protein conjugated to a detectable marker or therapeutic agent.

Further, the present invention provides monoclonal antibodies, antigen binding fragments or recombinant binding fragments thereof of the invention conjugated to a detectable marker for use in the imaging of cells expressing gp39 on their surface in a patient comprising administering to a patient a pharmaceutical composition including a monoclonal antibody, antigen binding fragment or recombinant binding fragment thereof of the invention described above conjugated to a detectable marker under conditions permitting the formation of antibody/ antigen complex on the surface of the cells expressing gp39, and detecting the presence of the antibody/antigen complex as indicated by the presence of the detectable markers.

### Description of the Figures

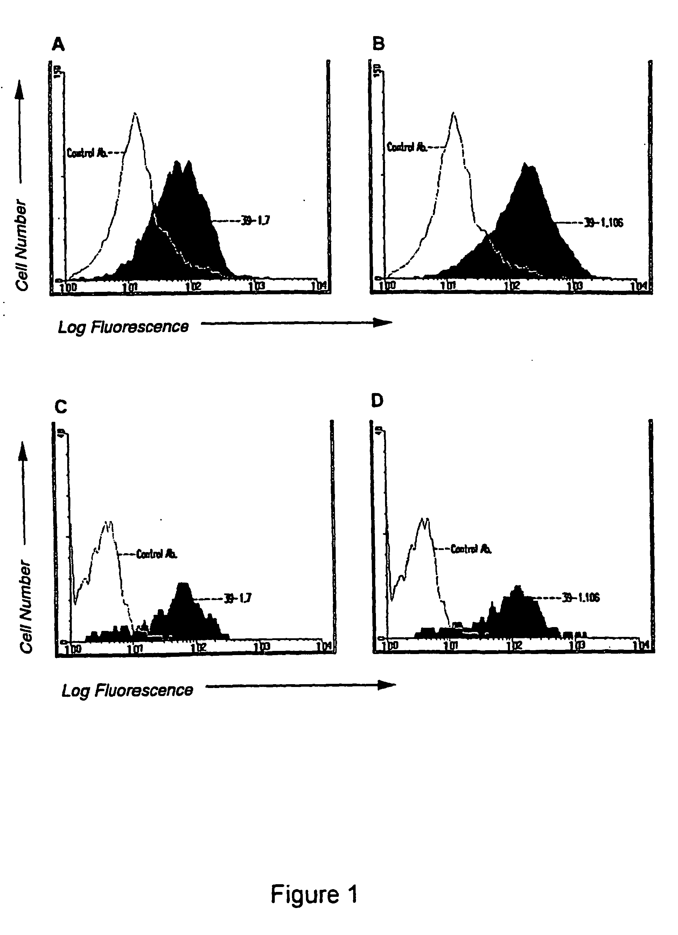

Figures 1A through ID demonstrate the binding of murine anti-human gp39 monoclonal antibodies 7 (39-1.7) and 106 (39.1-106) to activated human and macaque T cells. E rosette positive T cells were isolated from peripheral blood mononuclear cells, activated with PMA and ionomycin for six hours and then incubated with anti-human gp39 monoclonal antibodies 7, 106 or a negative control antibody. Cells were then incubated with an FITC goat anti-murine IgG polyclonal antisera followed by a phycoerythrin labeled mouse anti-human CD4 monoclonal antibody. Cells were gated for CD4⁻ cells and subsequently analyzed for anti-gp39 staining using a FACScan. Figures 1A and 1B Human T cells. Figures 1C and 1D Macaque T cells.

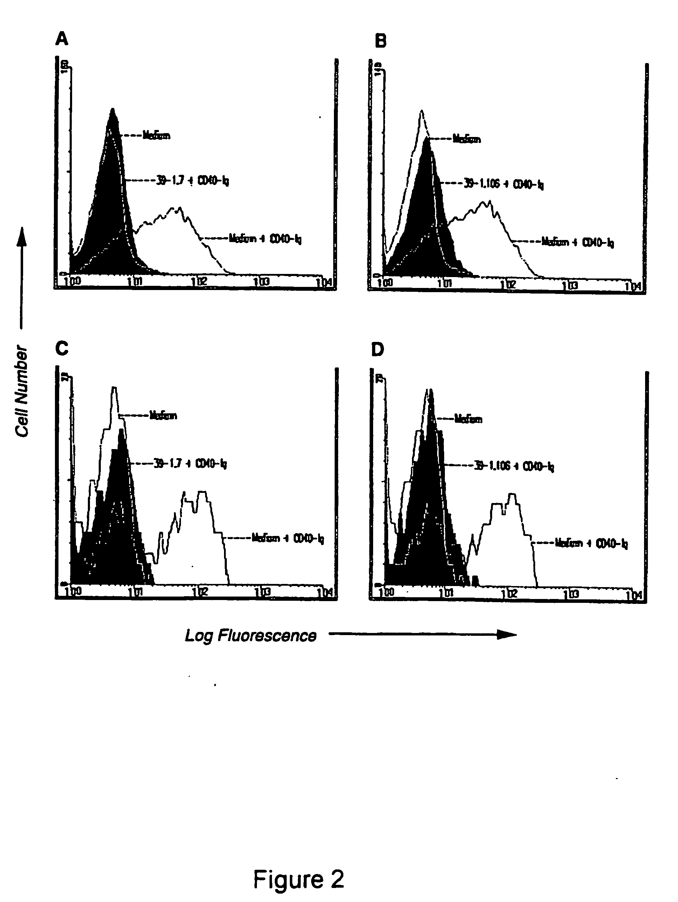

Figures 2A through 2C demonstrate the inhibition of human CD40-Ig binding to activated human and macaque T cells by murine anti-human gp39 monoclonal antibodies 7 and 106. E rosette positive T cells were isolated by peripheral blood mononuclear cells and stimulated with PMA and ionomycin for 6 hours. Activated T cells were incubated with murine anti-human gp39 monoclonal antibodies 7 (39-1.7) or 106 (39-1.106) at a concentration of 5 µg/ml followed by human CD40-Ig (20 µg/ml) After incubation, the cells were stained with phycoerythrin labeled goat anti-human IgG and analyzed on a FACScan. Figure 2A Human T cells and monoclonal antibody 7. Figure 2B Human T cells and monoclonal antibody 106. Figure 2C Macaque T cells and monoclonal antibody 7. Figure 2D Macaque T cells and monoclonal antibody 106.

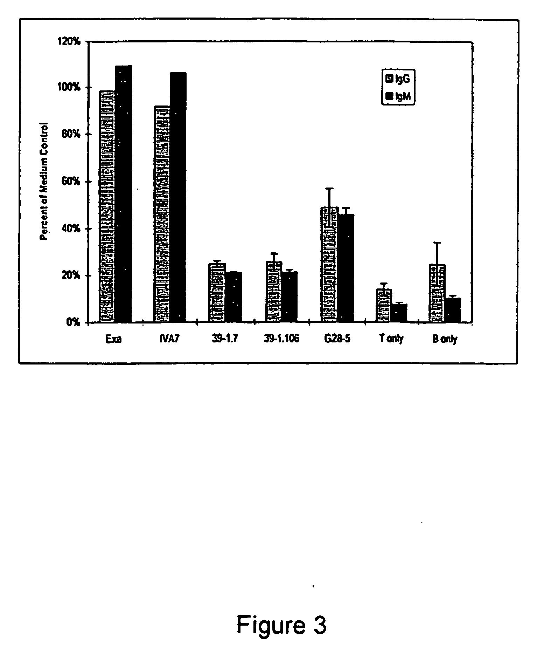

Figure 3 demonstrates the ability of murine anti-human gp39 monoclonal antibodies 7 and 106 to inhibit the production of IgG and IgM by macaque B cells stimulated by activated macaque T cells.

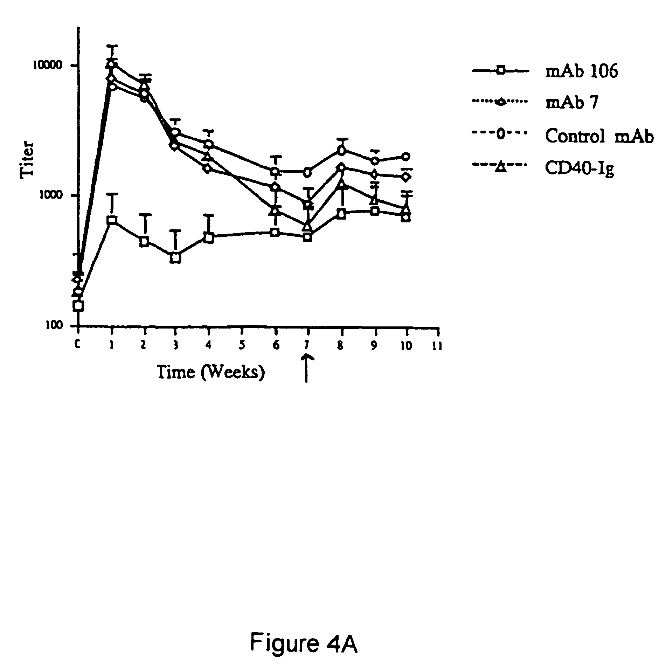

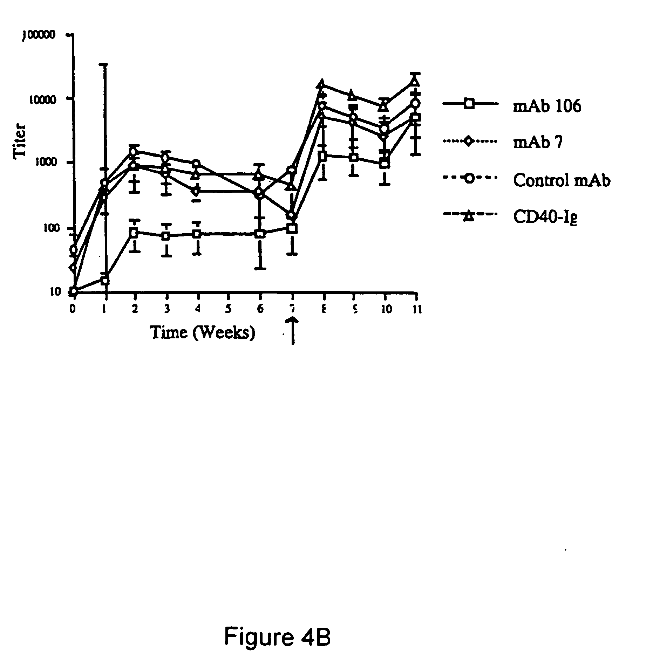

Figure 4A and 4B demonstrates the ability of gp39 binding proteins to suppress an immune response in macaques to sheep red blood cells. Serum was collected weekly and (Fig. 4A) anti-SRBC IgM titers and (Fig. 4B) anti-SRBC IgG titers were assessed by ELISA. Values represent mean + SEM for 4 monkeys per group. The arrow indicates the time of the secondary Immunization. Value reported is titer as determined by the dilution of serum yielding absorbance values five times the background value, where background is determined as the absorbance measurement recorded in the absence of serum.

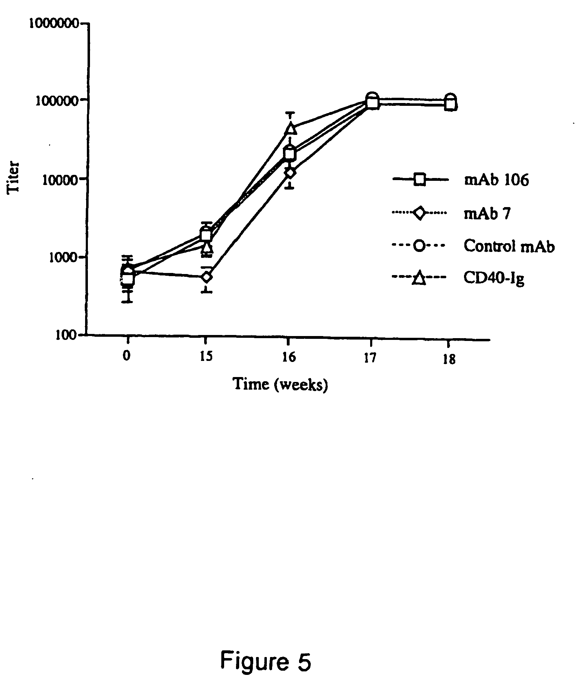

Figure 5 demonstrates the ability of macaques treated with gp39 binding proteins to generate an IgG response to KLH. Serum titers of specific monkeys' antibody were determined by ELISA. Results are the average of all monkeys in each group. Value reported is titer as determined by the dilution of serum yielding absorbance values five times the background value, when background is determined as the absorbance measurement recorded in the absence of serum

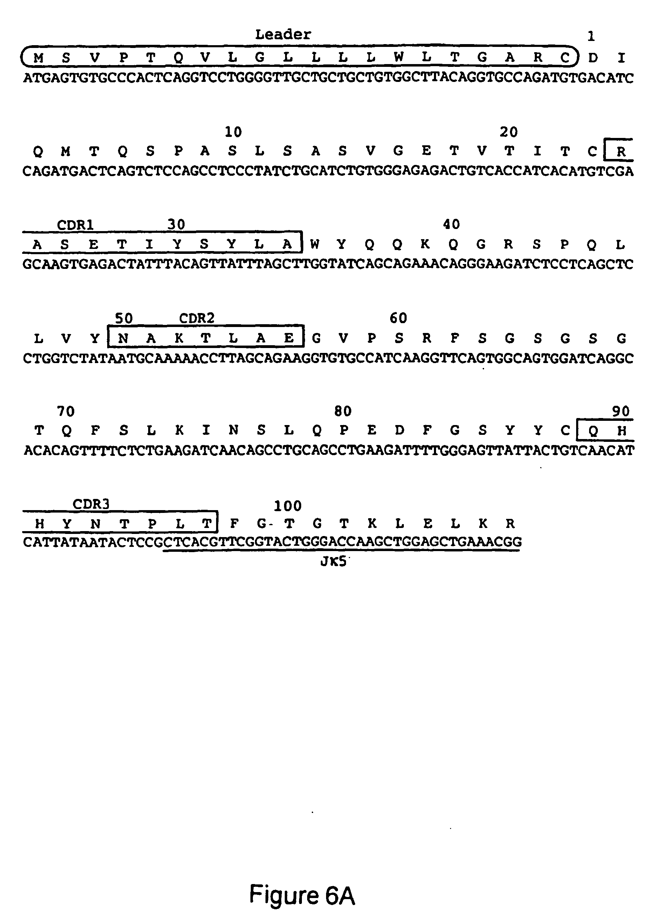

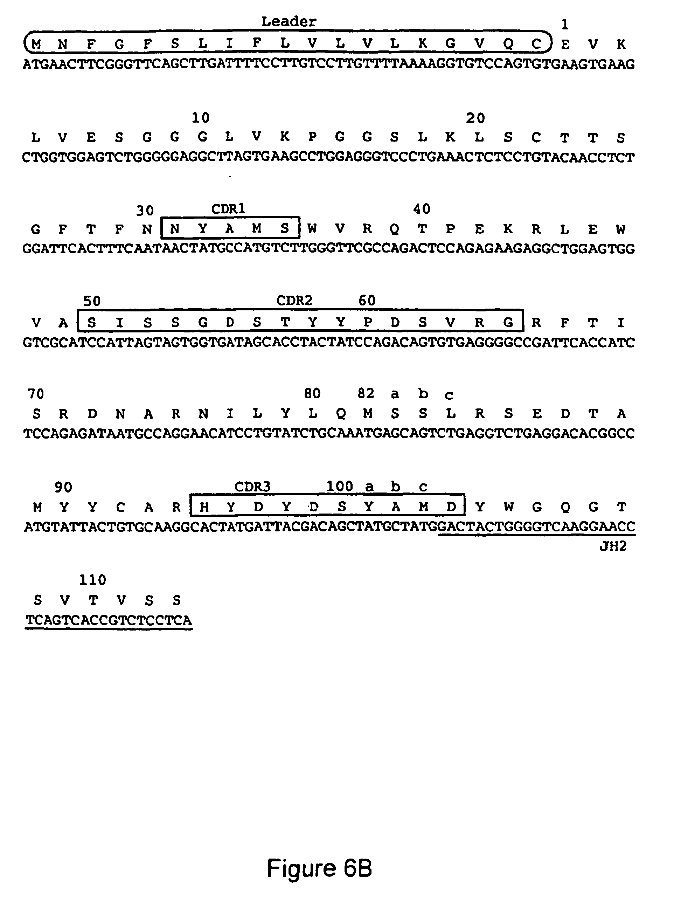

Figure 6 provides the nucleotide sequence for 106 VL (Seq. ID #II) and the deduced amino acid sequence (Seq. ID # 12). Figure 1B provides the nucleotide sequence for 106 VH (Seq. 1D #13) and the deduced amino acid sequence (Seq. ID #14). The leader sequences are encircled and the complimentarity determining regions are shown in boxes. The VL is a member of the murine kappa V subfamily and the V gene segment has rearranged with J_{K} 5 (Figure 6A, underlined). The VH is a member of the murine III(D) subgroup. The heavy chain V gene has rearranged with JH2 (Figure 6B. underlined).

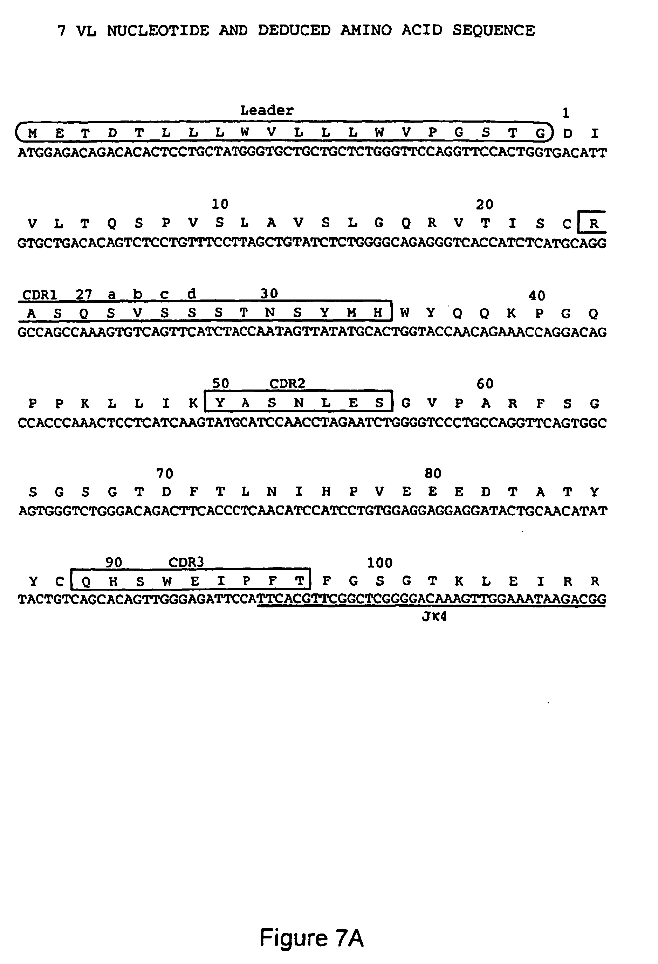

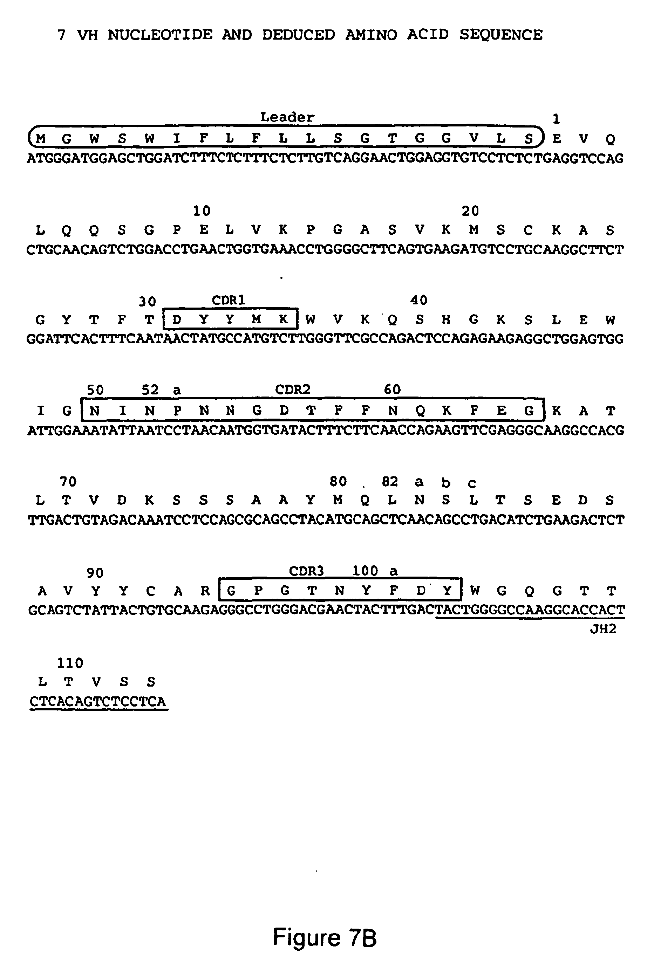

Figure 7 provides the nucleotide sequence for 7 VL (Seq. ID # 15) and the deduced amino acid sequence (Seq. ID #16). Figure 2B provides the nucleotide sequence for 7 VH (Seq. ID #17) and the deduced amino acid sequence (Seq. ID #18). The leader sequences are encircled and the complimentarity determining regions are shown in boxes. The VL is a member of the murine kappa II subfamily and the V gene segment has rearranged with Jκ 4 (Figure 7A, underlined). The VH is a member of the murine II(A) subgroup. The heavy chain V gene has rearranged with JH2 (Figure 7B, underlined).

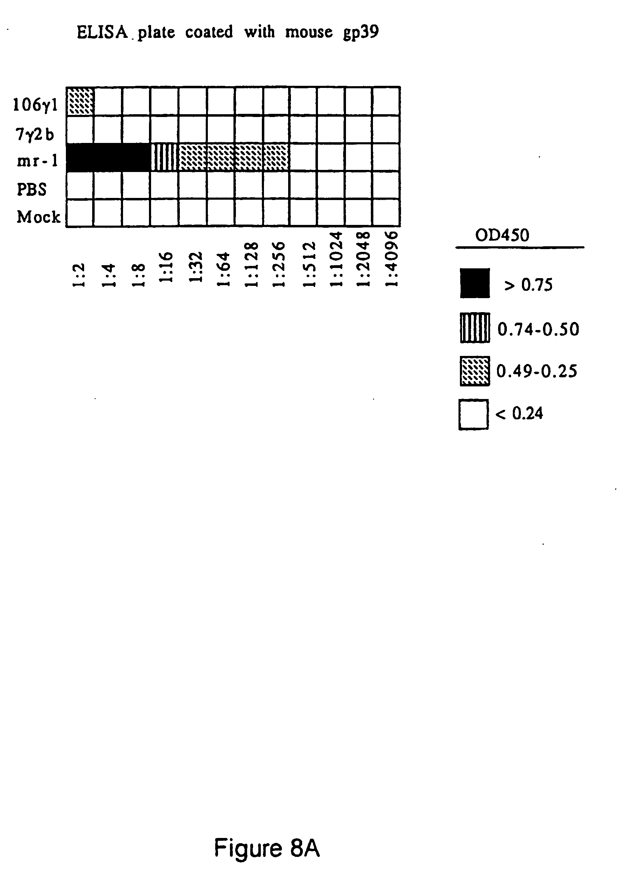

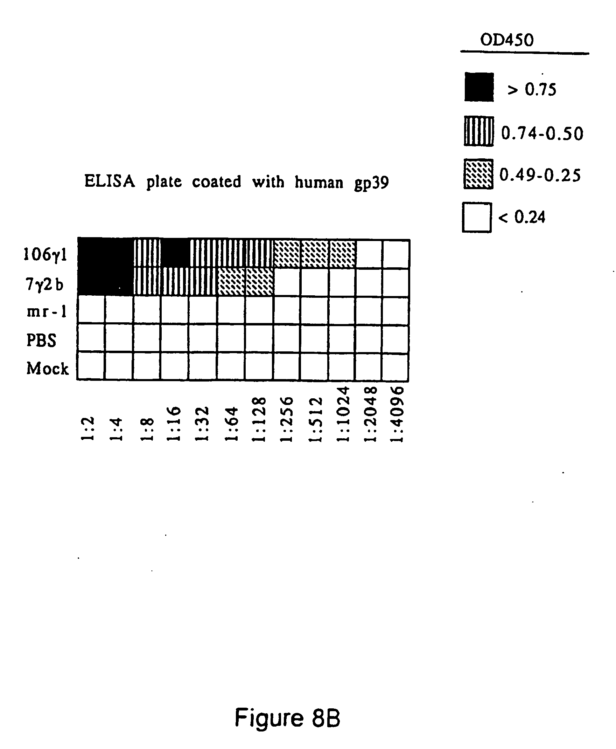

Figures 8A and B demonstrate a titration of 106 sFv-Ig and 7 sFv-Ig COS cell transfection supernatants binding to immobilized human gp39. Flat bottom 96-well plates coated with anti-mouse Lyt-2a and Lyt-2a-gp39 fusion protein were used to screen COS cell supernatants for functional anti-gp39 106 and 7 sFv-Ig. Two-fold dilutions of a representative clone for each sFv-Ig are shown. While mock transfection supernatant (no DNA added to COS cells) showed no activity, 106 sFv-Ig and 7 sFv-Ig bound to immobilized gp39 at dilutions in excess of I: 100 (for 106 sFv-Ig, binding could be detected down to a I: 1000 dilution of transfection supernatant). In comparison, an anti-mouse gp39 sFv (MR1 sFv-Ig) did not bind to human gp39 although it bound well to plates coated with anti-mouse Lyt-2a and Lyt 2a-murine gp39 fusion protein. 106 sFv-Ig and 7 sFv-Ig showed little to no reactivity on plates coated with anti-mouse Lyt-2a and Lyt 2a-murine gp39 fusion protein.

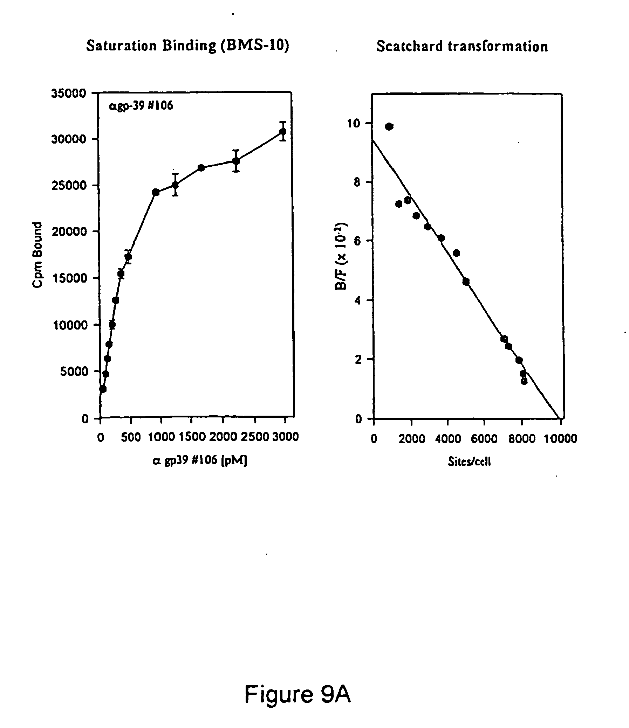

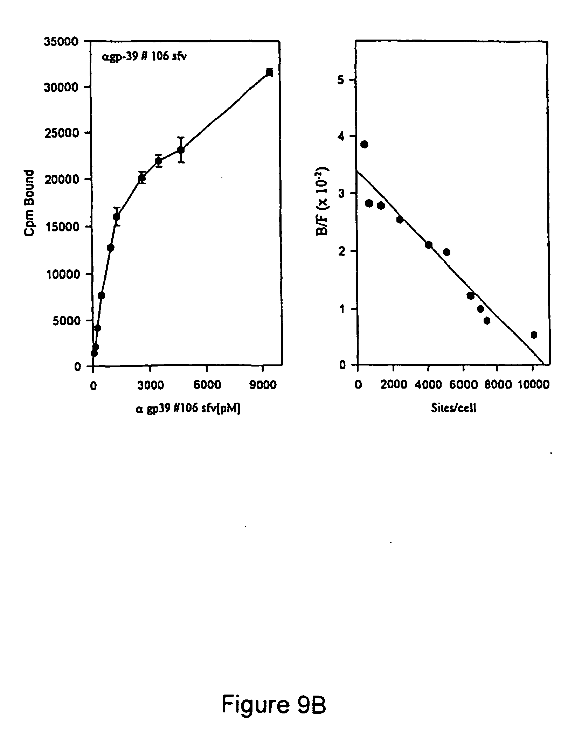

Figures 9A and 9B show the comparative binding of bivalent 106 monoclonal antibody and 106 sFv-Ig to Jurkat cells constitutively expressing gp39. lodinated bivalent 106 mAb was compared to iodinated 106 sFv-Ig for binding to gp39 expressed on BMS-10 Jurkat cells. The calculated affinities were Kd=4 x 10⁻¹⁰ ± 6 x 10⁻¹¹ for bivalent 106 mAb (Figure 9A) and Kd=1.6 x 10⁻⁹ ± 3.3 x 10¹⁰ for 106 sFv-Ig (Figure 9B). Scatchard transformation showed that both bivalent 106 mAb and 106 sFv-Ig bound approximately 10,000 sites per cell (Figures 9A and 9B).

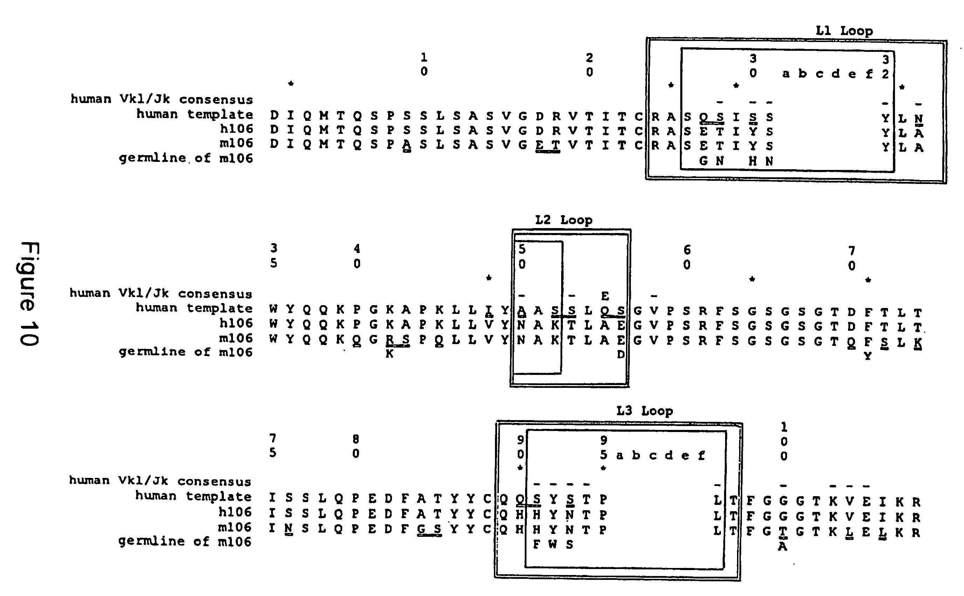

Figure 10 depicts the 106 VL humanization template. The original murine sequence is shown in the fourth row (m106, Seq. ID #27) with the murine germline sequence beneath it. The chosen human template sequence is shown in the second row (human template, Seq. ID #29) with its human consensus sequence above it. The humanized 106 VL sequence (h106, Seq. ID #28) is shown between the human template and the murine 106 VL sequence. It consists essentially of human framework residues and murine hypervariable residues. The hypervariable regions as defined by Kabat et al. (Sequences of Proteins of Immunological Interest, 4th ed., U.S. Health and Human Services, Washington, D.C. (1987)) are shown outlined with a double line The L1, L2 and L3 loops are outlined in a single line and structural determinants defined by Chothia are shown by asterisks (Chothia and Lesk, 1987,J. Mol. Biol. 196: 901). Human or murine residues differing from the humanized 106 VL are double underlined. The human Jκ was chosen on the basis of homology to the 106 Jκ.

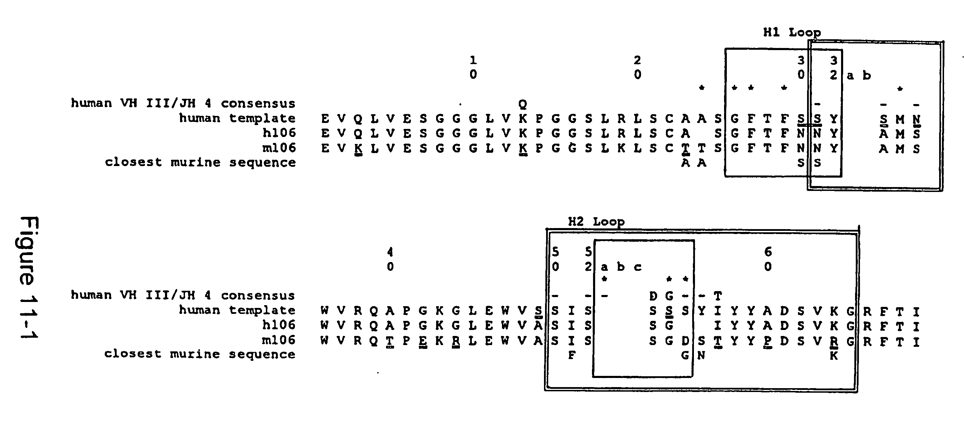

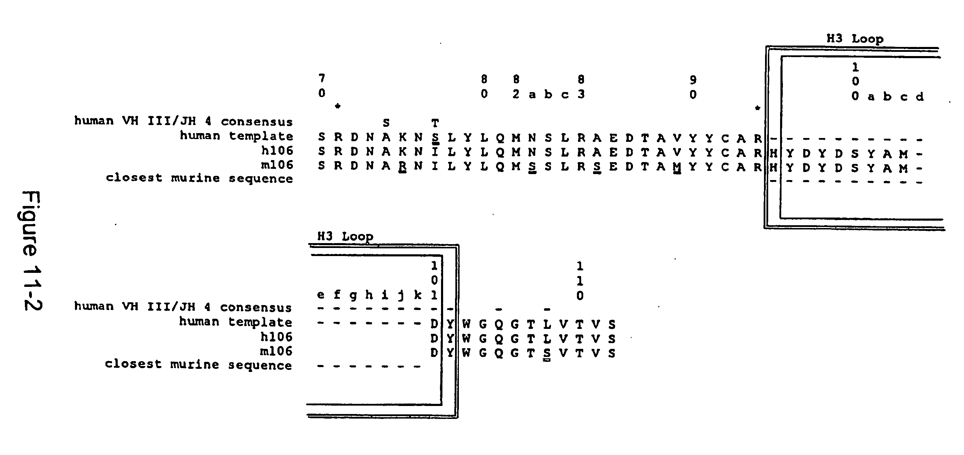

Figure 11 depicts the 106 VH humanization template. The original murine sequence is shown in the fourth row (m106. Seq. ID #30) with the closest murine sequence beneath it (a suitable germline sequence having only three residues in the H2 loop was not available; instead, a rearranged sequence was chosen that had an overall high homology to 106 VH and also had a three residue H2 loop). The chosen human template sequence is shown in the second row (human template, Seq. ID #32) with its human consensus sequence above it (human VHIII/JH4 consensus). The humanized 106 VH sequence (h106, Seq. ID #31) is shown between the human template and the murine 106 VH sequence. It consists essentially of human framework residues and murine hypervariable residues (outlined with a double line). The H1, H2 and H3 loops are outlined with a single line and structural determinants as defined by Chothia (*supra*) are shown by asterisks. Human or murine residues differing from the humanized 106 VH are double underlined. The human JH was chosen on the basis of homology to 106 JH.

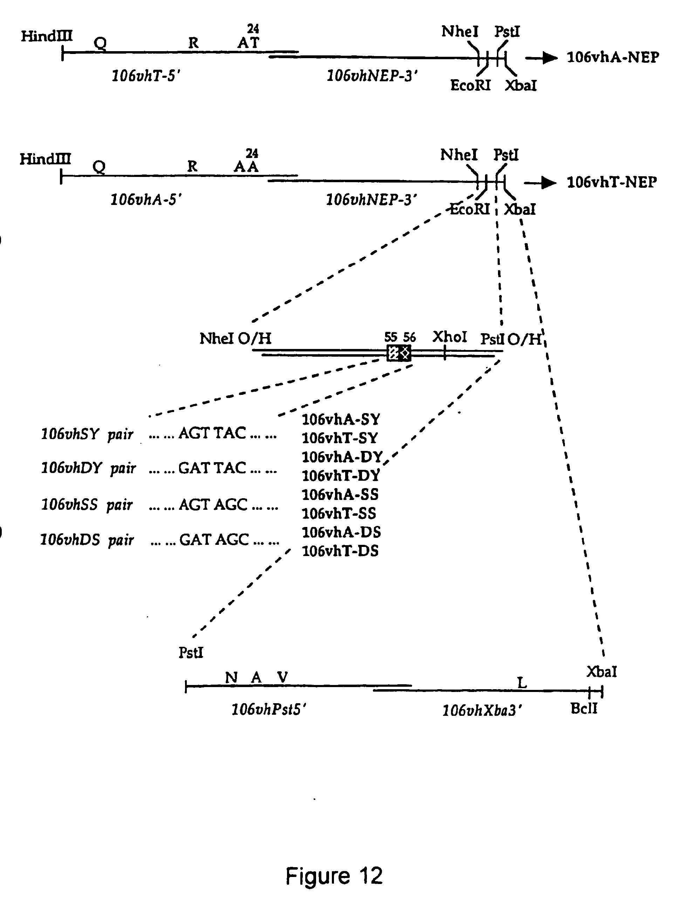

Figure 12 depicts the assembly of the eight humanized 106 VH. Two DNA fragments were amplified by PCR of the first 149 bases of the murine 106 VH using sense primers that encoded a *HindIII* site immediately prior to 106 VH sequence containing changes of three (106 vh T-5') or four (106 vhA-5') of the murine residues to human residues, and an antisense primer that encoded unique restriction sites (*Nhel, EcoRI, PstI* and *XbaI*). These fragments were digested with *HindIII* and *XbaI* and were ligated into pUC19, creating the two vectors 106 vhA-NEP and 106 vhT-NEP Three pairs of synthesized oligonucleotides encoded changes at one or two positions (106 vh SY, 106 vh DY, 106 vh SS) while 106 vh DS maintained the original murine sequence at residues 55 and 56. All four pairs also encoded additional humanized residues of Ile57, Ala60, Lys64 and Lys75 which are not illustrated for simplicity. In addition, they were engineered with *Nhel* and *Pstl* overhangs (O/H) and a unique *Xhol* site for diagnostic digests. The DNA fragments generated by these oligonucleotides were ligated into the 106 vhA-NEP and 106 vhT-NEP vectors at the *Nhel* and *Pstl* sites. A final PCR fragment was generated using the 106 vh Pst5' sense primer and the 106 vh Xba3' antisense primer using murine 106 VH as the template. These two oligonucleotides encoded four more changes from murine to human sequence. The DNA fragment was cloned into the previous constructs using *Pstl* and *Xbal* restriction sites.

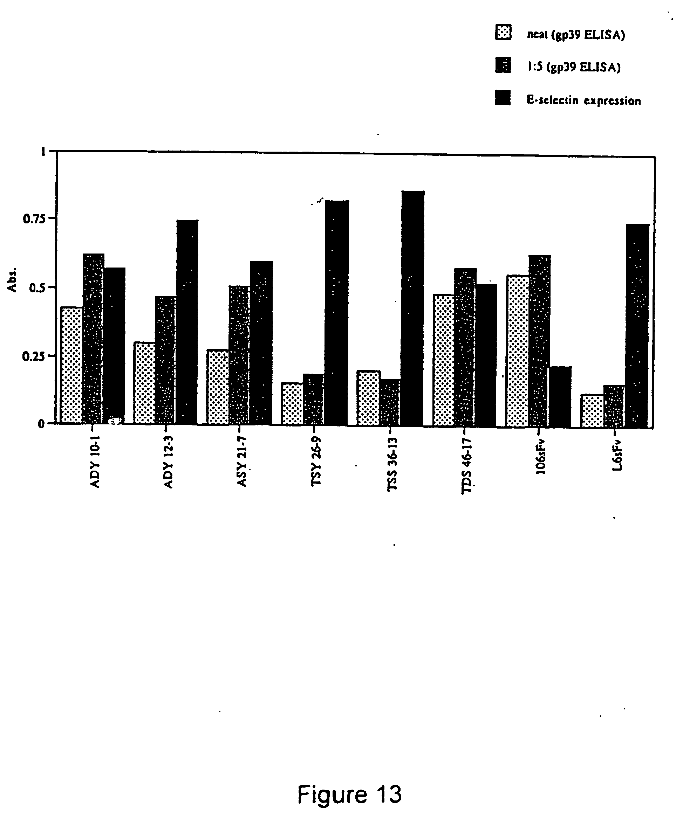

Figure 13 demonstrates the inhibition of E-selectin expression on endothelial cells. The black bars show expression levels of E-selectin. While the murine 106 sFv-lg shows strong inhibition, the L6 sFv-Ig negative control shows no inhibition. HuVL/106 vhA-DY ("ADY"), huVL/106 vhA-SY ("ASY") and hu VL/106 vhT-DS ("TDS") inhibit E-selectin expression, although not as effectively as the murine 106 sFv-Ig. Supernatants from the hu VL/106vhT-SY ("TSY"; no protein) and huVL,/106vhT-SS ("TSS"; aberrant protein) transfections did not show any activity.

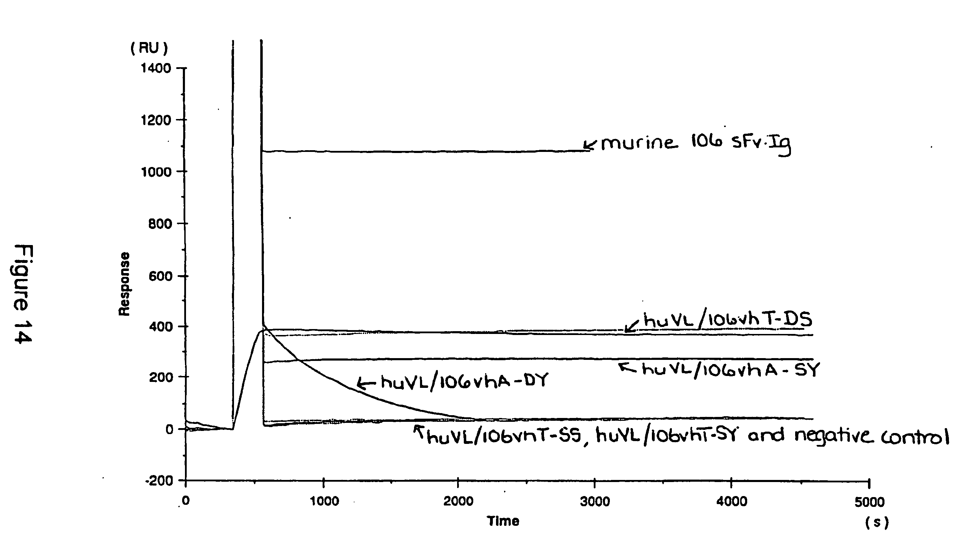

Figure 14 depicts the Biacore™ analysis of humanized 106 sFv-Ig proteins, binding to human gp39. Human gp39 was coated on chips and the various humanized 106 sFv-Ig transfection supernatants were tested for binding. The original murine 106 sFv-Ig bound very tightly (no off-rate observed, as shown by horizontal line). Proteins from the huVL/106vhA-DY ("ADY12-3"), huVL/106vhA-SY ("ASY21-7") and huVL/106vhT-DS (TDS46-17") transfection supernatants also bound tightly with no detectable off-rate. Supernatants from the huVL/106vhT-SY ("TSY26-9"; no protein) and hu VL/106vhT-SS ("TSS36-13"; aberrant protein) transfections did not bind to gp39-coated chips.

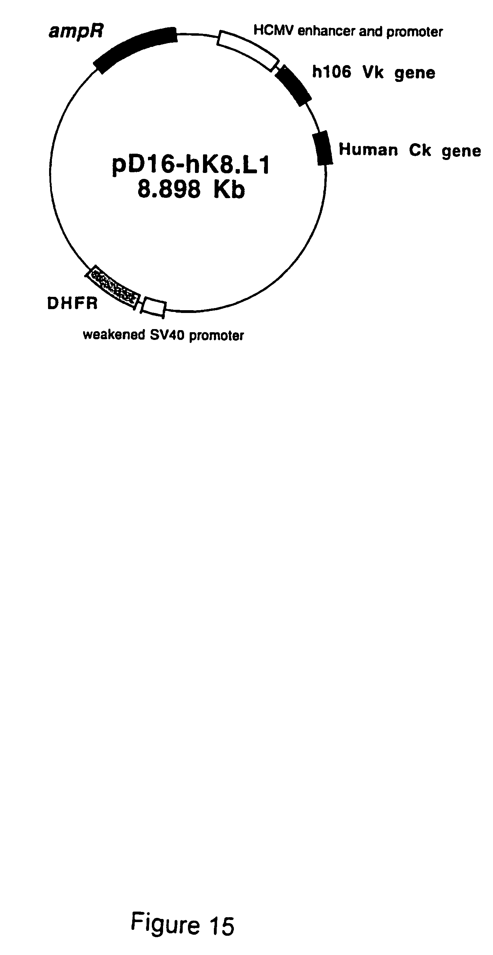

Figure 15 provides a depiction of the complete h106 light chain expression plasmid pD16-hK8.L1.

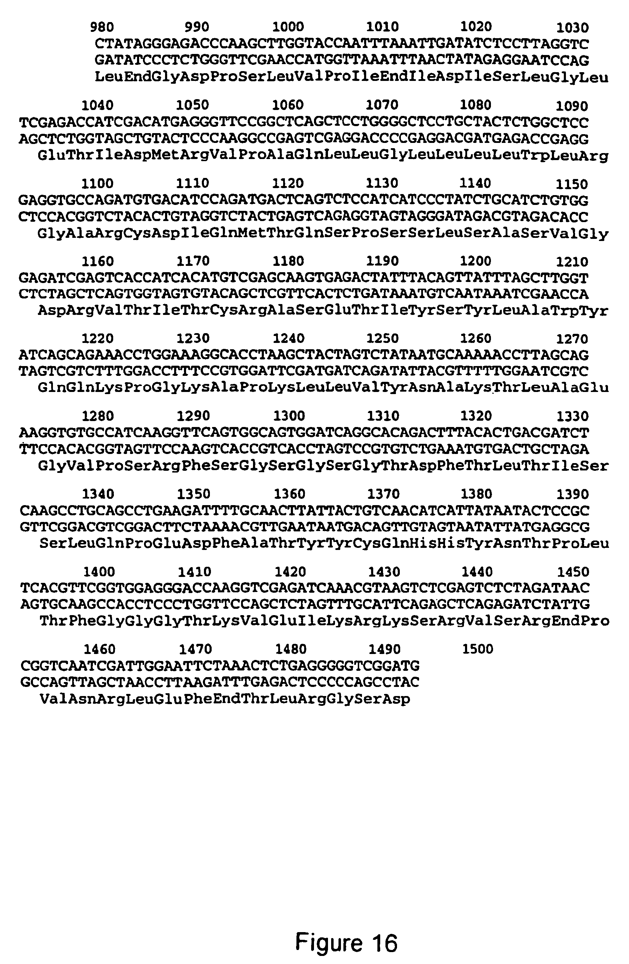

Figure 16 depicts the nucleic acid (Seq. ID #76) and amino acid residue (Seq. ID #77) sequences for the h106 immunoglobulin light chain insert which encodes the h106 signal peptide, variable region and the associated flanking regions.

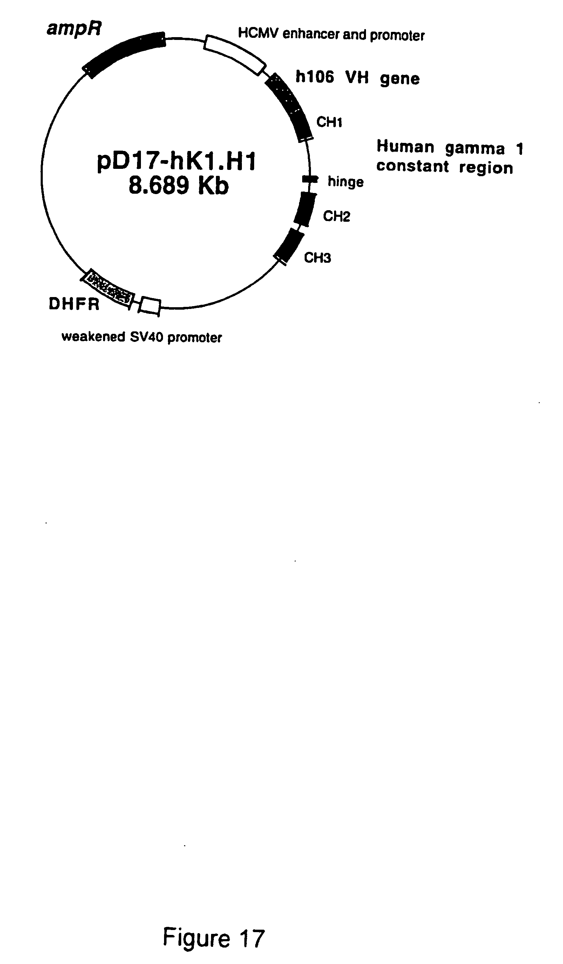

Figure 17 provides a depiction of the complete h106 heavy chain expression vector pD17-hK1.H1.

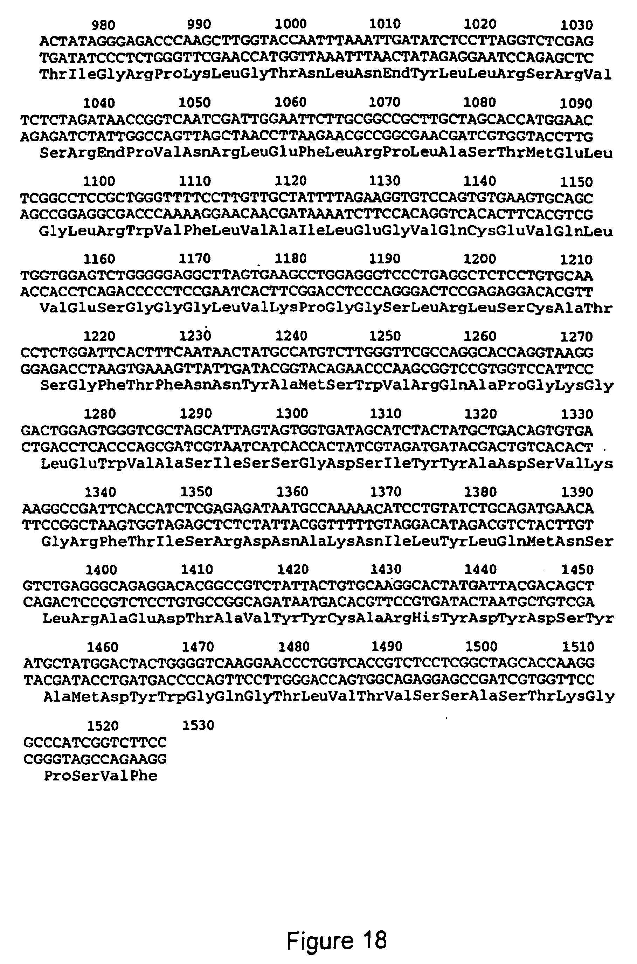

Figure 18 depicts the nucleic acid (Seq. ID #78) and amino acid residue (Seq. ID #79) sequences for the h106 heavy chain insert which encodes the h106 signal peptide, variable region and the associated flanking regions.

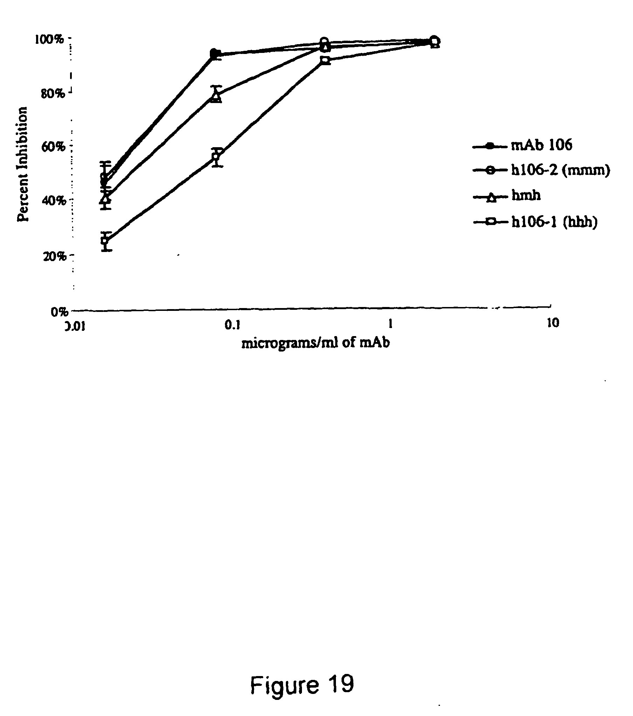

Figure 19 provides data for the inhibition of B cell proliferation by sgp39 in the presence of anti-human IgM. Resting tonsillar B cells were cultured in the presence of sgp39, rabbit anti-human IgM immunobeads, and the indicated amounts of monoclonal antibody for 72 hours. Plates were then pulsed with (³H]-thymidine and incubated for an additional 16 hours. After incubation the cells were harvested and [³H]-thymidine incorporation determined. All tests were performed in triplicate. Results are expressed as percent inhibition compared to cultures that contained only medium.

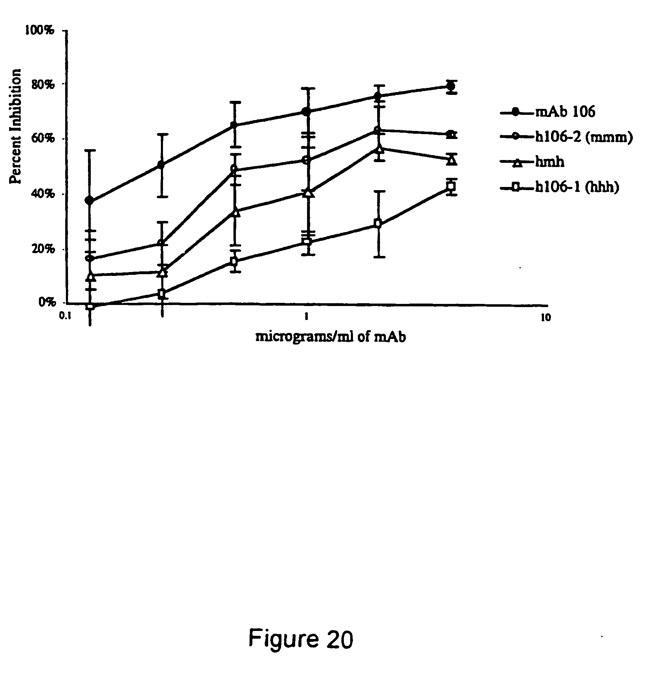

Figure 20 compares the ability of the various humanized 106 antibody proteins to inhibit the antibody production by human B cells stimulated with activated T cells. Human peripheral blood mononuclear cells were depleted of monocytes and natural killer cells and then separated into E rosette positive (T cells) and E rosette negative (B cells). T cells were subsequently treated with mitomycin C and cocultured with B cells in anti-CD3 monoclonal antibody coated wells of a 96 well plate in the presence of the indicated amount of monoclonal antibody. All tests were run in triplicate. Results are expressed as percent inhibition compared to cultures that contained medium only (no anti-gp39 monoclonal antibody).

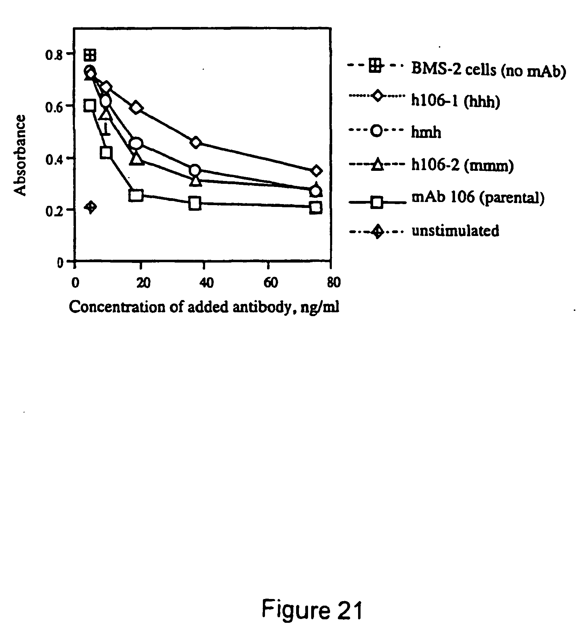

Figure 21 compares the ability of the various humanized 106 antibody proteins to inhibit the induction of E-selectin expression on endothelial cells by a gp39' T cell line. Human umbilical vein endothelial cells were cultured with BMS-2 cells, a Jurkat line known to express gp39, in the presence of the indicated amount of antibody. After four hours, the level of E-selectin expression was measured by ELISA. Error bars are smaller than the graph symbols.

### Detailed Description of the Invention

In order that the invention herein described may be more fully understood, the following description is set forth.

The present invention is directed to a group of monoclonal antibodies which recognize specific epitopes of the T cell membrane glycoprotein gp39, and to the hybridomas which produce and secrete these monoclonal antibodies. Also encompassed by the present invention are other monoclonal antibodies which can be made which competitively inhibit the binding of the specifically disclosed monoclonal antibodies to their epitopes. Fragments of the monoclonal antibodies and recombinant proteins having the variable region of the disclosed monoclonal antibodies are also included in the present invention, as are methods of using the monoclonal antibodies, fragments and recombinant binding proteins in diagnosing hyper IgM syndrome, in other cell adhesion and T cell assays, and in methods of modulating immune responses in a host.

The preparation of monoclonal antibodies can be accomplished by immortalizing a cell line producing antibody specific for an epitope on gp39. Typically, a monoclonal antibody of the present invention can be produced using well established hybridoma techniques first introduced by Kohler and Milstein. See, Kohler and Milstein, 1975, Nature 256:495. See also, Brown et al. 1981, J. Immunol. 127:539. Yeh et al. 1979, Proc. Nat'l. Acad. Sci. USA 76:297; Hellstrom et al. 1990. Cancer Research 50:2183.

These techniques involve the injection of an immunogen (e.g., cells or cellular extracts containing the gp39 antigen or purified gp39, either as native protein, a fragment containing an epitopic site, or a fusion protein) into an animal so as to elicit a desired immune response in that animal. Animals commonly used include many mammals, e.g., mouse, rat, cow, goat, sheep, rabbit, etc. The immunogen is commonly presented to the animal with an adjuvant, e.g., complete Freund's adjuvent, aluminum hydroxide gel, or the like. The animal may then be bled and the blood employed for the isolation of polyclonal antibodies. Alternatively, the peripheral blood lymphocytes, splenic lymphocytes (B-cells), or lymph node lymphocytes can be employed for fusion with an appropriate myeloma cell to immortalize the genes encoding monoclonal antibodies specific for gp39.

In the present invention, the monoclonal antibodies are partially characterized by their binding to a series of gp39 mutants. The binding avidity (strength of binding) of the antibodies to the mutant gp39 was compared to the binding avidity of the antibody to wild-type gp39. Binding avidity was characterized as poor if the comparison of the binding avidity to a particular mutant was less than 25-30% of the binding avidity to wild-type gp39; a weak or less profound reduction in reactivity was obtained if the binding avidity to a mutant was 25 to 30% to 50-55% of the binding avidity to wild-type gp39; a somewhat reduced reactivity was obtained if the binding avidity to the mutant was 50-55% to 75-80% of the binding avidity to wild-type; and similar or equivalent reactivity was obtained if the binding avidity to a mutant was 75-80% or greater than the binding avidity to a mutant. The antibodies of the present invention were also characterized by their isotype, binding to gp39 by Western blot, ability to suppress B-cell proliferation and ability to suppress immunoglobulin production.

While the invention is described by way of examples using murine monoclonal antibodies, the invention is not so limited and encompasses the use of, for example. human hybridomas (Cote et al. 1983, Proc. Nat'l. Acad Sci. USA 80:2026) or by transforming human B cells (e.g., with Epstein Barr Virus (EBV) *in vitro)* (Cole et al. 1985, in Monoclonal Antibodies and Cancer Therapy, Alan R. Liss, pp. 77-96).

The monoclonal antibodies can be of any of the classes or subclasses of immunoglobulins, such as IgM, IgD, IgA, IgE or subclasses of IgG known for each species of animal. Generally, the monoclonal antibodies can be used intact, or as epitope binding fragments, such as Fv, Fab, or F(ab')₂.

The cell lines of the present invention can find use other than for the direct production of the monoclonal antibodies. The cell lines can be fused with other cells (such as suitably drug-marked human myeloma, mouse myeloma, or human lymphoblastoid cells), to produce hybridomas, and thus provide for the transfer of genes encoding the monoclonal antibodies. Alternatively, the cell lines can be used as a source of the chromosomes, or genes, encoding the immunoglobulins, particularly those regions of the genes encoding the variable or epitope binding regions of the immunoglobulin, which can be isolated and transferred to cells by techniques other than fusion. This can particularly be accomplished by preparing cDNA libraries from mRNA), coding for the immunoglobulin and free of introns, then isolating and placing the DNA into suitable prokaryotic or eukaryotic expression vectors. Methods for the expression vectors can then be used to transform a host for production of immunoglobulin or epitope binding fragments. See, generally, U.S. Nos. 4,172,124. 4,350,683; 4,363,799; 4,381,292; and 4,423,147. See also, Kennet et al. 1980, Munoclonal Antibodies, Plenum Press, New York, and references cited therein.

More specifically, in accordance with hybrid DNA technology, the immunoglobulin or epitope binding fragments of the present invention can be produced in bacteria (See, Boss et al. 1984, Nucl. Acid Res. 12:3791 and Wood et al. 1985. Nature 314:446). For example, the messenger RNA transcribed from the genes coding for the light and heavy chains of the monoclonal antibodies produced by a cell line of the present invention can be isolated by differential cDNA hybridization employing degenerate cDNA probes derived from DNA sequences known to be common to mature immunoglobulin molecules of the parental cell type. The mRNA that does not hybridize will be rich for the messages coding for the desired immunoglobulin chains. As necessary, this process can be repeated to further enhance the desired mRNA levels. The subtracted mRNA composition can then be reverse-transcribed to provide for a cDNA mixture enriched for the desired sequences. The RNA may be hydrolyzed with an appropriate RNase and the ssDNA made double-stranded with DNA polymerase I and random primers, e.g., randomly fragmented calf thymus DNA. The resulting dsDNA can then be cloned by insertion into an appropriate vector, e.g., virus vectors, such as lambda vectors or plasmid vectors (such as pBR322, pACYC 184_{,} etc.). By developing probes based on known sequences for the constant regions of the light and heavy chains, those cDNA clones having the gene coding for the desired light and heavy chains can be identified by hybridization. Thereafter, the genes can be excised from the plasmids, manipulated to remove superfluous DNA, and then introduced in an appropriate vector for transformation of a host and ultimate expression of the gene. Other methods well known in the art can be used to isolate gene sequences which encode immunoglobulin molecules.

In the present application, RNA was isolated and cDNA was generated using PCR techniques with immunoglobulin constant regions as primers. The PCR amplified VH and VL fragments were selected, cloned, and used to determine the nucleotide sequences for the variable regions.

Conveniently, mammalian hosts (e.g., mouse cells) can be employed to process the immunoglobulin chains (e.g., join the heavy and light chains) to produce an intact immunoglobulin; and furthermore, secrete the immunoglobulin free of any leader sequences, if desired. Alternatively, one can use unicellular microorganism for producing the two chains, where further manipulation may be required to remove the DNA sequences coding for the secretory leader and processing signals, while providing for an initiation codon at the 5' terminus of the sequence coding for the heavy chain. In this manner, the immunoglobulins can be prepared and processed so as to be assembled and glycosylated in cells other than mammalian cells.

If desired, each of the chains may be truncated so as to retain at least the variable region, which can then be manipulated to provide for other recombinant binding proteins specific for the gp39 epitope recognized by the parental antibody.

One such recombinant binding protein is a chimeric antibody, in which the variable regions of a parental antibody are recombined with the constant regions of antibodies derived from a different species (e.g., murine variable regions recombined with human constant regions). Typically, the variable region of a monoclonal antibody of the present invention will be joined with the constant region of a human antibody. Chimeric antibodies which are largely human in composition are substantially less immunogenic than murine antibodies.

Another recombinant epitope binding protein is the single chain antibody. In such a construct, sometimes called an sFv, one variable region from both the heavy chain and light chain of the parental antibody are covalently linked through a peptide linker such that the epitope binding region is reformed. Multivalent single chain antibodies comprising heavy and light chain variable regions specific for one or more epitopes of gp39 can also be constructed. See EP 0 610.046 and WO 94/13806 for how such recombinant binding proteins can be constructed.

Still another type of recombinant binding protein is the humanized antibody wherein codons within the framework region of a nonhuman monoclonal antibody are changed through various methods of point mutagenesis to encode amino acid residues to make the murine framework more resemble a human framework region. See EP 0 578,515, EP 0 592,106. Jones et al. 1986, Nature 321:522; Riechmann et al. 1988. Nature 332:323*.* Changes can also be made to the complementarity determining regions (CDR) to make the entire variable region more resemble the surface character of a human antibody. The intention of making the various recombinant binding proteins is to alter either the immunogenicity of the antibody or an accessory activity related to the constant region or other active moiety recombined with the epitope binding region and to retain the gp39 epitope binding specificity of the original parental antibody.

This invention further provides compositions of the monoclonal antibodies and recombinant binding proteins of the present invention. These compositions can comprise the monoclonal antibodies and recombinant binding proteins of the present invention labeled with a detectable marker, for example, a radioactive isotope, enzyme, fluorophor, chromophore, etc. Other compositions can comprise the monoclonal antibodies or recombinant binding proteins of the present invention conjugated or linked to a therapeutic agent, such as a radioisotope, a toxin (i.e., *Pseudomonas exotoxin*), or a chemotherapeutic agent.

Conjugation or linkage of the antibody or recombinant binding protein of the present invention to the detectable marker or therapeutic agent can be by covalent or other chemical binding means. The chemical binding means can include, for example, glutaraldehyde, heterobifunctional, and homobifunctional linking agents. Heterobifunctional linking agents can include, for example, SMPT (succinimidyl oxycarbonyl-α-methyl-α(2-pyridyldition)-tolume, SPDP (N-succinimidyl3-(2-pyridylilithio) propionate and SMCC (succinimidyl-4-(N-mate-imidomethyl) cyclohexane-1-carboxylate. Homobifunctional linking agents can include, for example, DMP (dimethyl pimelimidate), DMA (dimethyl suberinidate) and DTBP dimethyl 3,3'-dithio-bispropionimidate.

Certain protein detectable markers and therapeutic agents can be recombinantly combined with the variable regions of the monoclonal antibodies of the present invention to construct compositions which are fusion proteins, wherein the monoclonal antibody variable regions maintain their binding specificity and the detectable marker or therapeutic agent retain their activity. Recombinant methods to construct these fusion proteins are well known in the art.

Pharmaceutical compositions comprising monoclonal antibody or recombinant binding proteins, either conjugated or unconjugated, are encompassed by the present invention. A pharmaceutical composition can comprise the monoclonal antibody and a pharmaceutically acceptable carrier. For the purposes of the present invention, a "pharmaceutically acceptable carrier" can be any of the standard carriers well known in the art. For example, suitable carriers can include phosphate buffered saline solutions, emulsions such as oil/water emulsions, and various types of wetting agents. Other carriers can also include sterile solutions, tablets, coated tablets, and capsules.

Typically, such carriers can contain excipients such as starch, milk, sugar, types of clay, gelatin, steric acid, or salts thereof, magnesium or calcium sterate, talc, vegetable fats or oils, gums, glycerols, or other known excipients. Such carriers can also include flavors and color additives, preservatives, or other ingredients. Compositions comprising such carriers are formulated by well known conventional means. See Remington's Pharmaceutical Science, 15th Ed., Much Publishing Company, Easton, Pennsylvania (1980).

The monoclonal antibodies and recombinant binding proteins of the present inventions find many *in vitro* and *in vitro* uses. For example, compositions of the present invention can find use *in vitro* to isolate soluble human gp39 and proteins having mutations in human gp39 associated with the human disease, such as X-linked hyper IgM syndrome. The compositions can also find use in diagnostic methods for differentiating between hyper X-linked IgM and CV1.

For diagnostic purposes, the monoclonal antibodies and recombinant binding proteins can be either labeled or unlabeled. Typically, diagnostic assays entail detecting the formation of a complex through the binding of the monoclonal antibody or recombinant binding protein to the human gp39 either at the cell surface or within the activated T cell. When unlabeled, the antibodies and recombinant binding proteins find use in agglutination assays. In addition, unlabeled antibodies can be used in combination with other labeled antibodies (second antibodies) that are specifically reactive with the monoclonal antibody or recombinant binding protein, such as antibodies specific for immunoglobulin. Alternatively, the monoclonal antibodies and recombinant binding proteins can be directly labeled. A wide variety of labels can be employed, such as radionuclides, fluorescers, enzymes, enzyme substrates, enzyme cofactors, enzyme inhibitors, ligands (particularly haptens), etc. Numerous types of immunoassays are well known in the art.

Commonly, the monoclonal antibodies and recombinant binding proteins of the present invention are used in fluorescent assays, where the subject antibodies or recombinant binding proteins are conjugated to a fluorescent molecule, such as fluorescein isothiocyanate (FITC). Because many mutant forms of human gp39 are not transported to the cell surface, T cells are isolated from a subject, activated and then the cells are permeabilized to allow the labeled antibody or recombinant binding protein to penetrate the cell and bind to mutant gp39 wherever it is present in the cell. Binding of the monoclonal antibodies to the intracellular gp39 and an inability to bind a soluble form of CD40 at the cell surface demonstrates the presence of certain point mutations in human gp39 has prevented localization of the gp39 molecule to the cell surface. This can be associated with human disease, such as X-linked hyper IgM. Presence of the bound antibody can be detected by a fluorescence activated cell sorter after excess labeled antibody or binding protein is washed away. Other conventional techniques well known to those skilled in the art can also be utilized.

Kits can also be supplied for use with the compositions of the subject antibodies and recombinant binding proteins for detecting the presence of mutant human gp39 molecules in solution or on activated T cells. Thus, the subject monoclonal antibody and recombinant binding protein compositions of the present invention may be provided, usually in a lyophilized form either individually or in combination with antibodies which bind other specific human gp39 mutants. The antibodies and recombinant binding proteins, which may be conjugated to a label or unconjugated, are included in the kits with buffers, such as Tris, phosphate, carbonate, etc., stabilizers, biocides, inert proteins, e.g., bovine serum albumin, or the like. Generally, these materials will be present in less than about 5% wt. based on the amount of active antibody, and usually present in total amount of at least about 0.001% wt. based again on the antibody concentration. Frequently, it will be desirable to include an inert extender or excipient to dilute the active ingredients, where the excipient can be present in from about 1 to 99% wt. of the total composition. Where a second antibody capable of binding to the monoclonal antibody or recombinant binding protein is employed, this will usually be present in a separate vial. The second antibody is typically conjugated to a label and formulated in an analogous manner with the formulations discussed above.

The monoclonal antibodies, particularly the recombinant binding proteins, single chain antibodies, chimeric antibodies and humanized antibodies, of this invention can also be incorporated as components of pharmaceutical compositions containing an amount of binding protein which is effective, for example, to modulate an immune response (i.e., an autoimmune response or allergic reaction) with a pharmaceutically acceptable carrier. Pharmaceutically accepted adjuvants (buffering agents, dispensing agents) may also be incorporated into the pharmaceutical composition. Such compositions can contain a single monoclonal antibody or recombinant binding protein specific for human gp39. Alternatively, a pharmaceutical composition can contain other biologically active molecules, for example, lymphokines, cytokines, other monoclonal antibodies or fusion proteins (i.e., CD28-lg, CTLA4-lg).

The monoclonal antibodies, recombinant binding proteins and pharmaceutical compositions thereof of this invention are particularly useful for oral or parenteral administration. Preferably, the pharmaceutical compositions can be administered parenterally, i.e., subcutaneously, intramuscularly or intravenously. Thus, this invention provides compositions for parenteral administration which comprise a solution of the monoclonal antibody or recombinant binding protein dissolved in an acceptable carrier, preferably an aqueous carrier. A variety of aqueous carriers can be used, e.g., water, buffered water, maltose, 0.4% saline, 0.3% glycine and the like. These solutions are sterile and generally free of particulate matter. These compositions can be sterilized by conventional, well known sterilization techniques. The compositions can contain pharmaceutically acceptable auxiliary substances as required to approximate physiological conditions such as pH adjusting and buffering agents, toxicity adjusting agents, and the like, for example, sodium acetate, sodium chloride, potassium chloride, calcium chloride, sodium lactate, etc. The concentration of antibody or recombinant binding protein in these formulations can vary widely, i.e., from less than about 0.5%, usually at or at least about 1% to as much as 15 or 20% by weight and will be selected primarily based on fluid volumes, viscosities, etc., preferably for the particular mode of administration selected.

Thus, a typical pharmaceutical composition for intramuscular injection could he made up to contain 1 ml sterile buffered water, and about 50 mg of monoclonal antibody. A typical composition for intravenous infusion could be made up to contain, for example, 250 ml of sterile Ringer's solution, and 150 mg of monoclonal antibody or recombinant binding protein. Actual methods for preparing parenterally administerable compositions will be known or apparent to those skilled in the an and are described in more detail in, for example, Remington's Pharmaceutical Science, 15th Ed., Mech Publishing Company, Easton, Pennsylvania (1980).

The monoclonal antibodies and recombinant binding proteins of this invention can be lyophilized for storage and reconstituted in a suitable carrier prior to use. It will be appreciated by those skilled in the art that lyophilization and reconstitution can lead to varying degrees of antibody activity loss and that use levels may have to be adjusted to compensate.

The pharmaceutical compositions of the present invention find use *in vivo* to inhibit the CD40/gp39 interaction. Blocking this interaction limits both primary and secondary antibody responses to T-cell dependent antigens and antibody production specific for these antigens. Therefore, the monoclonal antibodies, antigen binding fragments, and recombinant binding proteins can be used to inhibit the activation of B cells, modulating or inhibiting autoimmune disease (i.e., psoriasis, rheumatoid arthritis, systemic lupus erythematosis, diabetes mellitus, etc.), allergic responses, organ rejection or graft-versus-host disease. The compositions can also be used for imaging tumors which express gyp39, when labeled with a detectable marker. When conjugated with a therapeutic agent or as a fusion protein with a therapeutic agent, the monoclonal antibodies, antigen binding, fragment or recombinant binding proteins, can also he used to target the therapeutic agent to tumor cells.

The pharmaceutical compositions of the present invention find use *in vivo* to inhibit the CD40/gp39 interaction. Blocking this interaction limits both primary and secondary antibody responses to T-cell dependent antigens and antibody production specific for these antigens.

This invention is illustrated in the Examples which follow. This Example section is provided to aid in understanding the invention but is not intended to, and should not be construed to, limit in any way the invention as set forth in the claims which follow.

### EXAMPLE 1

### Generation and Initial Characterization of Monoclonal Antibodies Specific for gp39 - Fusion 1

### A. Immunization

A six-to-eight-week-old female BALB/c mouse was initially immunized intraperitoneally with 30 µg of a gp39-CD8 fusion protein (sgp39, Hollenbaugh et al. 1992, EMBO J. 11:4313-4321) in a volume of 100 µl of complete Freund's adjuvant. Approximately two weeks later, the mouse was similarly injected except the vehicle used was incomplete Freund's adjuvant. Three weeks later the mouse received an intravenous pre-fusion booster injection with 23 µg of sgp39 in a volume of 100 µl of phosphate buffered saline (PBS).

### B. Fusion

Three days after the pre-fusion booster, the spleen and lymph nodes (axillary, popliteal, inguinal, and mesenteric) were harvested. These were cut into small pieces with a scalpel and then gently pressed between the frosted glass ends of glass microscope slides in the presence of incomplete Iscove's medium (Iscove's modified Dulbecco's medium supplemented with penicillin and streptomycin to a final concentration of 100 U/ml and 100 µg/ml, respectively) to loosen lymphocytes from connective tissue. The suspension was gently pipetted to further loosen cells from each other and then the suspension was passed through a cell strainer (Falcon 2350) to remove clumps of connective tissue debris. The cell suspension was washed twice by centrifugation at 200 g for 10 minutes followed by resuspension of the cell pellet in incomplete Iscove's medium. After washing, a viable total leukocyte count was determined by trypan blue exclusion.

The fusion procedure was based on the methods of Lane et al. 1986 (Methods Enzymol. 121:183-192). Myeloma cells (X63-Ag8.653, Kearney et al. 1979, J. Immunol. 123:1548-1550) in log phase growth were washed twice by centrifugation at 200 g for 5 min. followed by resuspension of the cell pellet in incomplete Iscove's medium. The cells were then combined with the washed leukocytes in a 50 ml plastic centrifuge tube at a 1:4 ratio of myeloma cells to leukocytes and centrifuged at 200 g for 10 minutes. Following aspiration of the medium, the tube was gently tapped until the cell pellet became resuspended in the remaining small amount of medium. After incubation of the tube in a 37°C water bath for 1 min., 1.5 ml of freshly prepared 37°C polyethylene glycol-dimethyl sulfoxide solution [50% (w/v) Kodak 1450 polyethylene glycol, 5% (v/v) dimethyl sulfoxide, and 45% (v/v) phosphate buffered saline containing no calcium or magnesium, pH 8.0] was added to the cells over a 45 second period with constant swirling of the tube in a 37°C water bath. The fusion mixture was then diluted with 50 ml of 37°C complete Iscove's medium ((incomplete Iscove's medium supplemented with an extra 2 mM L-glutamine and 15% (v/v) fetal calf serum (FCS)) over a 90 second period as follows: 3 ml over the first 30 seconds. 9 ml over the next 30 seconds, and the remainder over the last 30 seconds. The tube was incubated at 37°C for 10 minutes after which it was centrifuged at 200 g for 5 minutes, the supernatant aspirated, and the cells resuspended in 120 ml of hybridoma medium [complete Iscove's medium supplemented with hypoxanthine (1 x 10⁻¹ M final concentration), aminopterin (4 x 10⁻⁷ M final concentration), thymidine (1.6 x 10⁻⁷ M final concentration), and 10% (v/v) hybridoma cloning factor (Boehringer Mimnheim)]. The cell suspension was plated into six 96-well cell culture plates (200 µl)/well) resulting in a plating density of 243,000 total cells (pre-fusion) per well. Wells were fed on days 3 and 5 post fusion by replacement of half the supernatant with fresh hybridoma medium and assayed for anti-gp39 specific antibody on day 8.

### C. Screening

Supernatants from cell culture wells having growing cells were initially screened for reactivity with the sgp39 protein immunogen as follows. Dynatech Immulon 2 EIA plates were coated with 1 µg/ml (100 µl/well) of antibody 53-6 (rat anti-mouse CD8, ATCC TIB 105) in 0.05 M sodium carbonate/sodium bicarbonate buffer, pH 9.6. The plates were sealed and incubated overnight at 4°C. All subsequent steps were performed at room temperature. Coating agent was removed and wells blocked with blocking reagent [(specimen diluent (Genetic Systems Corp.. Seattle, WA) diluted 1:10 in deionized water)] for one hour. Blocking reagent was removed and COS cell supernatant containing sgp39 protein, diluted 1:4 in complete Iscove's medium containing 2% FCS (2% FCS-Iscove's) was added (100 µl/well) and incubated for one hour. Fusion protein was removed and the wells were washed once with 200 µl of PBS-Tween (PBS containing 0.05% (v/v) Tween 20). Cell culture supernatant was then added (50 µl/well) and incubated for one hour. The cell culture supernatant was removed and the wells washed once with PBS-Tween prior to the addition of horseradish peroxidase (HRP) labeled goat anti-mouse IgG (Jackson immunological Laboratories) diluted 1:100,000 in blocking reagent followed by one hour incubation. Excess labeled antibody was removed and the wells were washed three times with PBS-Tween. This was followed by the addition of 100 µl/well tetramethylbenzidine (Genetic Systems Corp.) diluted 1:100 in 0.1 M citrate buffer, pH 5.5, containing 0.015% of a 30% H₂O₂ solution. Plates were incubated for 15 minutes and the reaction stopped by the addition of 3N sulfuric acid (50 µl/well). Optical density was measured at 450/630 nm on a Bio-Tek Instruments EL312 Microplate Reader.

Those cell culture supernatants found to be positive for binding to sgp39 where then tested for binding to CD72-CD8 fusion protein (sCD72) to assess for antibodies specific for gp39 rather than the murine CD8 portion of the fusion protein. Description of the construction of the chimeric gene encoding sCD72 and expression of the fusion protein transiently in COS cells are described in Hollenbaugh et al., 1992 (incorporated by reference herein in its entirety). The ELISA assay for binding to sCD72 was identical to that described above for sgp39 except that undiluted COS cell supernatant containing sCD72 was used in place of sgp39.

All supernatants that were reactive with the sgp39 and not with sCD72 were then tested for their ability to inhibit the binding of CD40-Ig fusion protein to sgp39. Briefly, Dynatech Immulon 2 E1A plates were coated with antibody 53-6 as described above. The wells were blocked and washed as above and COS cell supernatant containing sgp39 diluted 1:4 in 2% FCS-Iscove's was added (100 µl/well) and incubated for 1 hour. The sgp39 was removed and the plates were washed with 200 µl of PBS-Tween. Culture supernatants were then added (50 µl/well) and incubated for I hour, removed and the wells washed once with PBS-Tween. Purified CD40-Ig fusion protein (EP 555880) was then diluted to 2 µg/ml in 2% FCS-Iscove's, added to all wells (50 µl/well) and the plates incubated for one hour. Excess fusion protein was removed and the wells were again washed once with PBS-Tween prior to adding HRP labeled goat anti-human IgG (Jackson Immunological Laboratories) diluted 1:10,000 in blocking reagent (50 µl/well). After a one-hour incubation at room temperature, HRP labeled reagent was removed and the plates washed three times with PBS-Tween. Disclosure of bound HRP labeled reagent and measurement of resulting optical density was as described in EL1SA assays described above.

### D. Cloning

A number of wells were found which contained antibody specific for gp39 and which inhibited the interaction of gp39 with its ligand CD40 in an ELISA. The cells growing in these wells were then cloned and subjected to additional Screening criteria

Cloning was initiated with a "mini-cloning" procedure in which cells from designated master wells were first plated at a density of 10 or 20 cells per well in 96-well flat-bottom cell culture plates. One or two plates were established for each master well in a culture medium of complete Iscove's medium supplemented with 10% (v/v) hybridoma cloning factor (cloning medium) at a volume of 200 µl/well. Cells were cultured for 7 to 8 days at which time supernatants were again tested for gn39 reactivity and ability to inhibit the binding of CD40-Ig to gp39-CD8 fusion protein by ELISA (described above). From the wells in each miniclone set that satisfied these criteria, one well was cloned. Cells were removed from the selected well and diluted to a concentration in cloning medium that would provide a calculated density of one cell for every two wells. The cells were plated in two haft-area 96-well cell culture plates (Costar) in a volume of 100 or 150 µl/well.

After four or five days of culture, the wells were examined on an inverted microscope and those wells containing a single clone were marked. After a further three-four days of culture, supernatants from all wells were tested for g39 reactivity (gp39-CD8 fusion protein EL1SA, described above) and ability to inhibit the binding of CD40-Ig to gp39-CD8 by ELISA (described above). Supernatants from wells that were reactive with gp39-CDB, blocked the interaction of CD40-Ig with gp39-CD8, and came from wells marked as containing single clones were further examined for their ability to bind to a Jurkat T cell line that constitutively expressed gp39 on its surface (BMS-10, R. Mittler, Bristol-Myers Squibb) and to block the binding of CD40-Ig fusion protein to these cells. Clones that satisfied the latter two criteria were selected for further study.

Binding of antibody to BMS-10 cells was determined by fluorescent cell analysis. Briefly, 250,000 BMS-10 cells were counted, added to each tube, and centrifuged at 250 g for 5 minutes. Culture medium was aspirated and 100 µl of each supernatant containing antibody reactive with gp39-CD8 by ELISA was added to a tube. Controls included culture medium only or culture medium containing a negative control mouse monoclonal antibody. The mixture was incubated on ice for 30 minutes and then 2 ml of 2% FCS-Iscove's was added. The tubes were centrifuged at 250 g for 5 minutes and the supernatant was removed. FITC labeled F(ab')₂ goat anti-mouse IgG F(ab')₂ (Jackson Immunological Laboratories)) was diluted 1:500 in 2% FCS-Iscove's and 100 µl added to each tube. After a 30 minute incubation on ice, cells were washed twice with I ml of 2% FCS-Iscove's and resuspended in 250 µl of 2% FCS-Iscove's prior to analysis on a Becton Dickinson FACScan™.

Assessment of an antibody's ability'to block the binding of CD40-Ig to BMS-10 cells used the above procedure except that after washout of unbound anti-gp39 antibody, CD40-Ig, diluted to 20 µg/ml in 10% FCS-Iscove's, was added to each tube. 100 µl/tube. After a 30 minute incubation on ice, 2 mls of 2% FCS-Iscove's was added to each tube, the tubes centrifuged for five minutes at 350 g, and the supernatants aspirated to remove unbound CD40-Ig. Instead of an FITC-labeled anti-mouse Ig reagent, an appropriately diluted PE- or FITC-labeled F(ab')₂ goat anti-human IgG (Jackson Immunological Laboratories) was then added to each tube to detect bound CD40-Ig. Otherwise, the assay was completed and the cell analyzed as described above.

Following the procedures outlined above, a total of 23 mouse anti-human gyp39 monoclonal antibodies were derived. Each of the monoclonal antibodies was isotyped to identify its IgG subclass and their ability to recognize gyp39 was further characterized. An analysis of epitope specificity differences between the monoclonal antibodies was also carried out, as was the ability of the antibodies to inhibit T cell dependent B cell proliferation and immunoglobulin production.

### EXAMPLE 2

### Generation and Initial Characterization of Rat Monoclonal Antibodies Specific for gp39- Fusion 5

### A. Immunization

A four-week-old female Lewis rat was immunized with 50 µg of a gp39-CD8 fusion protein (Hollenbaugh et al.) suspended to a total of 400 µl in Ribi adjuvant (Ribi Immunochem) as per manufacturer's instructions. Of this volume, 200 µl was administered interperitoneally (IP) and the remaining volume was equally split between two subcutaneous sites. Three weeks later, this animal was re-imnmnized IP with 300 µl of PBS containing 50 µl of gp39-CD8 fusion protein. One month later, the rat was again immunized IP with 300 µl of PBS containing 30 µg of gp39-CD8. Three and one-half weeks later, this rat received an intravenous (IV) prefusion booster injection of 30 µg of gp39-CD8 in a volume of 300 µl PBS.

### B. Fusion and Screening

Three days later, harvest, preparation, and fusion of the rat spleen and lymph node cells to mouse myeloma cells were performed as for fusion 1 described in Example 1 with the following modifications. The rat leukocytes were fused with a variant of the X63-Ag8.653 mouse myeloma cell line termed H10 which had been transformed with the G⁴¹⁸ resistance gene. The cell suspensions from each fusion were seeded into seven 96-well cell culture plates at a plating density of 236,000 total cells (pre-fusion) per well and cultured in hybridoma media supplemented with a final concentration of 0.25 mg/ml geneticin.

Supernatants from cell culture wells in fusion 39-5 were screened for specific reactivity with gp39 using the gp39-CD8 and CD72-CD8 fusion protein ELISA as described in Example 1 with the following two modifications. Murine anti-murine CD8 monoclonal antibody produced from the 116-13.1 hybridoma (ATCC HB 129) at a concentration of 5 µg/ml, was used as the capture antibody for the fusion proteins instead of the 53-6 antibody and HRP labeled murine anti-rat IgG (Fc specific) (Jackson Immunological Laboratories) at a 1:20,000 dilution, was used as a tracer antibody to detect bound anti-fusion protein antibody instead of HRP goat anti-mouse IgG. Four wells were found to contain antibody specific for gp39-CD8 and not CD72-CD8 fusion protein. Of these, antibody is only one (39-5.6E9) was found to stain the gp39+ cell line BMS-10 when examined by flow cytometry as described earlier. Supernatant from this well was subsequently determined to completely block the binding of CD40-Ig to gp39-CD8 using the blocking ELISA described earlier and to completely inhibit the binding of CD40-Ig to BMS-10 cells when assessed by flow cytometry. A clonal cell line possessing all the above characteristics was obtained through the mini-cloning/cloning process described earlier.

### EXAMPLE 3

### Generation and Initial Characterization of Monoclonal Antibodies Specific for gp39- Fusion 7

### A. Immunization

A six-to-eight-week-old female BALB/c mouse was initially immunized subcutaneously at four sites with a total of 30 µg of a gp39-CD8 fusion protein in complete Freund's adjuvant. Approximately two and five weeks later, this mouse was similarly injected with 30 µg and 25 µg, respectively, of gp39-CD8 except that the vehicle for antigen was incomplete Freund's adjuvant. Five months after initial immunization, this mouse was injected intraperitoneally with 10 µg of fusion protein in incomplete Freund's adjuvant. Two weeks later, the mouse received an intravenous pre-fusion booster injection of 30 µg of gp39-CD8 fusion protein in PBS.

### B. Fusion and Screening

Three days later, harvest, preparation, and fusion of the mouse spleen and lymph node cells to mouse myeloma cells was performed as for fusion 39-1 except that only 1 ml of polyethylene glycol was used to fuse the cells. The cell suspension resulting from this fusion was seeded into 10 96-well cell culture plates at a plating density of 183,000 total cells (pre-fusion) per well. Wells were fed on days 3 and 6 post fusion by replacement of half the supernatant with fresh hybridoma medium and assayed for anti-gp39 specific antibody on day 9.

Supernatants were initially screened for anti-gp39 specificity in an ELISA based cell binding assay. Falcon or Costar 96-well flat bottom plates were coated with 3.5 µg/cm² of Cell-Tak (Collaborative Biomedical Products diluted in 0.1 M sodium bicarbonate, pH 8.0. Plates were incubated at room temperature for 30 minutes. Unbound Cell-Tak was aspirated and the wells washed twice with 150 µl/well of glass distilled water. BMS-10 cells were centrifuged and resuspended to a concentration of 2 x 10⁶ cells/ml in serum-free Iscove's medium. Fifty µl of this cell suspension was added to each well and the plates centrifuged for 5 minutes at 250 g. Plates were then incubated at room temperature for 30 minutes after which the medium was aspirated from the wells using an eight channel manifold (Drummond). Culture supernatants were then replica plated onto the assay plates, 50 µl/well, and the plates incubated for 1 hour. Supernatants were aspirated and the plates washed once with 150 µl/well of PBS containing 1% FCS. HRP labeled rat anti-mouse IgG (Zymed) diluted in PBS containing 5% FCS was added, 50 µl/well. After a one-hour incubation at room temperature, HRP labeled reagent was removed and the plates washed three times with PBS containing 1% fetal calf serum. Disclosure of bound HRP labeled reagent and the measurement of resulting optical density was as described in other ELISA assays detailed above.

As a secondary screen, supernatants from positive wells in the BMS-10 cell ELISA above were assayed for reactivity to sup39 and sCD72 using the respective fusion protein ELISAs described earlier. In this assay, HRP labeled rat anti-mouse IgG (Zymed) replaced the goat anti-mouse IgG used in earlier described assay. Confirmation of specific reactivity with gp39 positive BMS-10 cells was then performed using indirect immunofluorescence and FACS analysis as described earlier. Supernatants were also tested for their ability to inhibit CD40-Ig binding to sgp39 using the blocking ELISA described earlier. Supernatants were further analyzed as to their isotype using the gp39-CD8 ELISA except for one modification. Each supernatant was tested in quadruplicate and bound anti-gp39 antibody was then traced with four different HRP labeled anti-mouse isotype-specific reagents (Zymed, rat anti-mouse IgG1, IgG2a, or IgG2b, #04-6120, 04-6220, and 04-6320, respectively, and rabbit anti-mouse IgG3, #61-0420). This overall analysis identified one well that contained antibody specific for cell surface expressed gp39, blocked the binding of CD40-Ig to gp39-CD8 and was of the IgG2a isotype. Appropriate antibody producing cells from wells designated 39-73El2. 39-7.7.7G4, and 39-7.4Cl were minicloned and cloned as described earlier.

### EXAMPLE 4

### Generation and Initial Characterization of Monoclonal Antibodies Specific for gp39-Fusion 9

### A. Immunization

A six-to-eight-week-old female BALB/c mouse was initially immunized subcutaneously at four sites with a total of 30 µg of gp39-CD8 fusion protein in complete Freund's adjuvant. Two, five, 28 and 30.5 weeks later, the animal was similarly immunized with 30 µg, 25 µg, 25 µg, and 25 µg, respectively, of gp39-CD8 fusion protein in incomplete Freund's adjuvant. Three weeks later, the animal received an IV pre-fusion booster injection of 35 µg g39-CD8 in PBS.

### B. Fusion and Screening

Three days later, harvest, preparation, and fusion of the mouse spleen and lymph node cells to X63-Ag8.653 mouse myeloma cells was performed as for fusion 39-1 except that the ratio of myeloma cells was performed as for fusion 39-1 except that the ration of myeloma cells to leukocytes was 1:3 instead of 1:4. The cell suspension from this fusion was plated into 15 96-well culture plates at a density of 187,000 total cells (pre-fusion) per well.

Supernatants from cell culture wells in fusion 39-9 were screened for anti-gp39 antibody using the BMS-10 cell binding ELISA described earlier. All positive supernatants in this screen were then examined for their ability to block the binding of CD40-Ig to gp39-CD8 using the previously described blocking ELISA. Numerous wells positive for both criteria were noted.

In order to identify those wells that contained antibody with a gp39 epitope specificity different form that of earlier identified anti-gp39 monoclonal antibodies. each of the CD40-Ig locking, BMS-10 positive supernatants were screened by ELISA on the series of gp39 mutant proteins described earlier for the epitope analysis of antibodies from fusion 1. this screen identified several wells, including 39-9.27. 39-9.68, 39-9.246, and 39-9.274, for which there was not a significant loss of binding to any of the mutants examined.

In addition" one well designated 39-9. II demonstrated significantly reduced binding to the K143/A and especially the N180/A mutants, a pattern not seen previously. It was also shown in this assay that each of these supernatants recognized the native sgp39 and not the negative control sCD72 fusion protein. An appropriate antibody secreting clonal cell line for each of these five wells was obtained by limited dilution cloning as described earlier. Subsequent flow cytometry analysis of the cloned monoclonal antibody from each of the cell lines showed that they all specifically reacted with the BMS-10 cell line and all were capable of blocking the interaction of CD40-Ig with this cell line. Each of these monoclonal antibodies was isotyped as described subsequently and all except for 39-9.36, which was a murine IgG2b, were found to be murine IgGI.

### EXAMPLE 5

### Characterization of the anti-gp39 Monoclonal Antibodies

### A. Isotyping

Each of the murine monoclonal antibodies obtained by the above procedures was isotyped to identify its IgG subclass using an Isotype Ab-Stat Kit™ (SangStat Medical Corporation, Menlo Park, CA) or ISOStrip™ kit (Boehringer Mannheim) as per manufacturer's instructions. The isotype of the rat monoclonal antibody obtained in fusion 5 (39-5.6E9) was established using the gp39-CD8 fusion protein ELISA described above except that horseradish peroxidase labeled monoclonal antibodies specific for rat IgG1, IgG2a, IgG2b, and IgG2c (Zymed Laboratories, San Francisco, CA) at a dilution of 1: 1,000 were individually employed as tracer antibodies. The only tracer antibody which bound 39-5.6E9 was the anti-rat IgG2a antibody indicating that this monoclonal antibody was an IgG2a.

The monoclonal antibodies obtained in fusion 7 were analyzed as to their isotype using the sgp39 ELISA except for one modification. Each supernatant was tested in quadruplicate and bound anti-gp39 antibody was then traced with four different HRP labeled anti-mouse isotype-specific reagents (Zymed, rat anti-mouse IgG1, IgG2a, or IgG2b, respectively, and rabbit anti-mouse IgG3). The isotypes of the monoclonal antibodies of the present invention are shown in Table 1.

### B. Western Blot and Immuneprecipitation

Western blot evaluation was performed by two different procedures. In one, 1.5 µg of purified sgp39 fusion protein in 300 µl of loading dye [250 mM Tris, 0.002% (v/v) bromphenol blue, 40% (v/v) glycerol, pH 6.8, 15 µl of 20% SDS, 10 µl of 2-mercaptoethanol] was electrophoresed on a 12% SDS-polyacrylamide gel at 150 V for 1 hour. The separated proteins were transferred to nitrocellulose paper with a Bio-Rad mini-blot transfer apparatus according to manufacturer's instructions. After transfer, the nitrocellulose was allowed to dry at room temperature and then each lane was cut from the sheet as wide vertical strips which were placed individually into the wells of a Bio-Rad shallow well unit. The strips were incubated in 10 ml of a blocking solution of Tris Buffered Saline containing 5% (w/v) non-fat dry milk (TBS-T) for 2 hours at room temperature on a flat plane rocker. After incubation, the blocking solution was aspirated and the strips were rinsed twice with TBS-T.

Anti-gp39 monoclonal antibody was diluted to a concentration of about 12 µg/ml in TBS-T and 3 ml of antibody solution was added to each strip, one antibody per strip, for 2 hours at room temperature with rockling. Excess antibody solution was aspirated from each well and the strips were washed five times with 10 ml TBS-T. After washing, 10 ml of a 1:3,000 dilution of HRP goat anti-mouse Ig (Tago) in TBS-T was added to each well. The strips were incubated for two hours at room temperature and then washed five times as described above.

Detection of bound HRP conjugated antibody was performed using ECL detection reagents (Amersham) according to manufacturer's instructions. The detection solution was aspirated and excess liquid on the strips was removed by touching the end of the strips onto a paper towel. The strips were aligned inside a plastic page protector and the protector sealed. The sealed protector was then exposed to autoradiography film for variable time periods (1 second to 15 minutes and the films subsequently processed.

In a second procedure, 200 µl of spent supernatant from COS cells transfected with sgp39 was diluted with 25 µl of loading dye, heated to 100°C for 5 minutes, cooled on ice, and electrophoresed on a 10% SDS-polyacrylamide gel. Separated protein was transferred to a Bio-Rad PVDF™ membrane using a Hoeffer semi-dry transfer apparatus according to manufacturer's instructions. After the separated proteins were transferred to the membranes, the membranes were allowed to dry at room temperature and then the procedure as described above was followed to stain the membranes.

The anti-gp39 antibodies were also tested for the ability to immunoprecipitate gp39 from either transfected COS cells or activated T cells. Briefly, COS cells were transfected with a cDNA encoding human gp39 by the DEAE-dextran procedure using ten 150 mm plates at approximately 70% confluency. The following day, the COS cells were trypsinized and replated in eight T-150 cm² flasks. Media was removed after incubating overnight and the cells were washed once with modified Eagle's medium without cysteine or methionine (Gibco Select-amine Kit) and 20 ml of fresh cysteine/methionine-free media containing 0.02 mCi/ml Tran³⁵S label (ICN, Costa Mesa, CA) were then added and the cells were incubated overnight. The following day, the media was removed and the cells were rinsed once with PBS and 2 ml of lysis buffer (50 mM Tris, 150 mM NaCl, 1% NP-40, 0.25% deoxycholate) containing 1 nM phenylmethyl sulfonylfluoride (PMSF) and 25 µg/ml aprotinin was added to each flask. The flasks were placed on ice for 10 minutes, after which the buffer was removed. Aliquots were prepared and centrifuged in a microfuge at 4°C at maximum speed for two minutes. The supernatants were pooled and stored at -70°C prior to immunoprecipitation.

Immunoprecipitation was carried out by thawing the transfected COS cell lysates on ice and dividing the total volume into 10 aliquots. To eight tubes, 10 µg of an anti-gp39 antibody was added, while one tube received CD40 Ig as a positive control and one received no precipitating agent as a negative control. Samples were incubated on ice for 4 hours, after which 100 µl of Protein G Sepharose FF™ (Pharmacia) was added to each tube. The tubes were incubated on ice for 1 hour with mixing every 10 to 15 minutes. Samples were pulse spun in a microfuge and the supernatant was discarded. The pellets were washed by resuspension and pelleting three times with cold lysis buffer, then once with cold PBS. Following the last wash, 30 µl of SDS loading buffer containing β-mercaptoethanol was added to each tube. Samples were heated at 95°C for 5 minutes, pulse spun and the supernatant loaded on a 12% SDS-polyacrylamide gel. At the completion of electrophoresis, the gel was placed in 10% methanol, 10% acetic acid in water for two hours. The gel was then placed in Amplify™ fluorographic agent (Amersham) containing 10% glycerol for 15 minutes. The gel was dried on Whatman 3M paper under vacuum at 80°C for 45 minutes and exposed to X-ray film at -70°C for 1 to 7 days. Results of the assay are summarized in Table 1. Positive immunoprecipitations were indicated by the presence of a band at the same molecular weight as the CD40-lg control.

Radioimmunoprecipitation of gp39 from activated human peripheral blood T cells was carried out as follows. Fresh heparinized whole blood was diluted 1 : 1 with PBS and 40 ml was overlayed onto 10 ml of Lymphocyte Separation Media™ (Organon Teknika) as described above. The sample was centrifuged for 30 minutes at 220 g. Isolated lymphocytes were washed with PBS and resuspended in modified Eagle's medium lacking cysteine and methionine containing 10% dialyzed fetal bovine serum and 0.02 mCi/ml Tran ³⁵S Label™ at a final cell density of 3 x 10⁶ cells/ml. Cells were activated by the addition of PMA (10 ng/ml) and ionomycin (1 µg/ml) for nine hours, after which the cells were pelleted, the media removed and the cells lysed with lysis buffer containing PMSF and aprotinin. The cells were incubated with lysis buffer for 10 minutes on ice prior to transferring the sample to microfuge tubes and centrifuging for 2 minutes at 4°C. The supernatants were pooled and stored at -70°C until further processing. The precipitation was carried out as described above for transfected COS cells and the results are summarized in Table 1.

**TABLE I**

| Summary of Anti-Human gp39 mAbs | | | | | |
|---|---|---|---|---|---|
| mAb | Isotype | Binding to gp39+ Jurkat Cells | Inhibit Binding of CD40-Ig to gp39+ Jurkat Cells | Western Blot sgp39 | Radioimmune Precipitation |
| 39-1.3 | IgG1 | + | + | - | + (a) |
| 39-1.7 | IgG2b | + | + | - | + (a.b) |
| 39-1.21 | IgG1 | + | + | - | ND |
| 39-1.25 | IgG1 | + | ND | - | ND |
| 39-1.26 | IgG1 | + | + | - | + (a.b) |
| 39-1.29 | IgG2a | + | + | + | + (a) |
| 39-1.37 | IgG1 | + | + | - | ND |
| 39-1.52 | IgG1 | + | + | + | ND |
| 39-1.59 | IgG1 | + | + | - | ND |
| 39-1.61 | IgG1 | + | + | + | + (b) |
| 39-1.63 | IgG1 | + | + | - | ND |
| 39-1.77 | IgG1 | + | + | + | + (a.b) |
| 39-1.93 | IgG1 | + | + | + | ND |
| 39-1.106 | IgG1 | + | + | + | + (b) |
| 39-1.109 | IgG1 | + | + | + | ND |
| 39-1.122 | IgG2b | + | + | - | ND |
| 39-1.123 | IgG1 | + | + | - | ND |
| 39-1.124 | IgG1 | + | + | + | ND |
| 39-1.128 | IgG2b | + | + | - | + |
| 39-1.132 | IgG2b | + | + | - | + |
| 39-1.134 | IgG1 | + | + | + | + |
| 39-1.138 | IgG1 | + | + | - | ND |
| 39-1.156 | IgG1 | + | + | + | ND |
| 39-7.3E12 | IgG2a | + | + | - | ND |
| 39-5.6E9 | IgG2a | + | + | - | ND |
| 39-7.7G4 | IgG2a | + | + | + | ND |
| 39-9.27 | IgG1 | + | + | + | ND |
| 39-7.4Cl | IgG2b | + | + | + | ND |
| 39-9.68 | IgG2b | + | + | + | ND |
| 39-9.246 | IgG1 | + | + | + | ND |
| 39-9.11 | IgG1 | + | + | - | ND |
| 39-9.274 | IgG1 | + | + | + | ND |

| | | | | | |
|---|---|---|---|---|---|
| a - radioimmune precipitated from gp39 transfected COS cells b - radioimmune precipitated from activated human T cells ND - not done | | | | | |

### C. Examination of Binding of Anti-Human gp39 Monoclonal Antibodies with Activated and Non-Activated Normal Human T Cells.

Reactivity of the various anti-human gp39 monoclonal antibodies with activated and non-activated normal human T cells was assessed by indirect immunoflourescence followed by FACS analysis. Human blood mononuclear cells (PBMCs) were isolated by diluting whole blood 1 : 1 with PBS, overlaying 25 ml onto 10 ml of Lymphocyte Separation Medium (LSM, Organon Teknika) and centrifuging for 25 minutes at 450 g. Cells at the interface were collected and washed once in PBS. T cells were isolated by incubating the PBMCs with 150-fold AET-SRBC (sheep red blood cells treated with 0.143 M 2-aminoethylisothiouronium bromide (Sigma)) for 5-10 minutes on ice. E-rosette positive T cells (E'-T cells) were separated from the remaining cells by underlaying with cold LSM and centrifuging at 450 g for 25 minutes. The pellet (containing rosetted T cells) was collected and the sheep red blood cells were lysed with 0.83% ammonium chloride for 5 minutes at room temperature. Resulting T cells were washed once in 2% FCS-Iscove's and incubated overnight in 10% FCS-Iscove's at 1-3 x 10⁶ cells/ml in a humidified 37° C/6% CO₂ incubator. T cells were then activated by the addition of 10 ng/ml phorbol 12-myristate 13-acetate (PMA) (Sigma) and 1 µg/ml ionomycin (Sigma) and further incubation of the cells for 5-6 hours. A portion of the T cells did not receive PMA and ionomycin but were incubated for a further 5-6 hours and are referred to here as non-activated T cells. Indirect immunofluorescence and FACS analysis of the anti-human gp39 mAbs on these activated and non-activated T cells was performed as described earlier for FACS analysis of anti-gp39 antibodies on BMS-10 cells except that FITC labeled goat anti-mouse IgG (Becton Dickinson) was used as the second step reagent Additionally, a murine anti-human CD69 monoclonal antibody (Becton Dickinson) was used as a positive control for activation of the T cells. In this manner all the anti-gp39 mAbs were examined. All were found to stain activated T cells and were further shown to be completely unreactive with non-activated T cells.

### EXAMPLE 6

### Construction of gp39 Mutant Fusion Proteins

### A. Selection of gp39 Residues Targeted for Substitution:

Residues targeted for mutagenesis on gp39 were selected on the basis of a previously derived comparative protein model of the gp39 extracellular region (Aruffo et al., 1993 Cell 72:291-300), on the basis of structure-based sequence alignments of gp39 vs TNF-β and on the basis of the reported crystallographic contacts in the TNF-β/TNFR complex structure (Banner et al., 1993 Cell 73:43 1-445). Computer graphics analysis of the gp39 model was carried out using Insight II™ (BIOSYM Technologies Inc., San Diego, CA) on a Silicon Graphics Indigo™ workstation. Sequences were initially aligned using the GCG programs (Genetics Computer Group Inc., Madison. WI) and manually modified taking three-dimensional information and constraints of the TNF-β (Eck et al., 1992 J. Biol. Chem. 267:2119-2122) and the TNF-β/TNFR crystal (Banner et al., *supra*) structures into account.

### B. Construction of gp39 Mutants

Amino acid substitutions and silent mutations for diagnostic restriction enzyme cleavage sites were introduced into cDNA fragments encoding the extracellular domain of gp39 by using an overlay extension PCR protocol (Ho et al., 1989. Gene 77:51-59). The fusion genes encoding the mutant soluble gp39 (sgp39) proteins were prepared by subcloning the PCR amplified gp39 extracellular domain mutants into a mammalian expression vector containing a cDNA fragment encoding the extracellular domain of murine CD8 (Lyt 2a) (Hollenbaugh et al., 1992. EMBO J. 11 :4313-4331). The forward and reverse PCR primers used for the gp39 constructs have been previously described (Hollenbaugh et al., *supra*).

The PCR primers used for the gp39 mutants are: 
E129/A
5' AATCCTCAAAATGCGGCACATGTGATCAGTGCGGCC
AGCA GTAAAACAACA 3'                    SEQ ID. #1,
 
S131/A-T135/A
5' CAAAATGCGGCACATGTGATCAGTGAGGCCGCCAG
TAAAA CAGCATCTGTGTTACAGTGGGCT 3'
SEQ ID. #2.
 
K143/A
5' AGTAAAACAACATCTGTGCTGCAGTGGGCTGAAGCA
GGAT ACTACACCATGAGC 3'                    SEQ ID. #3,
 
Y145/A
5' AGTAAAACAACATCTGTGCTGCAGTGGGCTGAAAAA
GGAG CCTACACCATGAGCAACACT 3'          SEQ ID. #4,
 
N180/A
5' CAAGTCACCTTCTGTTCCGCTCGGGAGGCTTCGAGTC
AAG CTCCA 3'                                                    SEQ ID. #5,
 and 
F201/A-E202/A
5' AGCCTCTGCCTAAAGTCCCCCGGGAGAGCCGCGAGA
ATCT TACTCAGAGCT 3'                                    SEQ ID. #6.
 The corresponding reverse primers are the reverse compliment of the sequences listed above. Base changes that encode the alanine are shown in bold type. The diagnostic restriction sites added or deleted are underlined.

### C. Production and Characterization of Wild-Type and Mutant gp39 Proteins.

Wild-type and mutant ,gp39 proteins were produced from transiently transfected COS cells as described elsewhere (Hollenbaugh et al., 1992 supra: Noelle et al, 1992. Proc. Nat'l. Acad. Sci. USA 89:6550-6554). COS cells were transfected using DEAE-dextran. Forty-eight hours post transfection, culture supernatant containing soluble wild-type gp39 or soluble mutant gp39 were harvested and used in assays for monoclonal antibody binding and determination of epitope specificities on human gp39.

### D. Enzyme-linked Immunoassay for Monoclonal Antibody Binding to gp39 Mutant Proteins.

Results of Western blot assays indicated that at least two different epitopes on human gp39-CD8 fusion protein were being recognized. Monoclonal antibodies 39-1.29, 39-1.52, 39-1.61, 39-1.77, 39-1.93, 39-1.106, 39-1.109, 39.1.124, 39-1.134, 39-1.156, 39-7.7G4, 39-9.274, 39-9.27, 39-7.4Cl, 39-9.68 and 39-9.246, were found to bind gp39-CD8 on Western Blot while the remaining antibodies did not. In order to define further the epitopes recognized by the monoclonal antibodies generated each was tested for binding by ELISA to a series of gp39 mutant proteins containing single or double point mutations which replaced a native amino acid residue with alanine.

The ELISA assay used was carried out as follows. Immulon 2 EIA plates were coated with 100 µl/well of a 0.8 µg/ml solution of a monoclonal antibody specific for murine Lyt 2 or 2.1 (53-6 (ATCC TIB 105) or 116.13-1 (ATCC HB 129) for fusion 5) diluted in 0.05 M sodium carbonate/sodium bicarbonate buffer, pH 9.6. The plates were sealed and incubated overnight at 4°C. Following incubation, unbound antibody was removed and the plates were blocked for I hour with specimen diluent (Genetic Systems Corporation) diluted 1 :10 in deionized water. After removal of blocking agent, 50 µl/well of appropriately diluted (see below) COS cell supernatants containing wild-type or mutant gp39-CD8 fusion protein or a negative control sCD72 fusion protein were added. After a 2 hour incubation at room temperature, fusion proteins were removed and the plates washed once with 200 µl/well of PBS-Tween. Culture supernatants containing gp39-specific antibodies were appropriately diluted (see below) in 10% FCS-Iscove's and each was added (50 µl/well) in duplicate to wells containing each of the gp39 or control fusion proteins. As a control, 50 µl/well of biotinylated rat anti-mouse CD8 (see below) was added to each of the different fusion protein containing wells in order to confirm that approximately equal amounts of each fusion protein was present in all wells. After a two hour incubation at room temperature, unbound antibodies were removed and the plates washed once with PBS-Tween. For antibodies from fusions 1, 7 and 9, HRP labeled rat anti-mouse IgG (Zymed and HRP labeled streptavidin (Vector Laboratories) were appropriately diluted in blocking reagent and 50 µl/well added to wells having previously received anti-gp39 antibody and anti-mouse CD8, respectively. For antibodies from fusion 5, HRP labeled mouse anti-rat IgG (Jackson Immunological Laboratories) diluted 1 :40.000 in blocking reagent was added. After a one-hour incubation at room temperature, HRP labeled reagents were removed and the plates washed three times with PBS-Tween. Disclosure of bound HRP labeled reagents and the measurement of resulting optical density was as described in other ELISA assays detailed above.

Two important parameters of the above assay were to demonstrate that similar amounts of each of the different fusion proteins were used on (i.e.. bound to) the assay plates and that non-saturating concentrations of anti-gp39 antibodies were used such that optical density readings fell within the linear part of the response curve. To normalize the amount of fusion protein in all wells, serial dilutions of each fusion protein containing COS cell supernatant were evaluated in the assay described above and a dilution of each was chosen for final assays which yielded an optical density value in the linear portion of the response curve (usually between 0.3 and 0.9 absorbance units) that was within ± 10% of that seen on wild-type sgp39 when traced with biotinylated rat anti-mouse CD8 followed by HRP labeled streptavidin. The optimal dilution of each of the antibody containing supernatants to be used in final assays was determined by evaluating serial dilutions of each supernatant on wild-type sgp39 fusion protein in the ELISA described above. Optimal dilution was defined as that for which a subsequent two-fold dilution yielded a decrease in resulting optical density value. The optimal dilution as well as two serial two-fold dilutions of it were evaluated in final assays on each of the fusion proteins as described above.

Reactivity of each of the anti-gp39 mAbs on the six mutant sgp39 fusion proteins is shown in Table 2. Values depict the binding intensity on each mutant relative to that observed on wild-type (expressed as a percent) and represent the average of duplicate determinations ± the standard error of the mean (SEM). Only data from those assays in which the amounts of the different fusion proteins on the assay plates were indeed similar (as shown by anti-CD8 tracing) and which were achieved with a non-saturating concentration of anti-gp39 mAb (usually a two-fold dilution of the optimal dilution as defined above) are shown.

Based on an overall similarity of binding profile on each of the gp39 mutants combined with Western blot results, the 32 anti-gp39 mAbs have been divided into 12 groups. Each group is characterized by a unique binding pattern which suggests that the recognized epitope in each group of antibodies is different. Group 1, comprising mAbs 39-1.3, 39-1.21, 39-1.25, 39-1.63, 39-1.122, 39-1.123, and 39-1.138, have a notable defect in the recognition of mutants E129/A and S131/A-T135/A. These mAbs also demonstrate a somewhat less profound binding deficiency on mutant K143/A. Reactivity of these mAbs with mutants Y145/A, N180/A, and F201/A-E202/A is similar to wild-type gp39. Group 2 is represented by a single mAb, 39-1.59 This antibody is similar to those in group I with regard to strongly reduced binding to mutants E129/A, S11/A-T135/A, and K143/A but differs in that it also showed somewhat weaker binding on mutants Y145/A, N180/A, and F201/A-E302A. Antibodies in group 3 (39-1.37 and 39-1.132) and group 4 (39-1.124 and 39-1.156) are quite similar to each other in that they recognized the E129/A mutant quite poorly and showed a profound binding deficiency on mutant K 143/A. Reactivity of these mAbs with the other mutants was either slightly weaker or equivalent to that observed with wild-type gp39. Groups 3 and 4 are clearly different from each other, however, as indicated by the divergent results seen in Western blot analysis where 39-1.37 and 39-1.132 are blot negative while 39-1.124 and 39-1.156 are blot positive. Antibodies in group 5 include 39-1.7, 39-1.128, and 39-1.26. They are similar to mAbs in groups 3 and 4 in that they demonstrated a comparable loss of binding on mutant K 143/A but differ as evidenced by better recognition of mutant E129/A. Binding of these antibodies to mutants S131/A-T135/A, Y145/A, N180/A, and F20/A-E202/A was essentially equivalent to that observed on wild-type gp39. Group 6, comprising mAbs 39-1.52, 39-1.61, 39-1.77, 39-1.93, 39-1.106, 39-1.109, and 39-1.134, are distinguished from the other anti-gp39 mAbs by an almost total lack of reactivity with mutant F201/A-E202/A. In addition, these antibodies demonstrated a definite although not as significant reduction in reactivity on mutant K143/A. Reactivity of this group of antibodies with the other mutants in the panel was similar to that observed on wild-type gp39. Group 7 includes a single antibody, 39-1.29. This mAb is very similar to those in group 6 except that it appears to recognize the K 143/A mutant nearly as well as wild-type gp39. A single antibody, 3 9-7.3E 12, represents group 8. This antibody is notably different from all the others in that it reacted with all the mutants quite well with only a slight loss of reactivity on the K143/A mutant as compared to wild-type gp39. [Add discussion of Groups 9-12.]

Group 9 includes a single antibody, 39-5.6E9. This antibody shows a similar binding avidity to mutant E129/A as observed on wild-type gp39 and a somewhat reduced binding avidity on mutants S131/A-T135/A, N180/A and F201/A-E2002/A than that observed on wild-type gp39. What particularly distinguishes this group from the others is a poor binding avidity when compared to binding avidity to wild-type gp39 on mutants K143/A and Y145/A. This antibody was also unable to bind to gp39 on a Western blot.

Group 10 as exemplified by antibodies 39-7.7G4, 39-9.27. 39-7.4C1, 39-9.68 and 39-9.246 are characterized by their similar or somewhat reduced binding avidity to all the mutants when compared to the binding avidity on wild-type gp39. All of the antibodies were also able to bind to gp39 on Western blot.

Groups 11 and 12, antibodies 39-9.11 and 39-9.274, are similar in their reactivity pattern on the mutant gp39 proteins, but differ in their ability to bind gyp39 on Western blot. Antibody 39-9.11 was unable to bind gp39, while 39-9.274 does bind, on Western blot. The reactivity pattern for both antibodies on the mutant gp39 proteins was characterized as similar to that of wild-type gp39 for E 129/A, S131/AT135/A, Y145/A and F201/A-E202/A. The binding avidity was somewhat reduced on mutant K143/A when compared to the binding avidity on wild-type gp39 and poor on mutant N180/A.

Collectively, the gp39 mutant reactivity data coupled with the Western blot results define at least 12 different recognition profiles and thus 12 different epitope specificities among the 32 anti-human gp39 mAbs. As defined above, mAbs in groups 1, 2, 3, 5, 8, 9 and 11 appear to recognize epitopes that are discontinuous or conformational in nature while the specificity of those in groups 4, 6, 7, 10 and 12 appear to be specific for continuous or linear sequences of gp39.

**TABLE 2**

| Summary of Anti-Human gp39 Monoclonal Antibodies | | | | | | | | | |
|---|---|---|---|---|---|---|---|---|---|
| Antibody Group | Antibody | Isotype | Antibody Reactivity with gp39 Point Mutants* | | | | | | Western Blot |
| | | | E129/A | S131/A T135/A | K143/A | Y145/A | N180/A | F201/A E202/A | |
| 1 | 39-1.3 | G1 | 2±0.3 | 8±1.7 | 27±0.8 | 109±2.9 | 87±5.3 | 106±3.4 | - |
| | 39-1.21 | G1 | 11±0.4 | 13±0.2 | 61±6.9 | 142±11.9 | 126±13.6 | 103±1.1 | - |
| | 39-1.25 | G1 | 0±0.8 | 7±1.2 | 63±8.8 | 135±0.3 | 124±7.7 | 114±8.4 | - |
| | 39-1.63 | G1 | 12 ± 3.1 | 14±1.8 | 26±0.6 | 122±1.5 | 98±9.2 | 84±10.5 | - |
| | 39-1.122 | G2b | 4±0.9 | 4±1.8 | 60±7.9 | 109±17.7 | 83±11.2 | 88±9.3 | - |
| | 39-1.23 | G1 | 16±1.4 | 22±0.0 | 59±0.7 | 95±3.8 | 114±31.0 | 101±2.1 | - |
| | 39-1.138 | G1 | -4±0.2 | -3±0.7 | 54±3,5 | 116±7.1 | 99±4.3 | 124±6.6 | - |
| 2 | 39-1.59 | GI | 4±3.5 | 14±1.0 | 38±4.1 | 74±6.9 | 55±6.8 | 59 ±7.4 | - |
| | 39-1.137 | Gl | 8±02 | 97±4.4 | 6±0.1 | III±5.9 | 98±11.2 | 90 ± 8.5 | - |
| 3 | 39-1.132 | G2b | 23±2.1 | 77±0.6 | 5 ± 0.6 | 94±4.2 | 82±0.2 | 93 ±11 .2 | - |
| | 39-1.124 | Gl | 25±1.2 | 69 ± 6.7 | 4 ± 0.7 | 90 ± 9.3 | 75 ± 1.5 | 85 ± 2.0 | + |
| 4 | 39-1.156 | Gl | 31 ± 0.8 | 84 ± 16.7 | 8 ± 1.0 | 100 ± 25.6 | 73 ± 10.7 | 76 ± 2.5 | + |
| 5 | 39-1.7 | G2b | 70 ± 2.9 | 89 ± 0.5 | 5 ± 0.1 | 106 ± 43 | 93 ± 1.4 | 112 ± 5.0 | - |
| | 39-1 128 | G2b | 52 ± 0.0 | 116 ± 6.6 | 7 ± 1.8 | 123 ± 5.6 | 133 ± 23.1 | 102 ± 4.7 | - |
| | 39-1.26 | G1 | 96 ± 3.7 | 103 ± 6.3 | 6 ± 0.3 | 128 ± 19 | 137 ± 16.3 | 111 ± 4.3 | - |
| 6 | 39-1.52 | G1 | 124 ±0,6 | 109 ± 6.8 | 68 ± 3.0 | 148 ±15.8 | 144 ± 7.5 | 1 ± 0.9 | + |
| | 39-1.61 | G1 | 94±4.6 | 81 ± 0.1 | 53 ± 3.0 | 92 ± 2.4 | 92 ± 3.1 | 0±0.5 | + |
| | 39-1.77 | G1 | 128 ± 35.0 | 117 ± 21.5 | 75 ± 10.3 | 122 ± 22.8 | 147 ± 2.6 | 3 ± 1.0 | + |
| | 39-1.93 | G1 | 99 ± 2.1 | 81 ± 6.5 | 46 ± 3.6 | 103 ± 8.9 | 114 ± 11.4 | 6 ± 1.1 | + |
| | 39-1.106 | G1 | 130 ± 10.6 | 113 ± 6.0 | 52 ± 16.6 | 124 ± 1.5 | 144 ± 25.1 1 | 9 ± 1.3 | + |
| | 39-1.109 | G1 | 96 ± 1.8 | 72 ± 0.6 | 54 ± 8.5 | 108 ± 13.6 3.6 | 82 ± 7.9 | 4 ± 4.5 | + |
| | 30-1.134 | G1 | 109 ± 0.8 <>,8 | 79 ± 0.0 | 53 ± 3.2 | 98 ± 4.6 | 82 ± 7.7 | 3 ± 0.2 | + |
| 7 | 39-1.29 | G2a | 109 ± 14.5 | 105 ± 7.1 | 91 ± 0.6 | 125 ± 10.6 | 122 ± 2.2 | 1 ± 0.2 | + |
| 8 | 39-7.3E12 | G2a | 102 ± 1.1 | 82 ± 5.5 | 67 ± 3.0 | 114 ± 6.4 | 99 ± 5.8 | 94 ± 1.3 | - |
| 9 | 39-5.6E9 | G2a | 101 ± 9.3 | 65 ± 3.0 | 3 ± 6.0 | 20 ± 1.9 | 54 ± 4.5 | 49 ± 3.7 | - |
| 10 | 39-7 7G4 | G2a | 87 ± 0.5 | 73 ± 5.0 | 55 ± 5.5 | 95 ± 5.1 | 84 ± 6.9 | 61 ± 2.0 | + |
| | 39-9.27 | G1 | 117 ± 26.5 | 89 ± 13.5 | 60 ± 10.6 | 139 ± 6.9 | 63 ± 0.3 | 88 ± 1.2 | + |
| | 39-74C1 | G2b | 100 ± 0.7 | 89 ± 8.8 | 64 ± 3.4 | 116 ± 0. | 111 ± 7.0 | 81 ± 5.7 | + |
| | 39-9.687 | G2b | 93 ± 17.3 | 78 ± 7.9 | 78 ± 4.7 | 85 ± 3.5 | 81 ± 0.8 | 79 ± 10.0 | + |
| | 39-9.246 | G1 | 118 ± 49 9 | 100 ± 5.4 | 80 ± 5.3 | 117 ± 11.2 | 124 ± 23.5 | 101 ± 3.1 I | + |
| 11 | 39-911 | G1 | 88 ± 4.8 | 94 ± 11.1 | 58 ± 1.1 | 118 ± 6.8 | 8 ± 1.8 | 89 ± 5.5 | - |
| 12 | 39-9 274 | GI | 107 ± 0.8 | 100 ± 9.1 | 67 ± 5.9 | 129 ± 17.8 | 20 ± 2.1 | 130 ± 1.2 | + |

| | | | | | | | | | |
|---|---|---|---|---|---|---|---|---|---|
| * Average: SD of duree independent experiments | | | | | | | | | |

### EXAMPLE 7

### Inhibition of T-cell Dependent B-cell Proliferation and Immunoglobulin Production

Activated T cells can induce resting B cells to proliferate and differentiate into immunoglobulin secreting cells. Furthermore, cell contact between activated T cells and B cells is required for B cells to switch from IgM to IgG, IgA or IgE production. As the interaction between CD40 and its ligand is thought to play a critical role in these processes, it was anticipated that anti-gp39 monoclonal antibodies would be capable of interfering with these forms of T cell "help".

The inhibitory effects of anti-gp39 monoclonal antibodies on human B cell activation and differentiation was evaluated in an *in vitro* T cell dependent B cell proliferation and immunoglobulin synthesis assay system. In this system (Hirohata et al. 1988. J. Immunoll. 140:3736-3744), activated T cells induce B cell activation, proliferation, and polyclonal antibody production (IgG, IgM, and IgA) in an MHC-unrestricted, Ag non-specific manner. It requires direct contact between B and T cells for the observed B cell events to occur and as such is thought to represent a relevant *in vitro* system to study B cell/T cell interactions leading to Ab production.

Briefly, human blood mononuclear cells (PBMCs) were isolated by diluting whole blood I: I with PBS, overlaying 25 ml onto 10 ml of Lymphocyte Separation Medium (LSM, Organon Teknika) and centrifuging for 25 minutes at 450 g. Cells at the interface were collected and washed once in PBS. Isolated cells were diluted to 5 x 10°/ml in 2% FCS-Iscove's containing 0.35 mM L-leucyl-L-leucine methyl ester hydrobromide (Leu-LeuOMe, Sigma) and incubated at room temperature for 15 minutes to kill monocytes and NK cells (Ohlin et al., 1989. Imnumology 66:485-490). Treated cells were washed twice with 2% FCS-Iscove's prior to separation of T and B cells.

T cells were isolated by incubating the Leu-LeuOMe treated cells with 150-fold AET-SRBC (sheep red blood cells treated with 0.143 M 2-aminoethylisothiouronium bromide (Sigma)) for 5-10 minutes on ice. E-rosette positive T cells (E'-T cells) were separated from the remaining cells by underlaying with cold LSM and centrifuging al 450 g for 25 minutes. The pellet (containing rosetted T cells) was collected and the sheep red blood cells were lysed with 0.83% ammonium chloride for five minutes at room temperature. Resulting T cells were washed once in 2% FCS-Iscove's. These cells were subsequently treated with mitomycin C (5 x 10⁶ E'-T cells and 40 µg mitomycin C/ml) for 40 minutes at 37°C and then washed three times with 2% FCS-Iscove's. B cells were obtained from the interface of the tubes in which E⁺-T cells were isolated from AET-SRBC treated PBMCs by centrifugation over an LSM cushion (described above). These cells were washed once in 2% FCS-Iscove's and re-rosetted with AET-SRBC as described above to remove any residual T cells and again centrifuged over an LSM cushion. Cells at the interface were collected, washed once in 2% FCS-Iscove's and are referred to here as B cells.

Costar 96 well plates were coated with 50 µl/well of a 2 µg/ml solution of anti-CD3 monoclonal antibody 64.1 (Hansen et al., In Leucocyte Typing, Springer -Verlag, Inc., pp 195-212 (1984)) in serum free Iscove's medium for a minimum of four hours at room temperature. Excess antibody was aspirated from the wells and 100.000 mitomycin C treated T cells and 2,000 twice rosetted B cells in a total volume of 150 µl of culture medium (Iscove's modified Dulbecco's medium supplemented with 10% FCS) were added to each well. Supernatants collected from hybridomas producing anti-gp39 monoclonal antibody or a negative control monoclonal antibody were then added to each of three wells, 100 µl/well. Additional wells received the same volume of culture medium only. After six days of culture in a 37°C incubator containing 6% CO₂, each set of triplicate wells was assessed for B cell proliferation and total human IgG and IgM.

B cell proliferation was measured by tritiated thymidine uptake. After removal of 100 µl/well of culture supernatant for IgG and IgM analysis (see below), 50 µl of culture medium containing 1 µCi of [³H]thymidine (New England Nuclear) was added to each well. After a further 18 hours of culture at 37°C, the plates were frozen, thawed, and cells harvested onto glass fiber filter mats with a TOMTEC full plate cell harvester [³H]thymidine incorporation was measured with an LKB Wallace Beta-Plate liquid scintillation counter. Counts from triplicate wells were averaged and are presented in Table 3 as a percentage ± 1 SD of the values seen with medium only control wells.

Human IgG and IgM were quantitated by coating Immulon 2 EIA plates (Dynatech) with 100 µl/well of a I µg/ml solution of goat anti-human µg (Southern Biotechnology Associates) in 0.05 M sodium carbonate/sodium bicarbonate buffer, pH 9.6. Plates were sealed and incubated overnight at 4°C. Excess antibody was removed and plates blocked as described in earlier ELISA assays. Following blocking, all wells received 50 µl/well of 2XPTB (2X PBS containing 2% bovine serum albumin (Intergen) and 1% Tween 20)). Culture supernatants diluted 1:10 (for IgM analysis) and 1:40 (for IgG analysis) in culture medium were added to the wells, 50 µl/well, and incubated for one hour at room temperature. These dilutions were arrived at in a preliminary experiment using serial dilutions of culture supernatants from medium only wells and selecting that dilution(s) that yielded optical density values near the upper end of the most linear part of the response curve for IgG and IgM. Supernatants were removed, the plates washed twice with PBS-Tween and HRP labeled goat anti-human IgG or IgM (Jackson Immunological Laboratories and), appropriately diluted in 1XPTB (2XPTB diluted I: I with PBS), added to respective wells, 100 µl/well After a one hour incubation at room temperature, HRP labeled reagents were removed and the plates washed three times with PBS-Tween. Disclosure of bound HRP labeled reagents and the measurement of resulting optical density was as described in other ELISA assays detailed above. Optical density values from the triplicate wells were averaged and are presented in Table 3 as a percentage ± 1 SD of the values seen with medium only control wells.

As shown in Table 3. each of the anti-gp39 monoclonal antibodies tested was capable of significantly inhibiting the T cell driven proliferation of B cells, resulting in values that were only 2-4% of that seen in wells that did not receive g39 specific antibody. Concomitantly, the production of IgG and IgM were also significantly suppressed.

The inhibitory effect of the various anti-gp39 monoclonal antibodies on T cell dependent human B cell immunoglobulin production was further investigated in a more quantitative manner using defined concentrations of purified antibody. Antibodies were affinity purified from culture supernatants on Protein A Sepharose or GammaBind Plus Sepharose columns (Pharmacia) according to manufacturer's instructions and quantitated by optical density absorbance using an extinction coefficient of 1.4. Experiments were set up as described above with the following modifications. Half area Costar 96 well plates were utilized and the concentration anti-CD3 antibody used to coat the wells was 4 µg/ml. All wells received 150,000 mitomycin C treated T cells and 20.000 B cells in a total volume of 100 µl of culture medium. Anti-gp39 and negative control antibodies were diluted to 60, 6, and 0.6 µg/ml in culture medium and 50 µl of each dilution added to each of three wells for a final concentration of each antibody in the culture wells of 20.2 and 0.3 µg/ml. Control wells received 50 µl/well of culture medium only. Cells were cultured for a total of 10 days in a 37°C/6% CO2 incubator at which time supernatants from triplicate wells were pooled and assessed for total human IgG and IgM. Measurement of human IgG and IgM were as described above except that each pooled supernatant was assayed in triplicate, the supernatants were diluted in 2% FCS-Iscove's to much higher dilutions given the lounger period of cell culture (and thus antibody production), wells on the assay plates did not receive 2XPTB prior to addition of diluted supernatants, and HARP reagents were diluted in blocking buffer.

**TABLE 3**

| Suppression *of in vitro* B Cell Proliferation and Antibody Production by Anti-Human gp39 mAbs | | | |
|---|---|---|---|
| mAb | [³H]Thymidine Incorporation (% of Medium Control) | Ig Produced (% of Medium Control) | |
| | | IgM | IgG |
| 39-1.3 | 3.5 ± 0.7 | 33.5 ± 15.4 | 60.2 ± 3.6 |
| 39-1.7 | 3.3 ± 0.5 | 13.9 ± 9.1 | 31.8 ± 9.6 |
| 39-1.26 | 2.1 ± 0.1 | 10.0 ± 3.7 | 24.0 ± 8.3 |
| 39-1.29 | 3.9 ± 0.7 | 43.4 ± 11.4 | 39.8 ± 6.4 |
| 39-1.37 | 2.4 ± 0.2 | 20.2 ± 4.1 | 44.8 ± 10.9 |
| 39-1.61 | 3.4 ± 0.6 | 10.6 ± 4.6 | 32.2 ± 15.0 |
| 39-1.77 | 2.1 ± 0.6 | 11.7 ± 2.1 | 41.9 ± 2.1 |
| 39-1.106 | 2.9 ± 0.3 | 17.9 ± 4.9 | 28.6 ± 8.3 |
| 39-1.124 | 3.5 ± 0.6 | 17.0 ± 5.7 | 36.5 ± 16.9 |
| 39-1.128 | 3.1 ± 0.5 | 21.7 ± 9.5 | 53.3 ± 6.2 |
| 39-1.132 | 3.3 ± 0.4 | 13.0 ± 4.7 | 50.1 ± 2.0 |
| 39-1.134 | 2.3 ± 0.8 | 12.5 ± 4.1 | 335 ± 10.2 |
| 39-1.156 | 2.7 ± 0.1 | 12.2 ± 4.8 | 26.9 ± 5.6 |
| Neg. Cont. | 87.9 ± 7.9 | 87.9 ± 13.8 | 114.9 ± 28.5 |

Data from these experiments are presented in Table 4. At the highest concentration of antibody used, 20 µg/ml, all anti-gp39 antibodies significantly inhibited the production of both IgG and IgM. At this concentration, levels of human antibody generated were consistently 10-30% of that seen in the presence of medium only. As the concentration of anti-gp39 antibody was decreased so to, in general, was the level of inhibition. At the two lowest concentrations of anti-gp39 antibodies employed, 2 and 0.2 µg/ml, it was quite apparent that certain anti-gp39 antibodies including, in particular, 39-1.7, 39-1.26, 39-1.77, 39-1.106, 39-1.134. 39-7.3E12, and 39-5.6E9. were much more effective at inhibiting human IgG and IgM production than were others. This observation suggests that epitope specificity and/or antibody avidity is an important parameter in the degree to which monoclonal antibodies directed to gp39 can interfere with gp39-CD40 interaction.

**TABLE 4**

| Comparative Suppression of *in vitro* B cell Antibody Production by Anti-Human gp39 Monoclonal Antibodies | | | | | | |
|---|---|---|---|---|---|---|
| Monoclonal Antibody | Inhibition of in vitro Antibody Synthesis % of Medium Control ± SD* | | | | | |
| | IgG | | | IgM | | |
| | 20 µg/ml | 2 µg/ml | 0.2 µg/ml | 20 µg/ml | 2 µg/ml | 0.2 µg/ml |
| 39-1.3 | 9 ± 7 | 53 ± 16 | 91 ± 7 | 23 ± 3 | 63 ± 17 | 94 ± 15 |
| 39-1.122 | 13 ± 1 | 28 ± 6 | 70 ± 12 | 20 ± 23 | 60 ± 18 | 84 ± 6 |
| 39-1.138 | 21 ± 4 | 60 ± 14 | 90 ± 9 | 29 ± 23 | 91 ± 29 | 101 ± 22 |
| 39-1.59 | 21 ± 6 | 57 ± 10 | 85 ± 22 | 34 ± 25 | 82 ± 35 | 84 ± 13 |
| 39-1.37 | 18 ± 4 | 39 ± 23 | 74 ± 16 | 26 ± 16 | 62 ± 14 | 91 ± 12 |
| 39-1.132 | 11 ± 1 | 25 ± 17 | 66 ± 10 | 23 ± 24 | 60 ± 20) | 102 ± 23 |
| 39-1.124 | 11 ± 1 | 20 ± 8 | 54 ± 20 | 21 ± 28 | 47 ± 31 | 76 ± 13 |
| 39-1.156 | 11 ± 6 | 15 ± 13 | 43 ± 22 | 14 ± 6 | 28 ± 10 | 81 ± 8 |
| 39-1.7 | 17 ± 8 | 12 ± 7 | 34 ± 19 | 20 ± 6 | 27 ± 12 | 58 ± 15 |
| 39-1.128 | 19 ± 1 | 19 ± 3 | 55 ± 18 | 26 ± 11 | 46 ± 22 | 85 ± 21 |
| 39-1.26 | 22 ± 18 | 15 ± 9 | 41 ± 14 | 26 ± 13 | 27 ± 28 | 78 ± 2 |
| 39-1.77 | 11 ± 6 | 16 ± 5 | 38 ± 16 | 23 ± 39 | 28 ± 24 | 68 ± 34 |
| 39-1.106 | 8 ± 2 | 11 ± 5 | 21 ± 12 | 27 ± 23 | 22 ± 21 | 41 ± 14 |
| 39-1.134 | 10 ± 4 | 15 ± 5 | 28 ± 7 | 23 ± 13 | 27 ± 26 | 65 ± 32 |
| 39-1.29 | 10 ± 1 | 13 ± 3 | 49 ± 23 | 23 ± 16 | 37 ± 11 | 86 ± 9 |
| 39-7.3E12 | 9 ± 1 | 12 ± 4 | 37 ± 16 | 11 ± 2 | 18 ± 16 | 72 ± 10 |
| 39-5.6E9 | 16 ± 4 | 11 ± 5 | 20 ± 15 | 27 ± 4 | 14 ± 12 | 33 ± 5 |
| 39-7.7G4 | 12 ± 1 | 10 ± 6 | 57 ± 10 | 19 ± 3 | 27 ± 25 | 80 ± 14 |
| 39-9.11 | 30 ± 6 | 31 ± 17 | 72 ± 16 | 75 ± 35 | 74 ± 23 | 105 ± 40 |
| 39-9.274 | 8 ± 1 | 14 ± 6 | 81 ± 17 | 7 ± 8 | 38 ± 19 | 77 ± 8 |
| 39-9.27 | 13 ± 4 | 19 ± 11 | 79 ± 28 | 28 ± 14 | 41 ± 28 | 108 ± 23 |
| 39-7.4Cl | 13 ± 4 | 31 ± 7 | 74 ± 19 | 22 ± 8 | 49 ± 27 | 87 ± 4 |
| 39-68 | 29 ± 8 | 64 ± 15 | 91 ± 17 | 50 ± 17 | 88 ± 21 | 114 ± 17 |
| 39-9.246 | 12 ± 7 | 21 ± 6 | 71 ± 16 | 29 ± 16 | 43 ± 29 | 99 ± 35 |

| | | | | | | |
|---|---|---|---|---|---|---|
| *Average ± SD of three independent experiments except for data complied at 20 µg/ml antibody concentration for which there were only two experiments. | | | | | | |

### EXAMPLE 8

### Detection of Mutant gp39 in an X-Linked Hyper IgM Patient

Monoclonal antibodies with different binding characteristics with mutant human gp39 were used to determine whether point mutations in gp39 could be recognized in a blood sample taken from an X-linked-hyper IgM patient. In this assay, patients whose cells showed positive staining with the gp39 specific monoclonal antibodies, but no staining with CD40Ig, the normal ligand, would be known to express gp39 protein that is nonfunctional and could be diagnosed as X-HIM. Using a panel of monoclonal antibodies, with known differences in epitopes, provides for a greater number of differing mutations which can be detected in an X-HIM sample. Using this assay, it is not totally possible to exclude Common Variable Immunodeficiency as a diagnoses, but it is expected that a significant percentage of X-HIM patients can be detected by this approach. A subset of HIM patients, those whose gp39 defect results in a lack of internal expression due, for example, to a mutation to a stop codon early in the gp39 coding sequence, also could not be confirmed by this approach.

Briefly, T cells are isolated from a sample of peripheral blood lymphocytes from a patient by Ficoll gradient centrifugation followed by rosetting using sheep erythrocytes. Staining of fixed, permeabilized cells was performed using the methods flung et al. (J. Immunol. Methods 159:197-207 (1993) with modifications as described.

Isolated T cells were stimulated with PMA (10 ng/ml) and ionomycin (1 µg/ml) in the presence of monensin at 3 mM for three hours. The stimulated cells were then washed with PBS and fixed by incubation in 4% paraformaldehyde in Hank's balanced salt solution for 10 minutes at 4°C. The fixed cells were washed once with PBS. then permeabilized and blocked by incubation in blocking buffer (0. 1% saponin, 10% goat serum in PBS) for 10 minutes at room temperature.

Cells were pelleted and resuspended in 0.1% saponin, 2% fetal bovine serum in PBS and aliquots were prepared for staining at an approximate density of 1 x 10⁷ cells/ml. Monoclonal antibodies were added to a final concentration of 10 µg/ml and the cells were incubated at room temperature for 30 minutes prior to washing twice with blocking buffer and resuspension in blocking buffer containing FITC-conjugated goat anti-mouse Fc. After an additional 20 minute incubation at room temperature, the cells were washed twice with 2% FBS in PBS and analyzed by flow cytometry.

As a control and to test the feasibility of detecting gp39 in the interior of a cell normal T cells were isolated and treated as above except prior to fixation the cells were treated for 5 minutes with trypsin to remove surface expressed gp39. The cells were then fixed and stained as above. A comparison of staining of non-activated with activated T cells allows for the demonstration of specific staining within normal T cells.

In Table 5 is provided a summary of staining obtained with T cells isolated from an X-HIM patient with anli-gp39 monoclonal antibodies.

**TABLE 5**

| Summary of Staining of T Cells Obtained from X-HIM Patient with anti-gp39 mAb | | | | |
|---|---|---|---|---|
| Antibody | CD¹ | NC² | Western | Isotype |
| 39-1.3 | - | + | - | G1 |
| 39-1.122 | - | + | - | G2h |
| 39.1.138 | - | + | - | G1 |
| 39.1.124 | - | + | + | G1 |
| 39-1.7 | - | + | - | G2b |
| 39-1.26 | - | + | - | G2b |
| 139-1.106 | + | + | + | G1 |
| 39-1.134 | + | + | + | G1 |

| | | | | |
|---|---|---|---|---|
| 1 X-HiM patient CD (Aruffo et al. 1993. Cell ***72***:291) 2 Normal control | | | | |

### EXAMPLE 9

### Biological Activity of Anti-gp39 Monoclonal Antibodies in Cynomolgus Monkeys

In this example, two murine monoclonal antibodies binding different epitopes of human gp39 were examined for testing their ability to bind to activated T cells, block the binding of human CD40-Ig to activated T cells, and to inhibit T cell-dependent immunoglobulin production using peripheral blood lymphocytes isolated from the cynomolgus monkeys *Macacca fascicularis* and *Macacca nemestrina.* The results using PBL from these non-human primates were similar to those obtained using human PBL, supporting the use of the macaque as an appropriate animal model.

To test for the ability of the antibodies to bind to macaque T cells. *Macacca nemestrina* and *Macacca fascicularis* peripheral blood mononuclear cells (PBMC) were isolated by diluting whole blood 1:1 in PBS and overlaying 25 ml onto 10 ml of 95% Lymphocyte Separation Medium (Organon Teknika)/5% PBS and centrifuging for 25 minutes at 450 g. Cells at the interface were collected and washed once in PBS. T cells were isolated by incubating the PBMCs on ice with 150x AET-SRBC (sheep red blood cells treated with 0.143 M 2-aminoethyl- isothiouronium bromide hydrobromide (Sigma) for 5 to 10 minutes. E-rosette positive T cells were separated by underlaying with cold LSM and centrifuging at 450 g for 25 minutes. SRBC in the pellet were lysed with 0.83% ammonium chloride for 5 minutes at room temperature. Resulting T cells were washed and incubated overnight in 10% FCS-iscove's medium at I to 3 x 10⁶ cells/ml in a humidified incubator at 37°C, 6% CO₂. T cells were then stimulated with PMA (Sigma) and ionomycin (Sigma) at 10 ng/m) and I µg/ml respectively in 10% FCS-Iscove's for 5 to 6 hours in a humidified incubator at 37°C. 2.5 x 10⁵ activated T cells per 11x75 mm tube were centrifuged at 250 g for 5 minutes and culture medium aspirated.

Supernatants (100 µl) collected from the growing hybridomas containing antibodies 106 or 7 or a negative control antibody were then added to each tube and incubated on ice for 30 minutes. Two milliliters of 2% FCS-Iscove's medium was added to each tube and the tubes were centrifuged at 250 g for 5 minutes before the supernatant was aspirated. FITC-labeled rat anti-mouse IgG polyclonal antisera (Zymed) diluted 1:50 in 2% FCS-Iscove's medium was added to each tube at 100 µl/tube and incubated on ice for 30 minutes. Cells in each tube were washed twice with I ml of 2% FCS-Iscove's medium. Cells were then stained with a phycoerythrin-mouse anti-human CD4 monoclonal antibody, washed, and finally suspended in 250 µl/tube of 2% FCS-Iscove's medium prior to analysis on a Becton Dickinson FACScan. As can be seen in Figure 1 monoclonal antibodies 7 (39-1.7) and 106 (39-1.106) were both capable of recognizing activated human (Figures 1A and 1B) and macaque (Figures 1C and 1D) CD4' T cells.

Monoclonal antibodies 7 and 106 were also tested for their ability to block the binding of a CD40-Ig fusion protein to activated macaque cells. T cells were isolated and activated as described above. Following activation, 2.5 x 10⁵ T cells were added to 11x75mm tubes and centrifuged at 250 g for 5 minutes. The culture medium was removed by aspiration and 100 µl of supernatant containing anti-gp39 monoclonal antibodies 7 or 106, medium only, or negative control antibody were then added to each tube for a 30-minute incubation on ice. Supernatants were then diluted with 2 ml of 2% FCS-Iscove's medium, the tubes centrifuged at 250 g for 5 minutes and the supernatant removed by aspiration. CD40-Ig diluted to 20 µg/ml in 10% FCS-Iscove's medium was then added to each tube, 100 µl/tube, and incubated for 30 minutes on ice. Two ml of 2% FCS-Iscove's medium was added to each tube, and the tubes were centrifuged at 250 g for 5 minutes. The supernatants were removed to aspiration and phycoerythrin or fluorescein labeled F(ab')² goat anti-human IgG(Fc) (Jackson Laboratories) was diluted 1:5,000 or 1:500 respectively in 10% FCS-Iscove's medium and added to the cells. After a 30 minute incubation on ice, the cells were washed twice with 1 ml each of 2% FCS-Iscove's medium and finally suspended in 250 µl of 2% FCS-Iscove's medium per tube prior to analysis on a Becton Dickinson FACScan. The data is plotted in Figure 2 and demonstrates that both murine anti-gp39 monoclonal antibodies 7 and 106 were capable of inhibiting the binding of human CD40-Ig to activated human (Figures 2A and 2B) and macaque (Figures 2C and 2D) T cells.

To test the ability of monoclonal antibodies 7 and 106 to block the ability of B cells to produce antibodies after contact with activated T cells, peripheral blood mononuclear cell (PBMC) were isolated from *Macacca fascicularis* monkeys as described above.

The T cell fraction was treated with mitomycin C (5 x 10⁶ T cells and 40 µg mitomycin C (Sigma) per ml) for 40 minutes followed by three washes with 10% FCS-Iscove's medium. The B cell fraction (the interface layer from LSM centrifugation) was re-rosetted with AET-SRBC to remove any residual T cells. 96-well half area plates (Costar) were coated with 50 µl of a 4 µl/ml solution ofanti-CD3 antibody (FN-18, Clark, E. A. et al., 1987, Eur. J. Immunol. 17:1799-1805) in serum free Iscove's medium for a minimum of four hours at room temperature. Excess antibody was aspirated from the wells and 1.5 x 10⁵ mitomycin C treated T cells and 5 x 10³ twice rosetted B cells in 10% FCS-Iscove's medium were added. Purified anti-gp39 and control antibodies were diluted in 10% FCS-Iscove's medium to a final concentration, in culture, of 10, 1, 0.4 and 0.1 µg/ml and added to the wells. After 10 days, culture supernatant from triplicate wells were pooled, diluted, and assessed for total macaque IgG and IgM.

Macaque IgG and IgM were quantitated as described for human IgG and IgM except that supernatants were diluted 1:640 and 1:40 for IgG and IgM analysis, respectively. The results of the assay, when a concentration of 0.4 µg/ml is used are seen in Figure 3 and are expressed as the percent of total IgG and IgM compared to cultures treated with culture medium only in the absence of antibody. Antibodies Exa and IVA7 are isotype matched controls for monoclonal antibodies 106 and 7 respectively. Above, both monoclonal antibodies 7 and 106 were capable of inhibiting T cell dependent IgG and IgM antibody production by macaque B cells in a manner very similar to that seen with human cells. These data suggest that the cynomolgous monkeys would be a suitable non-human animal model to evaluate the *in vivo* activity of the anti-human gp39 monoclonal antibodies.

### EXAMPLE 10

### Ability of Murine Anti-Human gp39 Monoclonal Antibodies to Suppress T Cell Dependent Antibody Response In Cynomolgus Monkeys

In this example, three gp39-binding proteins, murine monoclonal antibodies 7 and 106 and a human recombinant CD40-Ig fusion protein, were evaluated for their ability to suppress the primary T cell dependent antibody response to antigens following intravenous administration to the cynomolgus monkey *Macacca fascicularis.* At the unoptimized dose level tested, monoclonal antibody 106 suppressed the humoral response, demonstrating the efficacy of anti-gp39 administration to suppress a T cell dependent humoral response in primates.

Four groups consisting of one male and three female macaques were treated with 4 mg/kg of monoclonal antibodies 7 or 106, or a control antibody I G I (specific for *Pseudomonas aeruginosa* protein F) or CD40-Ig on days 1, 3, 5, 8, 10 and 12 (course 1), and on days 50, 52, 54, 57, 59, and 61 (course 2). In addition, each animal was immunized intravenously on day 1 (primary immunization) and day 50 (secondary immunization) with 1.7 ml/kg of a 10% mixture of sheep red blood cells, a T cell dependent antigen. Following the elimination of all gp39-binding proteins from the sera of the animals to below detectable levels, each animal was re-immunized with sheep red blood cells and co-immunized with 10 mg/animal of the neoantigen keyhole limpet hemocyanin (KLH).

Serum was collected weekly and anti-sheep red blood cell antibody and anti-KLH antibodies (IgG and IgM) were assessed by ELISA. Antibody titers to sheep red blood cells were assayed by coating Immulon I plates with SRBC membranes at a concentration of 5µg/ml in PBS and incubating 12 to 18 hours at 4°C. The excess solution was removed and the plates were washed with PBS-Tween After washing the plates were blocked with PBS-Tween for 1 hour at room temperature. Serum samples were diluted serially in PBS-Tween and 60 µl was transferred to each well of the antigen coated plate. The diluted serum samples were incubated with the antigen for 1.5 hours and the excess sample was removed prior to washing the plates with PBS-Tween. Goat anti-human F(ab')₂ fragment IgG or IgM (Jackson Laboratories) diluted 1:10,000 100 µl were added to the appropriate wells and incubated for 1 hour at room temperature. After incubation the wells were washed with PBS-Tween and tetramethyl benzidine (Genetic Systems Corporation) diluted 1: 100 in substrate buffer (Genetic Systems Corporation) was added. Substrate was allowed to incubate for 15 minutes at room temperature prior to the addition of 1 NH₂SO₄ to stop the reaction. Absorbance values were recorded at 450 nm/630 nm using a microtiter plate reader.

Antibody titers to KLH were determined in a similar assay as above Briefly, Immulon II plated were coated with KLH (Pacific BioMarine Labs) at 10 µl/ml in 0.05 M carbonate/bicarbonate buffer, pH 9.6 for 12 to 18 hours at 4°C. The plates were washed with PBS-Tween and the plates were blocked with Specimen Diluent (Genetic Systems Corporation) diluted 1 :100 with distilled water for 1 hour at room temperature. Serum samples were serially diluted four fold in Specimen Diluent starting at 1/50. Sixty µl/well of each sample dilution was transferred to the antigen coated plates and incubated for I hour at room temperature. The remainder of the assay was carried out as described above, except goat anti-human F(ab')₂ fragment IgG and IgM were diluted 1:5,000.

In Figures 4A and 4B data is presented which demonstrates that at the unoptimized dose tested, monoclonal antibody 106 was capable of suppressing *in vivo* the ability of a macaque to mount a primary response to the sheep red blood cells. The suppression was not complete, but was approximately 10 to 15 fold more than that observed for the other anti-gp39 blocking proteins tested. Following the secondary immunization none of the proteins showed clear evidence of suppression, although anti-SRBC titers in the 106 treated group were significantly lower than in the other treatment groups. Monkeys in all of the groups received 10 mg KLH intramuscularly as a primary immunization on day 106 to investigate the ability of the monkeys to respond to a new antigen. Figure 5 shows that all groups of monkeys were able to mount a strong response to KLH, demonstrating that the treatment with anti-gp39 binding proteins was not nonspecifically immunosuppressive.

In another study, anti-human gp39 monoclonal antibody 106 was administered to cynomolgus monkeys at 20 mg/kg on days 1, 3 and 5. An anti-tumor associated murine monoclonal antibody, L6 ATCC HB 8677, was used as a negative control All animals were immunized intravenously on day 1 with a sheep red blood cell suspension (1.7 ml/kg of a 10% suspension) just prior to administration of the test compound. Blood samples were taken prior to administration of the sheep red blood cells or murine monoclonal antibodies and on days 8, 15, 22, 29, 36 and 43. Serum samples were tested for IgG and IgM antibody titers to sheep red blood cells.

The data from this experiment as seen in Tables 6 and 7, demonstrate that murine anti-human gp39 monoclonal antibody 106 significantly reduced the ability of the monkeys to mount a T cell dependent immune response to the sheep red blood cells. These data confirm the results of the experiments carried out in mice with antibody 106 specific for murine gp39 and the *in vivo* studies with monkeys and human peripheral blood lymphocytes with murine anti-human gp39 monoclonal antibody.

**TABLE 6**

| IgG Anti-Sheep Red Blood Cell Response | | | | | | | | | |
|---|---|---|---|---|---|---|---|---|---|
| | Animal # | Sex | Pre-dose | Day 8 | Day 15 | Day 22 | Day 29 | Day 36 | Day 43 |
| Group 1 mAb 106 | 223 | Male | 30¹ | 30 | 30 | 10 | <10 | 10 | 01 |
| | 224 | Male | 90 | 90 | 90 | 30 | 90 | 90 | 90 |
| | 225 | Female | 10 | <10 | <10 | <10 | <10 | 810 | 2430 |
| | 226 | Female | 10 | 10 | <10 | <10 | <10 | 90 | 90 |
| | | | | | | | | | |
| Group 2 mAb L6 | 227 | Male | 10 | 270 | 810 | 810 | 810 | 810 | 270 |
| | 228 | Male | 30 | 270 | 2430 | 810 | 810 | 810 | 810 |
| | 229 | Female | 30 | 2430 | 7290 | 2430 | 2430 | 810 | 810 |
| | 230 | Female | ND | ND | ND | ND | ND | ND | ND |

| | | | | | | | | | |
|---|---|---|---|---|---|---|---|---|---|
| 1. Antibody titers, the endpoint titer reported is defined as the recipricol of the dilution which is greater than two times above the plate background mean. 2. ND. not done | | | | | | | | | |

**TABLE 7**

| IgM Anti-Sheep Red Blood Cell Response | | | | | | | | | |
|---|---|---|---|---|---|---|---|---|---|
| | Animal # | Sex | Pre-dose | Day 8 | Day 15 | Day 22 | Day 29 | Day 36 | Day 43 |
| Group 1 mAb 106 | 223 | Male | 90¹ | 810 | 270 | 90 | 30 | 30 | 30 |
| | 224 | Male | 30 | 810 | 810 | 270 | 270 | 90 | 90 |
| | 225 | Female | 30 | 270 | 90 | 90 | 90 | 2430 | 2430 |
| | 226 | Female | 90 | 270 | 270 | 270 | 810 | 2430 | 2430 |
| | | | | | | | | | |
| Group 2 mAb L6 | 227 | Male | 270 | 810 | 2430 | 810 | 810 | 810 | 810 |
| | 228 | Male | 30 | 21870 | 21870 | 7290 | 7290 | 2430 | 2430 |
| | 229 | Female | 90 | 7290 | 7290 | 2430 | 2430 | 2430 | 810 |
| | 230 | Female | 90 | 2430 | 2430 | 2430 | 810 | 810 | 810 |

| | | | | | | | | | |
|---|---|---|---|---|---|---|---|---|---|
| 1. Antibody titers, the endpoint titer reported is defined as the reciprocal of the dilution which is greater than two times above the plate background mean. | | | | | | | | | |

### EXAMPLE 11

### Construction of Recombinant anti-gp39 Single-Chain Variable Regions

In this example, the nucleotide sequences of the heavy chain variable region (VH) and the light chain variable region (VL) of two anti-human gp39 monoclonal antibodies [39-1.7 (7) and 39-1.106 (106)] are determined and isolated. The DNA fragments encoding the VH and VL of each monoclonal antibody were then assembled into a continuous expression cassette using an intervening sequence encoding a (Gly₄Ser)₃ linker. The cassettes were expressed in mammalian cells and functional activity of the recombinant single chain antibody (sFv) molecules were determined

### A. Isolation of RNA, cDNA Synthesis and PCR Amplification

RNA was isolated from 5 x 10⁷ clone 106 or clone 7 hybridoma cells using an mRNA isolation kit (Stratagene, LaJolla, CA). cDNA was generated from the RNA using the StrataScript RT-PCR kit (Stratagene, LaJolla, CA) and immunoglobulin constant region specific antisense primers. The Cₖ-specific primer was complementary to nucleotide sequence 228 to 257 of the murine kappa light chain constant region This primer was used for first strand synthesis of both the clone 106 and clone 7 VL cDNAs. An IgG₁-specific antisense primer or an IgG₂ₕ-specific antisense primer were used to generate clone 106 and clone 7 VH cDNAs, respectively. The IgG₁-specific antisense primer was complementary to nucleotides 100 to 12 of the murine IgG₁ CH1 region and the IgG_{2b}-specific antisense primer was complementary to nucleotides 101 to 123 of the murine IgG_{2b} CHI region. First strand reactions were set up using 300 ng of antisense primer and 0.5 µg mRNA.

The cDNAs were purified using Geneclean™ (Bio 101. LaJolla, CA) and subsequently polyG-tailed with 10 mM dGTP and terminal deoxynucleotidyl transferase (Stratagene) for 1 hour at 37°C. Poly G-tailed cDNAs were purified again using GeneClean™. Two µl of each cDNA were amplified by anchor-PCR (Saiki et al., 1988. Science 239:487-491) in a total volume of 100 µl using 20 µMol of each dNTP, 100 pM of sense and antisense primers and 2U Taq polymerase. The sense primer contained a region complementary to the polyG tail (Loh et al. 1989. Science 243:217-220) and a *XhaI* site (underlined). 
5'-CGTCGATCTAGAGCATGTGCAAGTCCGATGAGTCCCCC
CCCCCCCCC-3' Seq. I.D. No. 7
 The antisense primers were nested primers containing a *HindIII* site (underlined) and annealed to either nucleotides 101-125 of murine C_{K}
5'-CGTCATAAGCTTCAGGAAGCACACGACTGAGGCAC-3' Seq. I.D. No.8
or to nucleotides 47-69 of murine IgG₁ CH1
5'-CGTCATAAGCTTGTCACCATGGAGTTAGTTTG-3' Seq. **I.D.** No. 9
or to nucleotides 38-62 of murine IgG_{2b} CH1
5'-CGTCATAAGCTTGAACCAGTTGTATCTCCACACCCAG-3' Seq. I.D. No. 10
Reactions were carried out in a Perkin-Elmer Cetus thermal cycler (Norwalk, CT) with a 33 cycle program of 30 sec. denaturation at 94°C, 90 sec. annealing at 45°C and 90 sec. extension at 72°C.

PCR-amplified VL and VH fragments were digested with *XbaI* and *HindIII,* ligated into the pUC 19 vector and transformed in DH5α E. *coli.* Clones containing VL or VH were identified by DNA sequencing. Consensus sequences for clone 106 (Figure 6A and Figure 6B) or clone 7 (Figure 7A and Figure 7B) were determined by analyzing the sequence of multiple VL or VH clones and alignment of the deduced amino acid sequences with previously published murine VL and VH sequences (Kabat et al. 1987. U.S. Department of Health and Human Services). The nucleotide and deduced amino acid sequence for clone 106 VL and VH are depicted in Figure 6A and Figure 6B (Seq. I.D. Nos. 11 through 14) and the nucleotide and deduced amino acid sequence for 7 VL and VH are depicted in Figure 7A and Figure 7B (Seq. I.D. No. 15 through 18).

### B. Construction of Clone 7 and Clone 106 sFv Expression Cassettes

Single chain sFv were constructed in the VL-VH orientation for both 7 and 106, each cassette containing an intervening (Gly₄Ser)₃ linker (Huston et al. 1988. Proc. Nat'l Acad. Sci. USA 85:5879-5883). To create the 106 VL-VH cassette, the clone 106 VL gene was reamplified from the pUC19 sequencing construct using a sense PCR primer (106 γl Sall) that encoded a Sall site immediately prior to sequence encoding the first residue of the mature VL. The antisense primer (106 γlvILK3') was complementary to sequence encoding the last nine residues of the VL and the first 12 residues of the (Gly₄Ser)₃ linker. Additionally, the 106 VH was reamplified from the pUC19 sequencing construct using a sense primer (106 γlvhLK5') that encoded the last 11residues of the (Gly₄Ser)₃ linker followed by the first nine residues of the mature VH and an antisense primer (106vhBcII) complementary to sequence encoding the last nine residues of the VH region and a *BcII* site. The modified VL and VH PCR products were then purified using Geneclean™ (Bio 101, LaJolla, CA) and were added to a single PCR reaction in the presence of excess sense VL (106 γlSalI) and antisense VH (106vhBclI) primers so that DNA encoding the individual 106 VH and VL domains were linked into a single coding region by overlap extension PCR.

Similarly, to create the 7 VL-VH sFv cassette, the 7 VL gene was reamplified from the pUC 19 sequencing construct using a sense PCR primer (7 γ2bSalI) that encoded a SalI site immediately prior to sequence encoding the first residue or the mature 7 VL; and an antisense primer (7 γ2bv1 LK3') complementary to sequence encoding the last nine residues of the VL and the first 12 residues of the (Gly₄Ser)₃ linker. DNA encoding 7 VH was reamplified from the pUC 19 sequencing construct with a sense primer (7 γ2bvhLK5') encoding the last 11) residues of the (Gly₄Ser)₃ linker followed by the first nine residues of the mature VH and an antisense primer (7 γ2bvhBclI) complementary to the sequence encoding the last nine amino acid residues of the VH region and a BclI site. DNA encoding 7 VH and VL were linked into a single coding region by overlap extension PCR using excess VL sense (7 γ2bSalI) and VH antisense (7 γ2bvhBclI) PCR primers.

**TABLE 8**

| Primers Used to Construct Clone 7 and Clone 6 sFv Expression Cassettes | |
|---|---|
| Primer | Sequence (5' to 3')¹ |
| | |
| 7 γ2bSalI | ATCGTCTAGGTCGACATTGTGCTGACACAGTCTCCTGTTTCC. SEQ ID. #19 |
| 7 γ2bVILK3' | GCCACCCGACCCACCACCGCCCGAGCCACCGCCACCCCGTCTT ATTTCCAACTTTGTCCC, SEQ ID. #20 |
| 7 γ2bVhLK5' | TCGGGCGGTGGTGGGTCGGGTGGCGGCGGATCTGAGGTCCAG CTGCAACAGTCTGGACCT.                    SEQ ID. #21 |
| 7 γ2bBclI | TCAGTGCTGATCAGAGGAGACTGTGAGAGTGGTGCCTTGGCC. SEQ ID. #22 |
| 106 γ I SalI | ATCGTCTAGGTCGACATCCAGATGACTCAGTCTCCAGCCTCC. SEQ ID. #23 |
| 106 γIVILK3' | GCCACCCGACCCACCACCGCCAGCGCCACCGCCACCCCGTTTC AGCTCCAGCTTGGTCCC.                                        SEQ ID. #24 |
| 106 γlVhLK5' | TCGGGCGGTGGTGGGTCGGGTGGCGGCGGATCTGAAGTGAAG CTGGTGGAGTCTGGGGGA.                                        SEQ ID #25 |
| 106 γlBclI | TCAGTGCTGATCAGAGGAGACGGTGACTGAGGTTCCTTGACC. SEQ ID. #26 |

| | |
|---|---|
| 1 Restriction sites underlined | |

The 106 and 7 VL-link-VH sFv gene cassettes were assembled for sFv-Ig expression in a variant of pUC19 called pUC-Ig that has been passed through a dam strain of *E. coli* (NEB 208) to allow restriction enzyme cutting at the *BelI* site. This vector contained the L6 Vκ leader sequence inserted as a *HindIII-SalI* fragment and a *BclI* site preceding sequence encoding the hinge-CH₂-CH₃ of human IgG₁ as cDNA. followed by a stop codon and an *XbaI* site. The cysteine residues in the hinge region were mutated to serines to favor the production of monomeric sFvlg (Hayden et al. 1994. Therapeutic Immunol. 1:3-15). The 106 and 7 VL-link-VH sFv gene cassettes were cut with *SalI* and *BcI* and were ligated into pUC-Ig DH5α *E. coli* were transformed with the constructs and colonies were screened for inserts.

The entire L6V_{κ} leader/VL-link-VH sFv/human Ig cassettes for both the 106 and 7 sFv were cut from pUC-Ig using *HindIII* and *XhaI* and were transferred to the pCDM8 mammalian expression vector. Following ligation of 7 and 106 sFv expression cassettes into the modified pCDM8 vector, the plasmids were amplified in MC1061/p3 *E. coli* cells and DNA was recovered and purified for transfection into COS cells.

### C. COS Cell Transfection, Purification, and Characterization of sFv-Ig Fusion Proteins

COS cells were transfected with expression plasmids as previously described (Linsley et al. 1991. J. Exp. Med. 173:721-730; Aruffo and Seed, 1987. Proc. Nat'l Acad. Sci. USA 84:8573-8577). Plasmid DNA was added to transfection media at 1µg/ml in a total volume of 12 ml/ 150 mm plate. After 7 to 10 days spent culture supernatant was pooled and cellular debris was removed by low-speed centrifugation

106 and 7 sFv-Ig were purified by applying clarified supernatant to a column of immobilized protein A (Repligen Corp., Cambridge, MA) equilibrated with 0.05 M sodium citrate, pH 8.0 (Linsley et al. 1991. J. Exp. Med. 173:721-730). For 500 ml of supernatant, 1 ml of packed bed volume of protein A was used. After loading the column (1ml/minute), the column was washed with 100 mM potassium phosphate. pH 8.0, and bound protein was eluted with 0.05 M sodium citrate, pH 3.0. Fractions were neutralized, pooled and dialyzed against PBS. Protein concentration was determined using a commercially available protein assay kit (Bio-Rad, Richmond, CA) based on the Lowry technique.

Expression levels and molecular size of the fusion proteins were determined by immunoprecipitation with protein A and SDS-PAGE, followed by Western blotting. Polyacrylamide gels forming a linear 6-15% gradient with a 4% stacker were run overnight at 10 mAmp. Gels were immunoblotted onto nitrocellulose membranes using a Western semi-dry transfer apparatus (Ellard Instruments. Seattle. WA) at 3 mAmp/cm² for 1 hour. Blots were blocked with 2% nonfat milk plus 0.1% Tween in PBS (blocking buffer) for I to 2 hours and then incubated with alkaline phosphatase conjugated goat anti-human IgG (Boehringer-Mannheim, Indianapolis, IN) al a 1:1500 dilution in blocking buffer for I hour. Blots were then washed three times with blocking buffer and were developed in Western blue (Promega, Madison. WI) for 5-15 min. before stopping color development by rinsing with distilled water.

The 7 sFv-Ig and 106 sFv-Ig proteins were tested for binding to human gp39 by an ELISA assay. Briefly, flat bottom flexible 96-well microtiter plates (Falcon) were coated overnight with rat anti-mouse Lyt2a monoclonal antibody 53-6 at 2 µg/ml in PBS at 4°C. After removing excess anti-mouse Lyt2a antibody, sgp39 as described earlier was added to the plates (100 µl per well) and incubated overnight at 4°C. Excess sgp39 was removed by washing, and clone 7 sFv-Ig or clone 106 sFv-Ig proteins (100 µl per well) were added. Plates were incubated for 2 hours at room temperature and washed twice with PBS containing 0.1% BSA. Goat anti-human IgG conjugated to horseradish peroxidase (American Qualex, Anaheim, CA) in conjugate buffer (Genetics Systems, Seattle, WA) was added (100 µl per well) and incubated for 1 hour at room temperature. Unbound conjugate was removed by two washes with PBS containing 0.1% BSA and 100 µl per well of a 1:100 dilution of EIA Buffered Substrate (Genetic Systems) was added to the wells. The color reaction was stopped with 30 µl per well of 3M H₂SO₄ and the optical density was measured at 450-595 nm with a Titertek multiwell plate reader.

Transfection supernatants from several clone 106 sFv-Ig and clone 7 sFv-Ig clones bound well to human gp39, and reacted very weakly (106) or not at all (7) on murine gp39. Representative results of 106 and 7 sFv-Ig binding are shown in Figure 8. Binding affinity determinations for the 106 sFv-Ig versus native 106 monoclonal antibody using purified radiolabeled protein were carried out. Saturation binding curves, shown in Figure 9, showed that labeled native 106 monoclonal antibody (Figure 9A) bound to Jurkat cells constitutively expressing gp39 with approximately three-fold greater affinity than 106 sFv-Ig (Figure 9B). However, the affinity of the 106 sFv-Ig was still quite high (measured Kd=1.6 x 10⁻⁹). It was determined that native 106 monoclonal antibody bound to 10,000 sites per cell by Scatchard transformation which is complete agreement with the number of sites per cell bound by 106 sFv-Ig (Figures 9A and 9B).

The ability of the 7 sFv-Ig and 106 sFv-Ig to inhibit the production of IgG and IgM in an *in vitro* T cell dependent B cell antibody production system and comparison of this effect with that seen for the parental 39-1.7 and 39-1.106 antibodies was assessed as described earlier for the parental antibodies with a few minor modifications. Cell cultures were initiated with 100,000 mitomycin C treated T cells and 2,000 B cells in Costar half area plates. Purified parental antibodies and their respective purified sFv.Igs were quantitated using a Bio-Rad Protein Assay kit. Each parental antibody was tested at final concentrations of 1, 0.5, 0.25 and 0.125 µg/ml. Each sFv-Ig was evaluated at final concentrations of 0.68, 0.34, 0.17, and 0.085 µg/ml. Although the concentration of parental antibody and its respective sFv-Ig in terms of µg/ml were different, the concentration of each with respect to the number of antigen binding fragments was equivalent when overall valency (two per parental antibody, one for its sFv-Ig) and molecular weight (160,000 kD for parental antibody, 55,000 kD for sFv-Ig) were taken into account. Thus, the final concentrations of antigen binding fragments (binding sites) compared in this experiment were 7.53, 3.76, 1.88, and 0.94 x 10¹² binding sites/ml.

Following addition of the antibodies and sFv-Igs, the plates were cultured in a humidified 37°C/6% CO₂ incubator for 10 days after which culture supernatants from triplicate wells were pooled and assessed for total human IgG and IgM as described earlier. Data are presented in Table 9 where Ig levels are expressed as a percentage of that observed with medium only (no anti-gp39 antibody) controls. As shown in Table 9, both the 7 sFv-Ig and 106 sFv-Ig were capable of substantially suppressing the production of both IgG and IgM by human B cells. Interestingly, their ability to suppress was at least equivalent and at some concentrations, even better, than that observed for the parental antibodies.

**TABLE 9**

| Suppression of *in vitro* Antibody Production by Whole Anti-Human gp39 Monoclonal Antibodies and their sFv-Ig Derivatives | | | | | | | | |
|---|---|---|---|---|---|---|---|---|
| Ab Conc. (binding sites x10¹²) | Inhibition of IgG Synthesis % of Medium Only Control | | | | Inhibition of IgM Synthesis % of Medium Only Control | | | |
| | 7 | 7 sFv-Ig | 106 | 106 sFvIg | 7 | 7 sFv-Ig | 106 | 106 sFv-Ig |
| 7.53 | 12 | 18 | 11 | 14 | 14 | 21 | 25 | 12 |
| 3.76 | 17 | 19 | 24 | 12 | 24 | 19 | 19 | 12 |
| 1.88 | 11 | 24 | 26 | 10 | 29 | 34 | 27 | 12 |
| 0.94 | 60 | 12 | 37 | 21 | 64 | 32 | 34 | 19 |

### EXAMPLE 12

### Humanization of Anti-gp39 Monoclonal Antibody

### A. Determination of Human Templates for 106 VL and VH

The murine 106 VL (kappa) and VH sequences were used to search a subset of immunoglobulin sequences from GenBank for murine germline nucleotide sequences with the closest homology to 106 VL with a FASTA search using only nucleotides encoding the mature peptide. This search produced two murine sequences followed by many human sequences, the best match being designated "Musigkva" (Accession No. J00545). The homology between the translated 106 VL and the translated J00545 (germline of 106) is shown in Figure 10. Only the differences are printed for the germline sequence. These differences are probable sites of somatic mutation. However, it is possible that 106 VL is derived from an as yet unidentified murine germline gene.

The human germline amino acid sequence with closest homology to 106 VL was determined by performing a FASTA search on a data base of immunoglobulin protein sequences. This data set contained both germline and rearranged sequences. After discarding the rearranged sequences, the best homology match was found with germline sequences designated "02" (Accession No. X59312) and "012" (Accession No. X593 15). It was noted that all but one (Leu90) of the structural determinants for the CDR loops were conserved, as was the size of the CDR loops between murine 106 VL and the human template. It was also noted that all of the CDR loops in the light chains of the murine sequence and human template belong to the same canonical structure class.

The murine nucleotide sequence with the closest homology to 106 VH was also determined by performing a FASTA search of the subset of GenBank immunoglobulin sequences using only nucleotides encoding the mature peptide as the query sequence. The search resulted in locating two murine sequences followed by many human sequences. The murine sequences designated "Musighin" (Accession No. M21520) showed significantly better homology than the other murine sequence. The GenBank annotation for M21520 lists it as a rearranged sequence. For the purpose of finding probable sites of somatic mutation, M21520 was used as a germline substitute and differences between it and 106 VH are shown in the bottom set of lines in Figure 11.

The human germline amino acid sequence with the closest homology to 106 VH was determined by performing a FASTA search on the immunoglobulin sequence data subset. After discarding the rearranged sequences, the best match was found with the "Hhg4" germline sequence (Accession No. X62129). It was noted that the size of the CDR loops was preserved between 106 VH and the human template and that all but two of the structural determinants for the CDR loops were conserved. None of the other highly homologous sequences gave a better fit in the structural determinants. The H I loops of the murine sequence and the human template were also found to belong to the same canonical structure class

### B. Refinement of 106 VL and VH Humanization Templates.

The canonical loop structures for the antigen binding loops L1, L2 and L3 of the VL domain and H1 and H2 of the VH domain were identified, and residues that were defined as structural determinants (Chothia and Lesk, 1987. J. Mol. Biol. 196-901; Lesk and Tramontano, In Antibody Engineering W.H. Freeman and Co., pp 1-38 (1992)) were retained as murine residues.

The murine 106 VL and VH amino acid sequences were used to search the Brookhaven databank for homologous sequences in which the crystal structure had been solved. The VL from the anti-lysozyme binding monoclonal antibody D1.3 was selected as a structural template for modeling of the 106 VL. The VH from the antipeptide monoclonal antibody 17/9 was chosen as a structural template for modeling of the 106 VH. These structures were combined to provide a composite template for 106 modeling using the set of invariant residues at the VL-VH interface. From the model, three additional framework residues which appeared to be important for maintaining the structure of the antigen binding sites were identified. In the VL, Ile48 was found to be structurally important and was retained as murine sequence. In the VH, two residues (Ala49 and Ile77) were also retained as murine sequence. The 106 model was not determinative of whether a human or murine residue was appropriate at positions 24, 55 and 56 of 106 VH

### C. Determination of the J-region Templates

The best human Jκ sequence was selected by homology to the murine Jκ sequence in Kabat et al. (Sequences of Proteins of Immunological Interest, 4th Edition, U.S. Health and Human Services, Washington, D.C. (1987)). Similarly, the best human JH sequence was selected by homology to the murine JH sequence in Kabat et al., *supra.*

### D. Humanization of the 106 VL

The oligonucleotide primers used to humanize the 106 VL are listed in Table 8. The first three changes (Ala at position 9 to Ser, Glu at position 17 to Asp, and Thr at position 18 to Arg) were encoded on the Hu106VLAre2 sense PCR primer. A *HindIII* site was added immediately 5' of the sequence encoding the mature VL for cloning the final humanized VL into pUC19. The next four changes were encoded in the Hu 106VLB2 antisense PCR primer (Gln at position 40 to Pro, Arg at position 42 to Lys, Ser at position 43 to Ala, and Gin at position 45 to Lys). Using Hu106VLAre2 and Hu106VLB2 with murine 106 sFv-Ig/CDM8 as template, the first humanized fragment was obtained by PCR. The sense PCR primer Hu106VLC and the antisense PCR primer 2Hu106VLD were used to humanize the second fragment. The sequence of Hu106VLC overlapped Hu106VLB2 such that the same four changes were encoded on Hu106VLC (Gln at position 40 to Pro, Arg at position 42 to Lys, Ser at position 43 to Ala, and Gln at position 45 to Lys). In addition, an *SpeI* site was engineered into Hu106VLC as a diagnostic site. This change did not alter the protein sequence. The 2Hu106VLD primer encoded the next four changes (Gln at position 70 to Asp, Ser at position 72 to Thr, Lys at position 74 to Thr, and Asn at position 76 to Ser). Using Hu106VLC and 2Hu106VLD with murine 106 sFv-Ig/CDM8 as template, the second humanized fragment was obtained by PCR.

**TABLE 10**

| | Primers Used for 106 VL Humanization |
|---|---|
| Hu106VLAre2 96-mer sense | 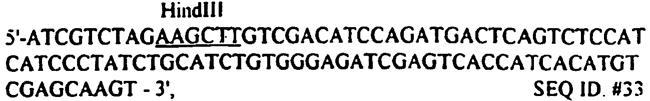 |
| Hu106VLB2 45-mer antisense | 5'-TAGTAGCTTAGGTGCCTTTCCAGGTTTCTGCTGATACCAAGCT AA - 3', SEQ ID. #34 |
| Hu106VLC 60-mer antisense | SpeI 5'-CCTGGAAAGGCACCTAAGCTACTAGTCTATAATGCAAAACCTT AGCAAAAACCTTAGCA - 3', SEQ ID. #35 |
| 2Hu106VLD 45-mer antisense | 5'-GAGATCGTCAGTGTAAAGTCTGTGCCTGATCCACTGCCACTGA AC - 3'. SEQ ID. #36 |
| Hu106VLE 75-mer sense | 5'-GACTTTACACTGACGATCTCAAGCCTGCAGCCTGAAGATTTTG CAACTTATTACTGTCAACATCATTATAATACT - 3'. SEQ ID. #37 |
| Hu106VLF 82-mer anti-sense | XbaI 5'- TCAGTGCTTCTAGAGCCACCCCGTTTGATCTCGACCTTGGTC CCTCCACCGAACGTGAGCGGAGTATTATAATGATG TTGAC-3' SEQ ID. #38 |
| Hu 106VLA2 24-mer sense | 3'-ATCGTCTAGAAGCTTGTCGACATC - 3'. SEQ ID. #39 |
| Hu106VLF2 24-mer anti-sense | 5'-TCAGTGCTTCTAGAGCCACCCCGT - 3'. SEQ ID. #40 |

The final humanized VL fragment was obtained using the Hu106VLE sense PCR primer and Hu 106VLF antisense PCR primer with murine 106 sFv-Ig/CDM8 as template. Hu106VLE partially overlapped 2Hu106VLD such that it encoded the same four changes (Gln at position 70 to Asp. Ser at position 72 to Thr, Lys at position 74 to Thr, and Asn at position 76 to Ser). Additionally, Hu106VLE encoded two additional changes (Gly at position 84 to Ala and Ser at position 85 to Thr). Hu 106VLF encoded the last four changes (Thr at position 100 to Gly, Leu at position 104 to Val, and Leu at position 106 to Ile). Hu106VLF also encoded an *XhaI* site immediately 3' of the VL sequence for cloning purposes. Humanized fragments 2 and 3 were then assembled and amplified by PCR by mixing the two humanized DNAs together in the presence of Hu 106VLC sense primer and Hu 106VLF antisense primer. This piece was purified, mixed with humanized fragment 1 and reamplified by PCR in the presence of the sense primer Hu106VLA2 and the antisense primer Hu106VLF3 such that a single PCR fragment was obtained. The amplified humanized 106 VL was then cut with *Hind*III and *Xba*I and ligated into pUC19. *E. coli* (strain DH5α) were transformed as usual and plasmid DNA from individual clones was sequenced to verify proper fragment assembly of the humanized 106 VL. One individual clone was designated hu106VL clone 10 and was used in the later constructions.

### E. Humanization of the 106 VH

Since the murine 106 model was not determinative of whether a human or murine residue was appropriate at positions 24, 55 and 56 of the humanized VH chain, an attempt was made to produce all 23 versions. The humanized VH genes were assembled in three steps. In the first step, PCR fragments encoding either of 2 versions (corresponding to Ala or Thr at amino acid position 24) of the amino acid sequence from position I to position 51 were generated and inserted into the *Hind*III *Xha*I sites of pUC19. These PCR products included the introduction of a unique *Nhe*I site (at nucleotide position 146), followed by *EcoR*I, *Pst*I and *Xha*I sites for the later insertion of other fragments. These changes did not affect the protein sequences of 106 VH. In the second step, oligonucleotides encoding any of four different versions (corresponding to Asp-Ser, Asp-Tyr, Ser-Ser, and Ser-Tyr at amino acid residue positions 55 and 56) of amino acids residues 49 through 80 were annealed and inserted into the *Nhe*I and *Pst*I sites of both of the vectors generated in step 1. Seven of the possible eight different vectors were isolated (the Ala-Asp-Ser form was not). In the third step a single PCR product encoding amino acid residues 80 through 112, which contained the nucleotide sequence encoding the remaining residues for humanized 106 VH(Figure 12), was generated and inserted into the *Pst*I and *Xba*I sites of the seven vectors produced in step 2.

**TABLE 11**

| | Primers Used for 106 VH Humanization |
|---|---|
| 106vhT-5' 106-mer sense | HindIII 5'-ATCGTCTAGAAGCTTGAAGTGCAGCTGGTGGAGTCTGGAGG AGGCTTAGTGAAGCCTGGAGGGTCCCTGAGGCTCTCCTGTGCA ACCTCTGGATTCACTTTCAATA - 3'     SEQ ID. #41 |
| 106VHA-5 106-mer sense | HindIII 5'- ATCGTCTAGAAGCTTGAAGTGCAGCTGGTGGAGTCTGGAGG AGGCTTAGTGAAGCCTGGAGGGTCCCTGAGGCTCTCCTGTGCA GCCTCTGGATTCACTTTCAATA - 3'     SEQ ID. #42 |
| 106vhNEP-3' 87-mer anti-sense | Xbal Pstl EcoRI Nhel 5'-TCAGTGCTCTAGAACCCTGCAGATCGAATTCAATGCTAGCG ACCCACTCCAGTCCCTTACCTGGTGCCTGGCGAACCCAAGACA TGG - 3' SEQ ID. #43 |
| 106vhSY-5' 97-mer sense | NheI 5'- CTAGCATTAGTAGTGGTAGTTACATCTACTATGCTGACAGT GTGAAAGGCCGATTCACCATCTCGAGAGATAATGCCAAAAA CATCCTGTATCTGCA - 3'     XhoI     SEQ ID. #44 PstI |
| 106vhSY-3' 89-mer anti-sense | XhoI 5'- GATACAGGATGTTTTTGGCATTATCTCTCGAGATGGTGAAT CGGCCTTTCACACTGTCAGCATAGTAGATGTAACTACCACTAC TAATG - 3' SEQ ID. #45 |
| 106vhDY-5' 97-mer sense | NheI 5'- CTAGCATTAGTAGTGGTGATTACATCTACTATGCTGACAGT GTGAAAGGCCGATTCACCATCTCGAGAGATAATGCCAAAAA CATCCTGTATCTGCA - 3'          XhoI          SEQ ID. #46 PstI |
| 106vhDY-3' 89-mer anti-sense | XhoI 5'- GATACAGGATGTTTTTGGCATTATCTCTCGAGATGGTGAAT CGGCCTTTCACACTGTCAGCATAGTAGATGTAATCACCACT ACTAATG - 3'                                                    SEQ. ID #47 |
| 106vhSS-5' 97-mer sense | NheI 5'- CTAGCATTAGTAGTGGTAGTAGCATCTACTATGCTGACAGT GTGAAAGGCCGATTCACCATCTCGAGAGATAATGCCAAAAA CATCCTGTATCTGCA - 3'.          XhoI          SEQ ID. #48 PstI |
| 106vhSS-3' 89-mer anti-sense | XhoI 5'- GATACAGGATGTTTTTGGCATTATCTCTCGAGATGGTGAAT CGGCCTTTCACACTGTCAGCATAGTAGATGCTACTACCACT ACTAATG - 3'                                                    SEQ ID. #49 |
| 106vhDS-5' 97-mer sense | NheI 5'- CTAGCATTAGTAGTGGTGATAGCATCTACTATGCTGACAGT GTGAAAGGCCGATTCACCATCTCGAGAGATAATGCCAAAAA CATCCTGTATCTGCA - 3'     XhoI     SEQ ID. #50 PstI |
| 106vhDS-3' 89-mer anti-sense | XhoI 5'- GATACAGGATGTTTTTGGCATTATCTCTCGAGATGGTGAAT CGGCCTTTCACACTGTCAGCATAGTAGATGCTATCACCACT ACTAATG-3'                                                    SEQ ID. #51 |
| 106vhPst5' 78-mer sense | PstI 5'- ATCGTCTAGCTGCAGATGAACAGTCTGAGGGCAGAGGACAC GGCCGTCTATTACTGTGCAAGGCACTATGATTACGAC - 3' SEQ ID #52 |
| 106vhXba3' 69-mer anti-sense | Xbal          Bcll 5'- TCAGTGCTCTAGATGATCAGAGGAGACGGTGACCAGGGTTC CTTGACCCCAGTAGTCCATAGCATAGCT- 3'     SEQ ID. #53 |

In greater detail, construction of the two vectors was initiated by generating two PCR fragments using 106sFv-Ig/CDM8 as template and either 106vhT-5' or 106vhA-5' as the sense primer and 106vhNEP-3' as the antisense primer. The sense primers encoded a *Hind*III site immediately 5' of the VH and the first three humanized VH changes (Lys at position 3 to Gln, Lys at position 19 to Arg, and Thr at position 23 to Ala). In addition, the 106vhA-5' sense primer humanized the residue at position 24 to Ala whereas the 106vhT-5' sense primer kept the residue as murine (Thr). The antisense primer encoded changes at residues 40, 42 and 44 (Thr to Ala, Glu to Gly and Arg to Gly, respectively) and also encoded four unique restriction sites (*NheI, EcoRI*, *PstI*, and *XbaI).* The two PCR'ed DNAs were then cloned into pUC 19 as *HindIII-XbaI* fragments and were used to transform DH5α *E*. *coli.* Clones containing both inserts were isolated (106vhA-NEP and 106vhT-NEP) and verified by DNA sequencing. The plasmids were then digested with *NheI, EcoRI* and *PstI* and linear DNA was isolated and purified.

The sense oligonucleotide in each of the four pairs of oligonucleotides that encoded the changes at positions 55 and 56 were phosphorylated and annealed to the corresponding antisense oligonucleotide. This generated dsDNA fragments that had a 5' *NheI* overhang and a 3' *PstI* overhang, and that contained a unique *XhoI* site. The primer pair 106vhDS-5' and 106vhDS-3' encoded murine residues at positions 55 and 56 (Asp-Ser); 106vhDY-5' and 106vhDY-3' encoded murine and human residues at positions 55 and 56, respectively (Asp-Tyr); 106vhSS-5' and 106vhSS-3' encoded human and mouse residues at positions 55 and 56, respectively (Ser-Ser); and 106vhSY-5' and 106vhSY-3' encoded human residues at positions 55 and 56 (Ser-Tyr). All of the primer pairs also encoded four additional changes from murine to human sequence (Thr to Ile at position 57, Pro to Ala at position 60, Arg to Lys at position 64 and Arg to Lys at position 75). The four fragments that were generated were then ligated into 106vhA-NEP/pUC19 and 106vhT-NEP/pUC19 previously digested with restriction enzymes. The plasmids were used to transform DH5α *E*. *coli* and DNA from clones that cut with *XhoI* were isolated and verified by DNA sequencing. Of the eight combinations, seven were obtained (106vhA-DS, representing human, mouse, mouse sequence at positions 24, 55 and 56, was never isolated from pUC19). The seven plasmids were digested with *PstI* and *XbaI* and were now ready to receive the final fragment. Three of the clones were selected for later use and were designated as follows: VH ASY 24-17 (hhh); VH TDS 15-34 (mmm); and VH ADY 7-8 (hmh).

The remaining residues that were changed to human sequence were encoded on sense primer 106vhPst5' (Ser to Asn at position 82a, Ser to Ala at position 84, and Met to Val at position 89) and antisense primer 106vhPst3' (Ser to Leu at position 108). For cloning purposes, primer 106vhPst5' also encoded a *PstI* site and 106vhPst3' encoded an *XhaI* site with a *BcII* site immediately preceding it. This fragment was obtained by PCR using 106sFv-Ig/CDM8 as template. The fragment was digested with *PstI* and *XbaI* and was ligated into the seven plasmids. Once again, the plasmids were used to transform DH5α *E. coli* and DNA from clones was sequenced to verify insertion.

### F. Assembly of Humanized 106 sFv Gene Cassettes

The humanized single chain Fv expression cassettes for 106 were assembled as for the original murine 106 sFv but using the following primers:

**TABLE 12**

| | Primers Used for Construction of Humanized 106 sFv Gene Cassettes |
|---|---|
| hu106V_{L}Sall 45-mer sense | SalI 5'- ATCGTCTAGGTCGACATCCAGATGACTCAGTCTCCA TCATCC - 3'                                        SEQ ID. #54 |
| hu106V_{L}LK3' 60-mer anti-sense | 5'- GCCACCCGACCCACCACCGCCAGCGCCACCGCCACC CCGTTTGATCTCGACCTTGGTCCC - 3'     SEQ ID. #55 |
| hu106V_{H}LK5' 60-mer sense | 5' -TCGGGCGGTGGTGGGTCGGGTGGCGGCGGATCTGAA GTGCAGCTGGTGGAGTCTGGAGGA - 3'     SEQ ID. #56 |
| hu106V_{H}BcII 45-mer anti-sense | BclI 5'- TCAGTGCTGATCAGAGGAGACGGTGACCAGGGTTCC TTGACC - 3'                                                    SEQ ID. #57 |

Briefly, DNA encoding the humanized 106 VL was cut from the pUC19 vector in which it was assembled. DNA encoding the seven humanized 106 VH were also cut from pUC19. The DNA fragments were purified and used in the following PCR reactions. The humanized 106 VL was amplified using the hu106V_{I.}SalI sense PCR primer that encoded a *SalI* site immediately prior to the first residue of the mature VL and an antisense primer (hu106V_{L}LK3') that was complementary to sequence encoding the last nine residues of the VL and the first 12 residues of the (Gly₄Ser)₃ linker. Additionally, the seven humanized 106 VH were amplified using a sense primer (hu 106V_{H}LK5') encoding the last 11 residues of the (Gly₄Ser)₃ linker followed by the first nine residues of the mature VH and an antisense primer (hu106V_{H}BclI) complementary to sequence encoding the last nine residues of the VH region and a *BclI* site. The modified 106 VL was mixed with each of the modified 106 VH DNA in the presence of excess VL sense primer (hu106V_{I.}SalI 5') and VH antisense primer (hu106V_{H}BclI) so that the individual humanized 106 VL was linked with each of the individual humanized 106 VH into seven VL-link-VH single coding regions by overlap extension PCR.

The humanized 106 VL-link-VH sFv gene cassettes were then assembled for sFv-Ig expression in the pUC-Ig vector. This vector contains the L6 V_{κ} leader sequence followed by a *SalI* site and a *BclI* site preceding sequence encoding the hinge-CH2-CH3 of human IgG1 and a stop codon flanked by an *XbaI* site. The hinge cysteines were mutated to serines to favor monomeric expression of sFv-Ig fusion protein. The humanized 106 VL-link-VH sFv gene cassettes were cut with *SalI* and *BclI* and were ligated into similarly digested pUC-Ig. DH5α *E*. *coli* were transformed with the constructs and colonies were screened for inserts. Six of the seven VH constructs were properly inserted into pUC-Ig. The entire L6 V_{κ} leader/humanized 106 VL-link-VH sFv/human Ig cassettes were cut from pUC-Ig using *HindIIII* and *XbaI* and were transferred to the pCDM8 mammalian expression vector and were amplified by transformation in *E*. *coli* strain MC1061/p3. Of the six, five were inserted properly into pCDM8. DNA was recovered from each for COS cell transfections.

Small-scale COS cell transfections were carried out in 60 mm tissue culture plates by the DEAE-dextran method. Three ml of transfection supernatant was recovered from each after three days of culture and were tested for the presence of soluble sFv-Ig fusion protein by Western blot and ELISA. In addition, an anti-human Ig sandwich ELISA was performed to quantify the amount of protein expressed by each construct and the on-rates of the different proteins binding to gp39 were measured by Biacore analysis.

### G. Preliminary Analysis of Humanized 106 sFv Expressed by Transient Transfection in COS Cells.

SDS-PAGE and Western blot of the transfection supernatants showed that of the five constructs used to transfect COS cells (humanized 106 sFv containing the 106 VH fragments 106vhT-DS, 106vhT-SS, 106vhT-SY, 106vhA-DY and 106vhA-SY), four secreted protein into the supernatant. There was no protein expressed by humanized 106 sFv containing 106vhT-SY (huVL/106vhT-SY). Of the four expressors, three expressed protein of correct size for an sFv-Ig fusion protein (55 kDa). HuVL/106vhT-SS produced a protein of aberrant size (approximately 97 kDa). Expression levels for HuVL/106vhT-DS appeared to be similar to murine 106 sFv while HuVL/106vhA-DY and HuVL/106vhA-SY expressed at lower levels.

The protein levels were quantified using a sandwich ELISA to detect the human Ig tail. ELISA plates were coated with goat anti-human Ig in PBS and blocked in PBS + 0.1% BSA. The transfection supernatants were incubated neat and at a 1:5 dilution for 1 hr at RT. The plates were then washed and incubated with goat anti-human Ig-horseradish peroxidase in ELISA conjugate buffer for I hr at RT. Plates were washed again and a 1:100 dilution of EIA Buffered Substrate (Genetic Systems Corporation) was added. The color reaction was stopped with 3M H₂SO₄ and the optical density was measured at 450-595 nm with a Titertek multiwell plate reader. Approximate protein concentrations were determined by comparison to a known concentration of CD40Rγ1 (CD40-Ig) that had been determined with the Bio-Rad protein concentration kit. The protein concentrations were:
huVL/106vhA-DY (clone 10) 0.62 µg/ml
huV1./106vhA-DY (clone 12) 0.82 µg/ml
huVL106vhA-SY (clone 21) 0.77 µg/ml
huVL/106vhT-SY (clone 26) 0 µg/ml
huVL/106vhT-SS (clone 36) 0.15 µg/ml
huVL/106vhT-DS (clone 46) 1.20 µg/ml

The supernatants were tested for their ability to block E-selectin expression on endothelial cells. Human umbilical vein endothelial cells (HUVECs, Clonetics Corporation) were cultured and stimulated in M199 (Medium 199, Gibco BRL) with additions to final concentrations as follows: 4 mM L-glutamine, 48.5 µg/ml penicillin, 80 µg/ml streptomycin, 1 mM sodium pyruvate (Sigma), 90 µg/ml heparin (Sigma), 30 µg/ml endothelial growth supplement (Collaborative Biomedical Products) and 20% fetal bovine serum. Endothelial cells were grown in tissue culture flasks treated with 1% gelatin, and plated at 1.5 x 10⁴ cells/well in flat-bottomed 96-well Costar tissue culture plates that had been coated with I µg/well fibronectin (Collaborative Biomedical Products). Endothelial cells were stimulated 1-2 days after plating. Cells were used at passage 4 or 5.

sgp39 and supernatants containing humanized 106 sFv-Igs were added in M199 plus additions in 100 µl per well and incubated at 37°C for 4 hours prior to assaying for E-selectin expression. Plates were then washed twice with cold PBS, fixed for 10 minutes with 0.5% glutaraldehyde in PBS at 4°C, washed four times with 3% goat serum/PBS/20 mM EDTA (blocking buffer) and blocked 1 hour at 37°C or overnight at 4°C in the same buffer. Cells were treated with 100 µl anti-E- and P-selectin (R&D Systems) at 0.25 µg/ml in blocking buffer for 1 hour at 37°C. Plates were washed four times with blocking buffer, incubated 1 hour at 37°C with horseradish peroxidase conjugated anti-mouse IgG in blocking buffer (Jackson ImmunoResearch, 100 µl/well, 1:2000 dilution) then washed four times. Plates were developed using EIA chromagen reagent in EIA buffered substrate (both from Genetic Systems, 100 µl/well, 1:100 dilution) and stopped with 100 µl per well of I N H₂SO₄. The absorbance was determined at dual wave lengths of 450 nm and 630 nm.

HuVL/106vhA-DY, huVL/106vhA-SY and huVL/106vhT-DS all inhibited E-selectin expression although not as effectively as the original murine 106 sFv-Ig (Figure 13). Differences may be accounted for by lower protein expression in huVL/106vhA-DY and huVL/106vhA-SY, although huVL/106vhT-DS appeared to express at levels comparable to the original murine 106 sFv-Ig.

The on-rates of the different proteins binding to gp39 were determined using the Biacore. HuVL/106vhA-SY and huVL/106vhT-DS both bound tightly to chips coated with gp39, with activity comparable to the original murine 106 sFv-Ig (Figure 14). Since these proteins did not come off, it was unclear whether the affinities of these sFv-Ig were very high (the profiles indicated affinities of Kd ∼ 10⁻¹⁰ M or greater) or whether the proteins were aggregated and were binding multivalently. HuVL/106vhA-DY did come off. From its profile, affinity was estimated to be approximately Kd = 10⁻⁷ to 10⁻⁸ M. The original murine 106 sFv-Ig had been measured at Kd = 1.6 x 10⁻⁹ M so it appears that huVL/106vhA-SY and huVL/106vhT-DS are high affinity humanized anti-gp39 sFv.

HuVL/106vhA-SY and huVL/106vhT-DS were found to bind tightly to human gp39 and show functional activity in inhibiting E-selectin expression on endothelial cells. Although huVL/106vhA-SY appears to express at lower levels than huVL/106vhT-DS, it is the "most human" of the humanized 106 sFv.

### EXAMPLE 13

### Generation of Humanized Monoclonal Antibody 106 As A Whole Antibody

Using the humanized 106 sFv-Igs as a template, the variable light chains and three forms of the variable heavy chain were amplified by PCR using primers that introduced a sequence encoding the signal sequence of the human antibody template. These PCR products were inserted into a vector containing sequences encoding the constant regions of human kappa (κ) or of a human gamma 1 (γl) to generate the complete light or heavy chain, respectively. These vectors also included appropriate drug resistance genes for the generation and amplification of stable cell lines expressing the protein. Proteins from each of the three constructs, (see Table 13 for nomenclature) were produced by transient expression in COS cells. These proteins were tested as crude supernatants in assays for the inhibition of B cell proliferation using soluble gp39 (sgp39) in the presence of anti-human IgM, the production of antibodies by B cells stimulated with activated T cells, and the induction of E-selectin expression on endothelial cells by a gp39' T cell line.

The following briefly describes the construction of the expression vectors for humanized anti-gp39 monoclonal antibody 106 with amino acids Thr, Asp, Ser at positions 24, 55 and 56, respectively (designated h106-2). Construction of the expression vectors went through a series of intermediate plasmids wherein various functional regions and restriction sites were manipulated. Also, the various regions encoding the required variable and other immunoglobulin domains were assembled.

**TABLE 13**

| Nomenclature | | | |
|---|---|---|---|
| Sequence at position | Alternative Protein Names | | |
| 24,55,56 | | | |
| TDS | mmm | hK1 | h106-2 |
| ADY | hmh | hK6 | |
| ASY | hhh | hK8 | h106-1 |

Briefly, three plasmids, pD13, pD16 and pD17, used in constructing the final vectors and expression cassettes were initially constructed.

### A. Construction of pD13

Plasmid pD13 was constructed and derived from the pcDNA3 plasmid (Invitrogen) in two steps. First, the SV40 promoter/enhancer and neomycin resistance gene was removed by digestion with *Nae*I and isolation of the 3.82 kb fragment. This was replaced by the SV40 promoter/enhancer and dhfr gene from pSV2-dhfr. The pSV2-dhfr sequence was isolated as a 1.93 kb fragment after digestion with *Pvu*II and *BamH*I. The 3.82 and 1.93 kb fragments were ligated together and used to transform MC1061 bacteria following filling in the protruding ends of the 1.93 kb fragment from pSV2-dhfr. The correct product (designated pD12) was confirmed by the release of an 890 bp fragment following *Hind*III digestion.

In the second step, the polylinker was replaced with alternative restriction sites by digesting the resultant vector above with *Asp*718 and *Bsp* 1201. Oligonucleotides LH7 (Seq. ID #64) and LH8 (Seq. ID #65) were annealed and cloned by ExoIII cloning (K. Hsio, 1993, Nucl. Acid. Res. 21:5528-5529) was used to complete the plasmid pD13. The resulting plasmid was used to transform competent *E. coli* DH5α and the correct product was confirmed by sequencing the polylinker region.

### B. Construction of pD 16 and pD 17

Plasmid pD16 was derived from the pcDNA3 plasmid (Invitrogen) in a series of steps which add (1) a polylinker sequence upstream of the CMV promoter for linearization, (2) deleting the SV40 promoter/enhancer and neomycin resistance gene and replacing them with the histone H3 transcription termination sequence, the SV40 promoter (enhancer deleted) and DHFR gene, and (3) insertion of the gastrin transcription termination sequence upstream of the CMV promoter. Plasmid pD 17 was derived from pD16 by the removal of the Nhel site from the linearization polylinker.

Plasmid pD16 was derived from pcDNA3 (Invitrogen) by first digesting with *Bg*/II and ligating oligonucleotides LH I (Seq. ID #58) and LH2 (Seq ID #59) into the plasmid after annealing the oligonucleotides. After ligation, the resulting plasmid (pcDNA3-LSI) was used to transform competent *E. coli* DH5α and the correct construct was confirmed by release of a 230 bp fragment following restriction enzyme digestion with *Nhe*I and *Nru*I*.*

Plasmid pcDNA3-LSI was then digested with *NgoM*I*, Pvu*I and *Bsm*I. Following digestion, a 2.0 kb *NgoM*I*-Pvu*I fragment was isolated. Plasmid pD 1 2 (described above) was digested with *Pvu*I and *Sph*I to remove the SV40 enhancer and a 3.6 kb fragment was isolated. Oligonucleotides LH3 (Seq. ID #60) and LH4 (Seq ID #61), encoding the histone H3 transcription termination sequence were annealed and then ligated with the 2.0 kb *NgoM*I*-Pvn*I fragment and 3.6 kb PvuI-*Sph*I fragment. The resulting plasmid pcTwD-LS I was confirmed by the production of 3.3, 0.95, 0.82 and 0.63 kb fragments after digestion with *Nhe*I plus *Nci*I and the production of 4.2, 1.0, 0.26 and 0.23 kb fragment after digestion with *Sph*I plus *BstE*II.

Insertion of the gastrin transcription termination sequence to form plasmid pD16 was accomplished by digesting pcTwD-LS I with *BssH*II and *Nar*I and isolating the 5.7 kb fragment and lighting with the annealed oligonucleotides LH5 (Seq ID #62) and LH6 (Seq ID #63). After ligation, the product was used to transform competent *E*. *coli* MC1061 and the correct construction was confirmed by the production of 4.8, 0.66 and 0.31 kb fragments after digestion with *NgoM*I plus *Spe*I and the production of 3.3, 1.0, 0.82 and 0.67 kb fragments following digestion with *NgoM*I plus *Nco*I*.*

Plasmid pD17 was derived from pD16 by digestion with *Bst*II and *Nhe*I and filling the protruding ends using Klenow polymerase. The reaction mixture was self-ligated and used to transform competent *E*. *coli* DH5α. This step removed the *Nhe*I site from the linearization polylinker and was important for a later step in the construction of pD17-hGla and pD17-hK8.HI I described later.

### C. Construction of the light chain expression vector

The expression vector for the light chain of humanized 106 was constructed in two phases. First, the light chain expression cassette containing the human Cκ gene is constructed and this is followed by construction of the completed light chain expression vector pD16-hK8.LI (Figure15).

Briefly, a 2.9 kb EcoRI fragment was isolated from pGk.II (Walls et al., 1993. Nucl. Acid. Res. 21:2921-2929) and this was ligated into the plasmid pD13 (described above) previously digested with *EcoR*I*.* This construct (pD 13-hCka) containing the human Cκ exon and flanking intron sequences was used to transform *E*. *coli* DH5α and the correct product was confirmed by restriction digestion Digestion with *EcoR*I resulted in fragments of 5.7, 2.8 and 0.3 kb and digestion with *Sac*I resulted in fragments of 7.1, 1.1 and 0.5 kb.

Construction of the light chain expression cassette was completed by removing the Cκ fragment along with the flanking polylinker sequences from pD13 and inserting it into pD 16. Plasmid pD 13-hCka was digested with *Asp*7181 and *Bsp*1201 to release the Cκ fragment and polylinker sequences. The same enzymes were used to linearize pD16 and the Cκ containing fragment was ligated into pD16 to form pD16-hCka. Following transformation of DH5α *E. coli* and amplification, the correct construct was confirmed by the release of 2.9 kb fragment following digestion with *Asp*7181 and *Bsp*120I and linearization following digestion with a restriction enzyme present in pD16, but not pD13. The nucleotide sequence was also confirmed by sequencing various regions of the construct.

In the second phase of construction of the light chain expression vector an oligonucleotide sequence encoding a signal peptide and the h106Vk sequence was assembled and inserted into the EcoRV and XhoI sites of pD16-hCka. The oligonucleotide sequence encoding the signal sequence and the h106Vk sequence was assembled by PCR SOEing (splice by overlapping extension reactions) (Ho, S.N. et al., 1989, Gene 77:51-59) using hu106VL clone 10 as a template and primers LH9 (Seq. ID # 66), LH 10 (Seq. ID # 67), LH 11 (Seq ID #68) and LH 12 (Seq. ID # 69). In combination, primers LH9, LH10 and LH11 encode an *Eco*RV restriction site, a signal peptide, and the 5' end of hu 106 Vk. Primer LH12 encodes Jκ, intron sequences, and an *XhoI* restriction site. The complete construct was used to transform competent *E*. *coli* DH5α by homologous recombination (Babek et al., 1993, Nucl. Acids Res. 21:3601-3602). Plasmid DNA from colonies was screened by PCR for the presence of a 441 bp insert following digestion with *Asp*718I and *Xho*I*.*

The final construct of the plasmid is depicted in Figure 15 and was further confirmed by sequencing the entire V region insert and some of the flanking regions. Clones containing the correct fragment sizes were designated pD16-hK8.L1A, pD16-LK8.L1B, pD16-hK8.L1C. Sequencing of clone pD16-hK8.LIA discovered one mutation in the nucleotide sequence of the clone which did not appear to disrupt the ability of the sequence to express protein and did result in mature light chains. The nucleic acid sequence (Seq. ID # 76) and amino acid residue sequence (Seq. ID #77) are depicted in Figure 16. The mutation changed the initiation Methionine signal sequence to ATC, but there is another Methionine coded for two amino acid codons downstream.(Figure 16).

### D. Construction of the heavy chain expression vector

Expression vectors for the humanized whole antibody 106 heavy chain were also constructed in two phases. In the first phase, DNA encoding exons for CH1, CH2 and CH3 of human gamma I constant region were inserted into pD17. In the second phase, variable regions of monoclonal antibody 106 were assembled and ligated into the vector containing the sequences for the heavy chain constant regions.

Using pNγ1.12 (Walls et al., 1993, *Nucl. Acid. Res. 21*:2921-2929) as a template the human gamma I gene was amplified by PCR using primers LH 13 (Seq. ID #70) and LH14 (Seq. ID # 71). The sense primer (LH13) contained a ClaI site, for cloning into pIC20R, followed by the human γ1 5' CH 1 domain sequences. The nucleotides encoding the first two amino acid residues (Alanine and Serine) were mutated so as to encode an Nhel restriction site (GCTAGC). LH 14, the antisense primer had a unique BstEII site in CH 1 followed by an Xhol site for cloning into pIC20R. The 0.2 kb PCR fragment and pIC20R(previously digested with *Cla*lI and *Xho*I) were briefly treated with Exonuclease III and used in transformation of *E*. *coli* DH5α. Colonies containing inserts were determined by PCR and the DNA sequence was verified. The resultant plasmid was designated pIC-hGINhe.Bst.

The remaining portions of the human genomic immunoglobulin gamma I gene were inserted into the expression cassette by digesting pNg1.16 (Walls et *al., supra)* with *Hind*III and *BamH*I*.* The *Hind*III site is just 5' of CH I, while the *BamH*I site is 3' of the CH3 domain. plC-hGlNhe.Bst was digested with *Hind*III and *BamH*I and ligated with the *Hind*III*-BamH*I fragment of pNg1.16. Transformation resulted in a plasmid carrying the 5' end of the gamma I gene followed by the full gamma I gene. This was confirmed by release of 3.1 and 2.6 kb fragments when the plasmid was digested with *EcoR*I*.*

The repeated portion of the gamma 1 gene was removed by digestion with *BstE*II*,* self-ligation of the plasmid and transformation. Deletion of the repeat was confirmed by DNA sequencing the regions around the *BstE*II site. The resulting vector, designated pIC-hGINhe.Bam, contains the human gamma I gene beginning at codon I (with an artificial NheI site) through the *BamH*I site 3' of the human gamma I CH3 domain.

pIC-hGINhe.Bam was then digested with *Nhe*I and *BamH*I to release the gamma I insert. The gamma 1 insert was ligated into pD 13 (described above), which had been previously digested with *Nhe*I and *BamH*I*,* and used to transform *E*. *coli* DH5α. The correct product designated pD13.hGI a was confirmed by the production of 5.1 and 3.25 kb fragments after digestion with *Bg*/II and the production of 3.55, 3.25 and 1.54 kb fragments after digestion with *Bgl*II and *BamH*I*.*

The expression cassette designated pD17-hGla was constructed from pD13-hG I a by digesting pD13-hG1a a with *Asp*7181 and *BamH*I and isolating the 2.7 kb fragment. This fragment contains the human gamma 1 gene and flanking polylinker sequences. DM I (Dam^{.}) bacteria were used to amplify pD 17 and the isolated plasmid was digested with *Asp*718I and *Bcl*I giving a 5.6 kb vector fragment. The 2.7 kb human gamma I encoding fragment was ligated into the isolated vector fragment and used to transform competent *E*. *coli* DH5α and the proper construction confirmed by the release of 7.13 and 1.26 kb fragments following digestion with BstEII.

Construction of the heavy chain expression vector was completed by inserting a PCR fragment encoding a signal peptide and the h106 Vh sequence, ASY, into the *Nhe*I and *Xho*I sites of pD 17-hG I a. The PCR fragment was constructed by splice overlap extension using VHASY 24-17 (hhh) as template and primers LH15 (Seq. I D #72), LH16 (Seq. LD # 73), LH17 (Seq. ID # 74) and LH18 (Seq. ID # 75). In combination, primers LH 15, LH16 and LH 17 encode an EcoRV restriction site, a signal peptide and the 5' end of h106 Vκ. Primer LH16 encodes the JH portion of the variable heavy chain gene through an artificial Nhel site at the 5' end of the CH 1 domain. This construction provides for no intron between the JH and CH I domains. The PCR reactions were repeated with the two outside primers (LH 15 and LH 18) to ensure a full length product. The outside primers also provided sufficient overlap with the vector sequences in the target region of the vector to allow cloning by homologous recombination.

To insert the PCR fragment into pD17-hGla, *EcoRV* and *Nhe*I were used to linearize the vector and the fragments were ligated and used to transform competent *E*. *coli* DH5α by homologous recombination (Babeck et al., 1993, *Nucl. Acids. Res. 21*:3601-3602). The presence of an insert was determined by PCR and the sequencing of the variable region. Figure 17 is a depiction of the final construction for pD 17-hK8.H1. DNA sequencing was carried out for the insert and flanking regions to confirm the final product. The nucleic acid (Seq. ID # 78) and amino acid residue (Seq. ID # 79) sequences for the sequenced regions are depicted in Figure 18.

Two other constructs containing codons which encode the murine amino acid residues at positions 24, 55 and 56 (pD 17-hK 1.H 1) of the variable heavy chain region and the human amino acid residues at positions 24 and 56 and the murine amino acid residue at position 55 (pDl7-hK6.H1) were also constructed. The constructions were carried out in the same way as described above for pD17-hK8.H1 except the PCR templates VHTDS 15-34 (mmm) and VHADY 7-8 (hmh), respectively, were used.

**TABLE 14**

| Primers Used in the Construction of Humanized Monoclonal Antibody 106 |
|---|
| LH1 - 5' primer for linearizing site polylinker upstream of CM V promoter |
| 5'-GATCTGCTAGCCCGGGTGACCTGAGGCGCGCCTTTGGCGCC-3' Seq. ID #58 |
| |
| LH2 - 3' primer for linearizing site polylinker upstream of CMV promoter written in 3' to 5' orientation, |
| 3'-ACGATCGGGCCCACTGGACGCCGCGCGGAAACCGCGGCTAG-5' Seq. ID #59 |
| |
| LH3 - 5' primer for histone H3 transcription termination sequence |
| 5'-CCGGGCCTCTCAAAAAAGGGAAAAAAAGCATG-3' Seq. ID #60) |
| |
| LH4 - 3' primer for histone H3 transcription termination sequence |
| 3'-CGGAGAGTTTTTTCCCTTTTTTTC-5' Seq. ID #61 |
| |
| LH5 - 5' primer for gastrin transcription termination sequence |
| 5'-CGCGCCGGCTTCGAATAGCCAGAGTAACCTTTTTTTTTA ATTTTATTTTATTTTATTTTTGAGATGGAGTTTGG-3' |
| |
| LH6 - 3' primer for gastrin transcription termination sequence |
| 3'-GGCCGAAGCTTATCGGTCTCATTGGAAAAAAAAATTAA AATAAAATAAAATAAAAACTCTACCTCAAACCGC-5' |
| |
| LH7 - cloning polylinker for pD13 |
| 5' TAGGGAGACCCAAGCTTGGTACCAATTTAAATTGATATC TCCTTAGGTCTCGAGTCTCTAGATAACCGGTCAATCGAT TGGGATTCTT 3' |
| |
| LH8 - cloning polylinker for pD13 |
| 5' GACACTATAGAATAGGGCCCTTCCGCGGTTGGATCCAAC ACGTGAAGCTAGCAAGCGGCCGCAAGAATTCCAATCGAT TGACCGGTTA 3' |
| |
| LH9 - sense primer signal peptide region hu106 VL |
| ATCTCCTTAGGTCTCGAGACCATGGACATGAGGGTTCCGG CTC |
| |
| LH 10 - anti-sense primer signal peptide region hu106 VL |
| GGAGCCAGAGTAGCAGGAGCCCCAGGAGCTGAGCCGGAAC CCTCA |
| |
| LH11 - sense primer signal peptide region hu 106 VL |
| CTGCTACTCTGGCTCCGAGGTGCCAGATGTGACATCCAGAT |
| GACTCAGTCTC |
| |
| LH12 - antisense primer hu 106VL, Jκ-and 3' intron |
| GATTGACCGGTTATCTAGAGACTCGAGACTTACGTTTGATC TCGACCTTGG Seq. ID #69 |
| |
| LH13 - sense primer Nhe I to BstE II fragment of human gamma I gene, Cla I and Nhe I sites underlined |
| 5' GACCATGATTACGAATTCCATCGATGCTAGCACCAAGGG CCCATCGGTCTTCC 3' Seq. ID #70 |
| |
| LH14 - antisense primer Nhe 1 to BstE II fragment of human gamma I gene, Xho I and BstE II sites underlined |
| 5' AGCTTTCGCGAGCTCGAG GGTCACCACGCTGCTGAGGGA Seq. ID #71 |
| |
| LH 15 - sense primer for signal peptide region hu 106 VH |
| TTGCGGCCGCTTGCTAGCACCATGGAACTCGGCCTCCGCTG GGTT Seq. ID #72 |
| |
| LH16 - anti-sense primer signal peptide region hu106 VH |
| CTGGACACCTTCTAAAATAGCAACAAGGAAAACCCAGCGGA GGCCGAG Seq. ID #73 |
| |
| LH17 - sense primer signal peptide region hu106 VH |
| ATTTTAGAAGGTGTCCAGTGTGAAGTGCAGCTGGTGGAGTC TGG Seq. ID #74 |
| |
| LH18 - antisense primer hu 106VH, JH-CH I region |
| TGGGCCCTTGGTGCTAGCCGAGGAGACGGTGACCAGGGTTC CT Seq. ID #75 |

### EXAMPLE 14

### Testing of Humanized Monoclonal Antibody 106 for Biologic Activity

In this example, the three forms of humanized monoclonal antibody 106 were produced by transient expressions from COS cells. The antibodies were tested for (1) inhibition of B cell proliferation, (2) production of antibodies by activated T cell stimulated B cells, and (3) induction of E-selectin expression on endothelial cells.

Transfection of COS cells was carried out as previously described (Linsley et al. 1991. J. Exp. Med. 173:721-730; Aruffo and Seed, 1987. Proc. Nat'l Acad. Sci. USA 84:8573-8577). Plasmid DNA was added to transfection media at I µg/ml in a total volume of 12 ml/150 mm plate. After seven to 10 days, the culture media was collected, centrifuged to clarify and passed over a proteinA sepharose column as described above. The column was then washed with PBS and bound antibody eluted with 0.05 M sodium citrate, pH 30. Fractions were neutralized, pooled and dialyzed against cold PBS. The dialyzed eluate was concentrated using a Centriprep concentrator (Amersham) and protein concentration were determined by absorbance at 280 nm using an extinction coefficient of 1.35 m/mg⁻¹cm⁻¹.

Inhibition of B cell proliferation by soluble gp39 (sgp39) and anti-human IgM was determined by culturing 5 x 10⁵ resting human tonsillar cells in the presence of sgp39, rabbit anti-human IgM immunobeads and varying amounts of the humanized 106 monoclonal antibody in 96 well plates for 72 hours at 37°C, 6% CO₂. The plates were then pulsed with 1 µCi/well [³H]-thymidine for 18 hours and [³H] incorporation determined. All tests were performed in triplicate and the results are expressed as percent inhibition compared to cultures maintained in culture medium only. As seen in Figure 19, all three humanized whole antibody constructs of monoclonal antibody 106 were capable of inhibiting the stimulation of B cell proliferation by soluble gp39 and anti-human IgM. The construct containing the amino acid residues from the murine sequence at positions 24, 55 and 56 was the most similar to the original murine 106 antibody.

Antibody production by human peripheral B cells stimulated with activated T cells was tested as described above. Briefly, human peripheral blood mononuclear cells were depleted of monocytes and natural killer cells and then separated into T cells and B cells by E rosetting. The isolated T cells were treated with mitomycin C and than co-cultured with B cells in anti-CD3 monoclonal antibody coated wells of a 96 well plate in the presence of varying concentrations of the different forms of humanized 106 monoclonal antibodies. All tests were run in triplicate and the results are expressed as percent inhibition compared to cultures that contained medium which did not contain any anti-gp39 monoclonal antibody.

As can be seen in Figure 20, all of the antibodies were able to at least partially block the ability of activated T cells to stimulate antibody production by B cells. None of the humanized antibodies were as capable as the murine anti-gp39 monoclonal antibody 106 at blocking antibody production. The antibody having murine amino acid residues at positions 24, 55 and 56, h106-2, was the most successful.

The humanized whole antibody constructs were also tested for their ability to prevent the induction of E-selectin expression on endothelial cells by a gp39' T cell line. As described above, except that the human umbilical vein endothelial cells were co-cultured with BMS-2 cells (a Jurkat line known to constitutively express gp39) instead of sgp39, in the presence of the humanized anti-gp39 monoclonal antibodies. After four hours, the level of E-selectin expression was measured by ELISA. Figure 21.

Results for this assay, as with the previous assays, demonstrate that all of the humanized whole antibody constructs made inhibit the interaction between gp39 and CD40 preventing, in this case, the induction of the expression of E-selectin on endothelial cells by activated T cells. Also, as demonstrated by the assays above, the construct having murine amino acid residues at positions 24, 55 and 56, h106-2, was most like the parental murine monoclonal antibody 106.

### Cell Line Deposits

The following hybridoma cell lines were deposited with the American Type Culture Collection, 12301 Parklawn Drive, Rockville, Maryland 20852-2776, USA under the terms of the Budapest Treaty.

| Hybridoma | ATCC Designation |
|---|---|
| 39-1.29 | HB 11808 |
| 39-1.132 | HB 11809 |
| 39-1.134 | HB 11810 |
| 39-1.106 | HB 11811 |
| 39-1.7 | HB 11812 |
| 39-1.37 | HB 11813 |
| 39-1.77 | HB 11814 |
| 39-1.59 | HB 11815 |
| 39-1.122 | HB 11816 |
| 39-1.156 | HB 11817 |
| 39-1.128 | HB 11818 |
| 39-1.124 | HB 11819 |
| 39-1.26 | HB 11820 |
| 39-1.138 | HB 11821 |
| 39-1.3 | HB 11822 |
| 39-7.3E12 | HB 11823 |
| 39-5.6E9 | |
| 39-9.246 | |
| 39-9.11 | |
| 39-9.274 | |

## Claims

1. A monoclonal antibody, an antigen binding fragment or recombinant binding protein thereof, which is specific for human gp39, wherein the antibody is that secreted by the hybridoma 39-1.7 designated ATCC HB 11812, 39-1.128 designated ATCC HB 11818, or 39-1.26 designated ATCC HB 11820.

2. The antigen binding fragment of claim 1, wherein the fragment is derived from the antibody secreted by the hybridoma 39-1.7 designated ATCC HB 11812, 39-1.128 designated ATCC HB 11818, or 39-1.26 designated ATCC HB 11820.

3. The antigen binding fragment of claim 1, wherein the fragment is a Fab, F(ab¹)₂, or Fv.

4. The recombinant binding protein of claim 1, wherein the protein is an sFv, a humanized antibody or a recombinant protein comprising a variable region of an antibody of claim 1.

5. An *in vitro* method for the detection of X-linked hyper IgM syndrome comprising:
(a) isolated peripheral blood lymphocytes provided from a patient;
(b) activating the isolated peripheral blood lymphocytes;
(c) fixing and permeabilizing the isolated and activated peripheral blood lymphocytes,
(d) admixing a monoclonal antibody, antigen binding fragment or recombinant binding fragment thereof of any of claims 1 through 4 with the activated, fixed and permeabilized peripheral blood lymphocytes;
(e) detecting antibody bound to the cells.

6. The method of claim 5, wherein the antibody, antigen binding fragment or recombinant binding fragment thereof, is conjugated with a detectable label.

7. The method of claim 5, wherein the method further comprises the step of adding a labeled secondary antibody specific for the first antibody, antigen binding fragment or recombinant binding fragment thereof, after step (d).

8. The method of claim 7, wherein the label is a fluorophore, radioactive isotope, enzyme, or chromophore.

9. A monoclonal antibody, an antigen binding fragment or recombinant binding protein thereof of any of claims 1 through 4, conjugated to detectable marker.

10. A monoclonal antibody, an antigen binding fragment or recombinant binding protein thereof of any of claims 1 through 4, wherein the detectable marker is a fluorophore, radioactive isotope, enzyme or chromophore.

11. A monoclonal antibody, an antigen binding fragment or recombinant binding protein thereof of any of claims 1 through 4, wherein the antibody is conjugated to a therapeutic agent.

12. A monoclonal antibody, an antigen binding fragment or recombinant binding protein of claim 11, wherein the therapeutic agent is a radioisotope, a toxin, or a chemotherapeutic agent.

13. The recombinant binding protein of any of claims 1 through 4, wherein the recombinant binding protein comprises a variable region capable of binding to the antigen and a protein toxin or therapeutic agent in the form of a fusion protein.

14. Hybridoma HB 11812, HB 11818, HB 11820 as deposited with the American Type Culture Collection.

15. An isolated and purified nucleic acid sequence which encodes an immunoglobulin light chain variable having the amino acid residue sequence of Sequence ID# 16.

16. The nucleic acid sequence of claim 15, wherein the nucleic acid sequence is that of Sequence ID# 15.

17. An isolated and purified nucleic acid sequence which encodes an immunoglobulin heavy chain variable region having the amino acid residue sequence of Sequence ID# 18.

18. The nucleic acid sequence of claim 17, wherein the nucleic acid sequence is that of Sequence ID# 17.

19. A pharmaceutical composition comprising a monoclonal antibody, antigen binding fragment or recombinant binding fragment thereof, of any of claims 1 through 4 and a pharmaceutically acceptable carrier.

20. A pharmaceutical composition comprising a monoclonal antibody, antigen binding fragment or recombinant binding fragment thereof, of any of claims 1 through 4, conjugated to a detectable marker and a pharmaceutically acceptable carrier.

21. A pharmaceutical composition comprising a monoclonal antibody, antigen binding fragment or recombinant binding fragment thereof, of any of claims 1 through 4, conjugated to a therapeutic agent and a pharmaceutically acceptable agent.

22. A monoclonal antibody, antigen binding fragment or recombinant binding fragment thereof, of any of claims 1 through 4, conjugated to a detectable marker for use in the imaging of cells expressing gp39 on their surface in a patient comprising:
(a) administering to a patient a pharmaceutical composition of claim 20 under conditions permitting the formation of a binding protein/antigen complex on the surface of the cells expressing gp39; and
(b) detecting the presence of the binding protein/antigen complex on the surface of the cells as indicated by the presence of the detectable marker.

## Patentansprüche

1. Monoklonaler Antikörper, ein Antigenbindungsfragment oder rekombinantes Bindungsprotein desselben, das für humanes gp39 spezifisch ist, wobei der Antikörper der ist, der vom Hybridom 39-1.7, bezeichnet ATCC HB 11812, vom 39-1.128, bezeichnet ATCC HB 11818 oder vom 39-1.26, bezeichnet ATCC HB 11820, sezerniert wird.

2. Antigenbindungsfragment nach Anspruch 1, wobei das Fragment vom Antikörper abgeleitet ist, der vom Hybridom 39-1.7, bezeichnet ATCC HB 11812, vom 39-1.128, bezeichnet ATCC HB 11818 oder vom 39-1.26, bezeichnet ATCC HB 11820, sezerniert wird.

3. Antigenbindungsfragment nach Anspruch 1, wobei das Fragment ein Fab, F(ab¹)₂ oder Fv ist.

4. Rekombinantes Bindungsprotein nach Anspruch 1, wobei das Protein ein sFv, ein humanisierter Antikörper oder ein rekombinantes Protein ist, umfassend eine variable Region eines Antikörpers nach Anspruch 1.

5. In-vitro-Verfahren zum Nachweis des X-gekoppelten Hyper-IgM-Syndroms, umfassend:
(a) aus dem peripheren Blut isolierte Lymphozyten, bereitgestellt von einem Patienten;
(b) Aktivierung der aus dem peripheren Blut isolierten Lymphozyten;
(c) Fixierung und Permeabilisierung der aus dem peripheren Blut isolierten und aktivierten Lymphozyten,
(d) Beimischung eines monoklonalen Antikörpers, eines Antigenbindungsfragments oder eines rekombinanten Bindungsfragments desselben nach einem der Ansprüche 1 bis 4 mit den aktivierten, fixierten und permeabilisierten Lymphozyten aus dem peripheren Blut;
(e) Nachweis eines an die Zellen gebundenen Antikörpers.

6. Verfahren nach Anspruch 5, wobei der Antikörper, das Antigenbindungsfragment oder das rekombinante Bindungsfragment desselben mit einem nachweisbaren Label konjugiert ist.

7. Verfahren nach Anspruch 5, wobei das Verfahren nach Schritt (d) ferner den Schritt des Zufügens eines für den ersten Antikörper spezifischen markierten sekundären Antikörpers, eines Antigenbindungsfragments oder eines rekombinanten Bindungsfragments desselben umfasst.

8. Verfahren nach Anspruch 7, wobei das Label ein Fluorphor, radioaktives Isotop, Enzym oder Chromophor ist.

9. Monoklonaler Antikörper, ein Antigenbindungsfragment oder rekombinantes Bindungsprotein desselben nach einem der Ansprüche 1 bis 4, konjugiert an einen nachweisbaren Marker.

10. Monoklonaler Antikörper, ein Antigenbindungsfragment oder rekombinantes Bindungsprotein desselben nach einem der Ansprüche 1 bis 4, wobei der nachweisbare Marker ein Fluorphor, radioaktives Isotop, Enzym oder Chromophor ist.

11. Monoklonaler Antikörper, ein Antigenbindungsfragment oder rekombinantes Bindungsprotein desselben nach einem der Ansprüche 1 bis 4, wobei der Antikörper an ein therapeutisches Mittel konjugiert ist.

12. Monoklonaler Antikörper, ein Antigenbindungsfragment oder rekombinantes Bindungsprotein nach Anspruch 11, wobei das therapeutische Mittel ein Radioisotop, ein Toxin oder ein chemotherapeutisches Mittel ist.

13. Rekombinantes Bindungsprotein nach einem der Ansprüche 1 bis 4, wobei das rekombinante Bindungsprotein eine variable Region umfasst, die zur Bindung an das Antigen fähig ist, und ein Proteintoxin oder therapeutisches Mittel in der Form eines Fusionsproteins.

14. Hybridom HB 11812, HB 11818, HB 11820 wie bei der American Type Culture Collection hinterlegt.

15. Isolierte und gereinigte Nukleinsäuresequenz, die für eine Immunglobulin-Leichtketten variable Region mit der Aminosäurerest-Sequenz von SEQ ID NO: 16 kodiert.

16. Nukleinsäuresequenz nach Anspruch 15, wobei die Nukleinsäuresequenz die von SEQ ID NO: 15 ist.

17. Isolierte und gereinigte Nukleinsäuresequenz, die für eine Immunglobulin-Schwerketten variable Region mit der Aminosäurerest-Sequenz von SEQ ID NO: 18 kodiert.

18. Nukleinsäuresequenz nach Anspruch 17, wobei die Nukleinsäuresequenz die von SEQ ID NO: 17 ist.

19. Pharmazeutische Zusammensetzung, umfassend einen monoklonalen Antikörper, ein Antigenbindungsfragment oder ein rekombinantes Bindungsfragment desselben nach einem der Ansprüche 1 bis 4 und einen pharmazeutisch verträglichen Träger.

20. Pharmazeutische Zusammensetzung, umfassend einen monoklonalen Antikörper, ein Antigenbindungsfragment oder ein rekombinantes Bindungsfragment desselben nach einem der Ansprüche 1 bis 4, konjugiert an einen nachweisbaren Marker und einen pharmazeutisch verträglichen Träger.

21. Pharmazeutische Zusammensetzung, umfassend einen monoklonalen Antikörper, ein Antigenbindungsfragment oder ein rekombinantes Bindungsfragment desselben nach einem der Ansprüche 1 bis 4, konjugiert an ein therapeutisches Mittel und einen pharmazeutisch verträglichen Träger.

22. Monoklonaler Antikörper, ein Antigenbindungsfragment oder rekombinantes Bindungsfragment desselben, nach einem der Ansprüche 1 bis 4, konjugiert an einen nachweisbaren Marker zur Verwendung bei der Bildgebung von Zellen, die auf ihrer Oberfläche gp39 exprimieren, in einem Patienten, umfassend:
(a) Verabreichung einer pharmazeutischen Zusammensetzung nach Anspruch 20 an einen Patienten unter Bedingungen, welche die Bildung eines Bindungsprotein/Antigen-Komplexes auf der Oberfläche der gp39 exprimierenden Zellen zulassen; und
(b) Nachweis des Vorliegens des Bindungsprotein/Antigen-Komplexes auf der Oberfläche der Zellen, wie durch das Vorliegen des nachweisbaren Markers angezeigt ist.

## Revendications

1. Anticorps monoclonal, fragment de liaison à l'antigène ou protéine de liaison recombinante de celui-ci, lequel est spécifique pour gp39 humaine, où l'anticorps est celui qui est sécrété par l'hybridome 39-1.7, désigné ATCC HB 11812, 39-1.128 désigné ATCC HB 11818 ou 39-1.26 désigné ATCC HB 11820.

2. Fragment de liaison à un antigène selon la revendication 1, où le fragment est dérivé de l'anticorps sécrété par l'hybridome 39-1.7, désigné ATCC HB 11812, 39-1.128 désigné ATCC HB 11818 ou 39-1.26 désigné ATCC HB 11820.

3. Fragment de liaison à un antigène selon la revendication 1, où le fragment est un Fab, F(ab¹)₂ ou Fv.

4. Protéine de liaison recombinante selon la revendication 1, où la protéine est un sFv, un anticorps humanisé ou une protéine recombinante comprenant une région variable d'un anticorps selon la revendication 1.

5. Procédé *in vitro* de détection d'un syndrome hyper-IgM lié à l'X, comprenant:
(a) des lymphocytes isolés du sang périphérique fourni par un patient;
(b) activer les lymphocytes isolés du sang périphérique;
(c) fixer et perméabiliser les lymphocytes isolés du sang périphérique et activés;
(d) ajouter et mélanger un anticorps monoclonal, un fragment de liaison à l'antigène ou une protéine de liaison recombinante de celui-ci selon l'une quelconque des revendications 1 à 4, avec les lymphocytes du sang périphérique activés, fixés et perméabilisés;
(e) détecter l'anticorps lié aux cellules.

6. Procédé selon la revendication 5, dans lequel l'anticorps, le fragment de liaison à l'antigène ou la protéine de liaison recombinante de celui-ci, est conjugué à un marqueur détectable.

7. Procédé selon la revendication 5, où le procédé comprend en outre l'étape consistant à ajouter un anticorps secondaire marqué spécifique pour le premier anticorps, fragment de liaison à l'antigène ou protéine de liaison recombinante de celui-ci, après l'étape (d).

8. Procédé selon la revendication 7, dans lequel le marqueur est un fluorophore, un isotope radioactif, une enzyme ou un chromophore.

9. Anticorps monoclonal, fragment de liaison à l'antigène ou protéine de liaison recombinante de celui-ci selon l'une quelconque des revendications 1 à 4, conjugué à un marqueur détectable.

10. Anticorps monoclonal, fragment de liaison à l'antigène ou protéine de liaison recombinante de celui-ci selon l'une quelconque des revendications 1 à 4, où le marqueur détectable est un fluorophore, un isotope radioactif, une enzyme ou un chromophore.

11. Anticorps monoclonal, fragment de liaison à l'antigène ou protéine de liaison recombinante de celui-ci selon l'une quelconque des revendications 1 à 4, où l'anticorps est conjugué à un agent thérapeutique.

12. Anticorps monoclonal, fragment de liaison à l'antigène ou protéine de liaison recombinante selon la revendication 11, où l'agent thérapeutique est un radio-isotope, une toxine ou un agent chimiothérapeutique.

13. Protéine de liaison recombinante selon l'une quelconque des revendications 1 à 4, où la protéine recombinante comprend une région variable capable de se lier à l'antigène et une toxine protéique ou un agent thérapeutique sous forme d'une protéine de fusion.

14. Hybridome HB 11812, HB 11818, HB 11820 tels que déposés auprès de la American Type Culture Collection.

15. Séquence d'acide nucléique isolé et purifié qui code une région variable de chaîne légère d'immunoglobuline ayant la séquence de résidus d'acides aminés de la SEQ ID NO:16.

16. Séquence d'acide nucléique selon la revendication 15, où la séquence d'acide nucléique est celle de la SEQ ID NO:15.

17. Séquence d'acide nucléique isolé et purifié qui code une région variable de chaîne lourde d'immunoglobuline ayant la séquence de résidus d'acides aminés de la SEQ ID NO:18.

18. Séquence d'acide nucléique selon la revendication 17, où la séquence d'acide nucléique est celle de la SEQ ID NO:17.

19. Composition pharmaceutique comprenant un anticorps monoclonal, un fragment de liaison à l'antigène ou un fragment de liaison recombinant de celui-ci selon l'une quelconque des revendications 1 à 4 et un véhicule pharmaceutiquement acceptable.

20. Composition pharmaceutique comprenant un anticorps monoclonal, un fragment de liaison à l'antigène ou un fragment de liaison recombinant de celui-ci selon l'une quelconque des revendications 1 à 4, conjugué à un marqueur détectable et un véhicule pharmaceutiquement acceptable.

21. Composition pharmaceutique comprenant un anticorps monoclonal, un fragment de liaison à l'antigène ou un fragment de liaison recombinant de celui-ci selon l'une quelconque des revendications 1 à 4, conjugué à un agent thérapeutique et un véhicule pharmaceutiquement acceptable.

22. Anticorps monoclonal, fragment de liaison à l'antigène ou fragment de liaison recombinant de celui-ci selon l'une quelconque des revendications 1 à 4, conjugué à un marqueur détectable à utiliser dans l'imagerie de cellules exprimant gp39 sur leur surface chez un patient, comprenant:
(a) administrer à un patient une composition pharmaceutique selon la revendication 20 dans des conditions permettant la formation d'un complexe de liaison protéine/antigène sur la surface des cellules exprimant gp39; et
(b) détecter la présence du complexe de liaison protéine/antigène sur la surface des cellules, telle qu'indiquée par la présence du marqueur détectable.
